# EUROPEAN PATENT APPLICATION

(11) **EP 4 374 850 A2**
(43) Date of publication of application: **29.05.2024**
(21) Application number: 24156669.4
(22) Date of filing: 21.09.2018
(51) Int. Cl.: A61K 9/00

(54) **METHODS OF PREVENTING METHIONINE OXIDATION IN IMMUNOCONJUGATES**

(30) Priority: 22.09.2017 US 201762562049 P; 17.10.2017 US 201762573322 P; 31.07.2018 US 201862712584 P
(62) Divisional of application: 18783303.3
(71) Applicant: ImmunoGen, Inc., Waltham, MA 02451-1477 (US)
(72) Inventor: FLEMING, Michael, Londonderry, NH (US); GANGAR, Amit, Waltham, MA (US); YODER, Nicholas, C., Brookline, MA (US); BAI, Chen, Arlington, MA (US); HILDERBRAND, Scott, Alan, Swampscott, MA (US); HUTCHINS, Benjamin, M., Boxborough, MA (US)
(74) Representative: Marks & Clerk LLP

(57) **Abstract**

The present invention generally relates to methods of preventing methionine oxidation in immunoconjugates. The present invention also relates to pharmaceutical compositions of immunoconjugates in which the amount of methionine oxidation is minimized.

## Description

### REFERNCE TO RELATED APPLICATIONS

This application claims the benefit of the filing date, under 35 U.S.C. § 119(e), of U.S. Provisional Application No. 62/562,049, filed on September 22, 2017, U.S. Provisional Application No. 62/573,322, filed on October 17, 2017, and U.S. Provisional Application No. 62/712,584, filed on July 31, 2018. The entire contents of each of the above-referenced applications are incorporated herein by reference.

### FIELD OF THE INVENTION

The present invention generally relates to methods of preventing methionine oxidation in immunoconjugates. The present invention also relates to pharmaceutical compositions of immunoconjugates in which the amount of methionine oxidation is minimized.

### BACKGROUND OF THE INVENTION

Cell binding agent-drug conjugates (such as antibody-drug conjugates (ADC)) are emerging as a powerful class of anti-tumor agents with efficacy across a range of cancers. Cell binding agent-drug conjugates (such as ADCs) are commonly composed of three distinct elements: a cell-binding agent (*e.g.,* an antibody); a linker; and a cytotoxic moiety. The cytotoxic drug moiety can be covalently attached to lysines on the antibody, resulting in conjugates that are heterogeneous mixtures of ADCs bearing varying numbers of drugs attached at different positions on the antibody molecule. Alternatively, the cytotoxic drug moiety can be covalently linked to cysteine thiol groups on the antibody through a thiol-reactive group, such as a maleimde group, to form site-specific ADCs.

Benzodiazepine compounds, including tricyclic benzodiazepines, such as pyrrolobenzodiazepines (PBD), and tetracyclic benzodiazepines, such as indolinobenzodiazepines, have been employed as cytotoxic agents in linkage with antibodies to generate ADCs, which have shown promising antitumor activities. These benzodiazepine compounds contain imine bonds, which can bind to the minor groove of DNA and interfere with DNA function, resulting in cell death.

Therefore, there is a need to develop new methods for preparing conjugates of cell-binding agent and imine-containing benzodiazepine drugs as well as new pharmaceutical compositions of these conjugates that are stable during the manufacturing process and/or storage.

### SUMMARY OF THE INVENTION

The present invention is based on the surprising findings that the immunoconjugates comprising benzodiazepine cytotoxic agents may be prone to methionine oxidation, particularly in the presence of light. Comparative data with naked antibodies suggest that the methionine oxidation is induced, at least in part, by the presence of the benzodiazepine cytotoxic agent. It is surprisingly found that the presence of methionine in the pharmaceutical compositions of antibody-benzodiazepine immunoconjugates can reduce the amount of methionine oxidation observed. In addition, the presence of methionine antioxidant in the conjugation reaction between the antibodies and the benzodiazepine cytototoxic agents can significantly decrease the amount of methionine oxidation in the immunoconjugates produced.

One aspect of the invention provides a pharmaceutical composition comprising an immunoconjugate described herein and 0.1 mM to 20 mM methionine.

In certain embodiments, the immunoconjugate in the pharmaceutical compositions of the present invention is represented by the following formula: 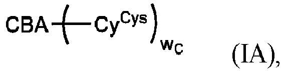
CBA is an antibody or antigen-binding fragment thereof;
Wc is 1 or 2; and
Cy^{Cys} is represented by the following formula: 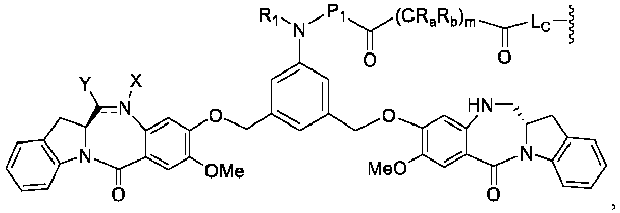
or a pharmaceutically acceptable salt thereof, wherein:
   the double line -̅ -̅ between N and C represents a single bond or a double bond, provided that when it is a double bond, X is absent and Y is -H or a (C₁-C₄)alkyl; and when it is a single bond, X is -H or an amine protecting moiety, Y is -OH or -SO₃H;
   R₁ is -H or a (C₁-C₃)alkyl;
   P₁ is an amino acid residue or a peptide containing 2 to 5 amino acid residues;
   Rₐ and R_{b}, for each occurrence, are independently -H, (C₁-C₃)alkyl, or a charged substituent or an ionizable group Q;
   m is an integer from 1 to 6; L_{C} is represented by 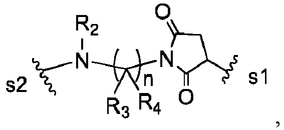 s1 is the site covalently linked to CBA, and s2 is the site covalently linked to the -C(=O)- group on Cy^{Cys}; wherein:
      R₂ is -H or a (C₁-C₃)alkyl
      R₃ and R₄, for each occurrence, are independently -H or a (C₁-C₃)alkyl; and n is an integer between 1 and 10.

In certain embodiments, the immunoconjugate in the pharmaceutical composition of the present invention is represented by the following formula: 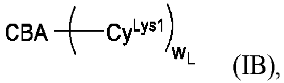 wherein:
CBA is an antibody or antigen-binding fragment thereof;
W_{L} is an integer from 1 to 20; and
Cy^{Lys1} is represented by the following formula: 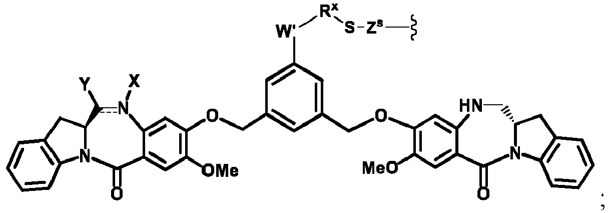
or a pharmaceutically acceptable salt thereof, wherein:
   the double line -̅ -̅ between N and C represents a single bond or a double bond, provided that when it is a double bond, X is absent and Y is -H or a (C₁-C₄)alkyl; and when it is a single bond, X is -H or an amine protecting moiety, and Y is -OH or -SO₃H;
   R^{x} is independently a (C₁-C₆)alkyl;
   W' is -NR^{e},
   R^{e} is -(CH₂-CH₂-O)ₙ₁-R^{k};
   n1 is an integer from 2 to 6;
   R^{k} is -H or -Me;
   Z^{S} is selected from any one of the following formulas: 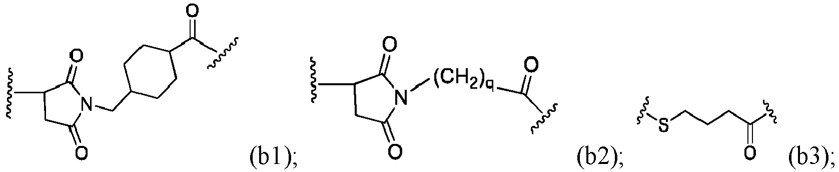 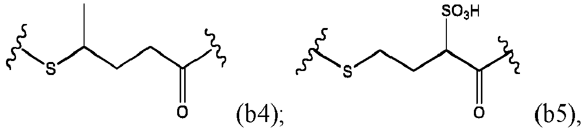 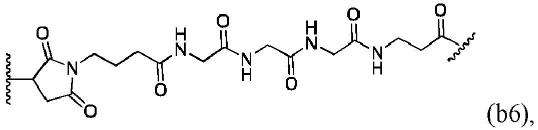 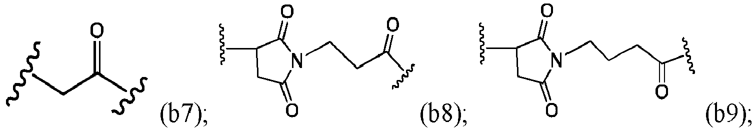 and 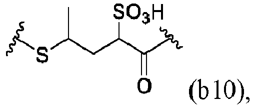 or a pharmaceutically acceptable salt thereof, wherein q is an integer from 1 to 5.

In certain embodiment, the present invention provides a pharmaceutical composition comprising 1 mM to 4 mM of methionine and an immunoconjugate represented by the following formula: 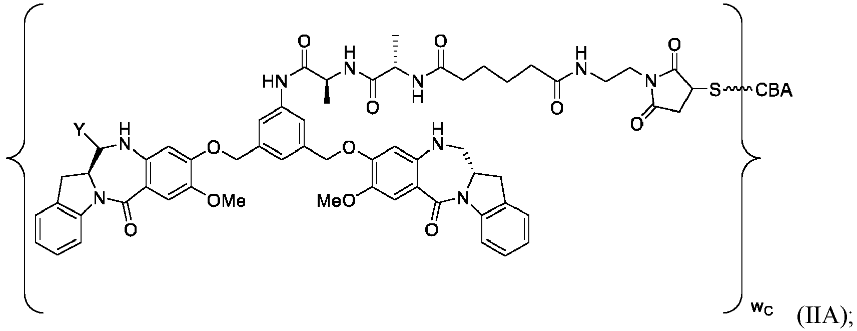 or a pharmaceutically acceptable salt thereof, wherein:
Y is -SO₃H or sodium salt thereof;
W_{C} is 2; and
CBA is an anti-CD123 antibody comprising: a) an immunoglobulin heavy chain having the amino acid sequence set forth in SEQ ID NO:8; and b) an immunoglobulin light chain having the amino acid sequence set forth in SEQ ID NO: 10.

In certain embodiments, the present invention provides a pharmaceutical composition comprising 1 mM to 4 mM of methionine and an immunoconjugate represented by the following formula: 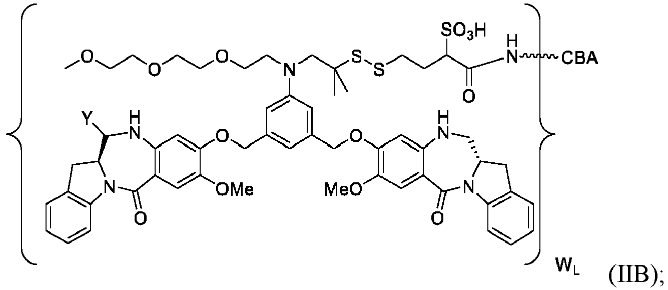 or a pharmaceutically acceptable salt thereof, wherein:
Y is -SO₃H or sodium salt thereof;
W_{L} is an integer from 1 to 10; and
CBA is an anti-CD33 antibody comprising an immunoglobulin heavy chain having the amino acid sequence set forth in SEQ ID NO: 18 and an immunoglobulin light chain having the amino acid sequence set forth in SEQ ID NO:20.

Another aspect of the present invention provides a method of reducing the amount of methionine oxidation in an immunoconjugate described herein comprising mixing the immunoconjugate with 0.1 mM to 20 mM methionine to give a pharmaceutical composition comprising the immunoconjugate and methionine.

In certain embodiments, the immunoconjugate is represented by formula (IA) described above.

In certain embodiments, the immunoconjugate is represented by formula (IB) described above.

In yet another aspect, the present invention provides a method of preparing immunoconjugates described herein comprising reacting a cell-binding agent (CBA) with a cytotoxic agent or a cytotoxic agent-linker compound described herein in the presence of an antioxidant to reduce the amount of methionine oxidation in the immunoconjugates.

In certain embodiments, the present invention provides a method of preparing an immunoconjugate represented by the following formula: 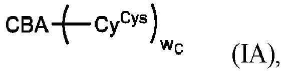 comprising reacting a CBA with a cytotoxic agent represented by the following formula: 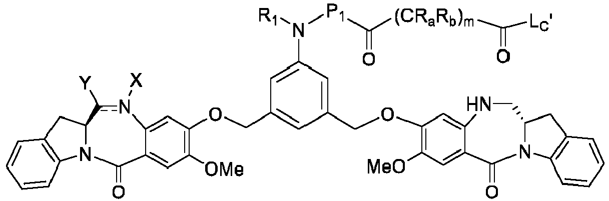 or a pharmaceutically acceptable salt thereof, in the presence of an antioxidant, wherein:
CBA is an antibody or antigen-binding fragment thereof;
Wc is 1 or 2; and
Cy^{Cys} is represented by the following formula: 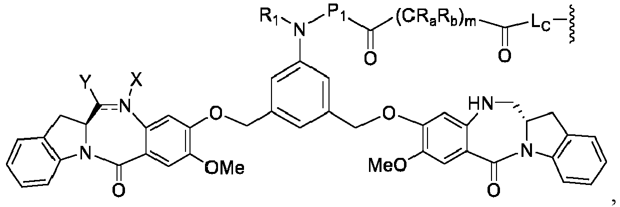
or a pharmaceutically acceptable salt thereof, wherein:
   the double line -̅ -̅ between N and C represents a single bond or a double bond, provided that when it is a double bond, X is absent and Y is -H or a (C₁-C₄)alkyl; and when it is a single bond, X is -H or an amine protecting moiety, Y is -OH or -SO₃H;
   R₁ is -H or a (C₁-C₃)alkyl;
   P₁ is an amino acid residue or a peptide containing 2 to 5 amino acid residues;
   Rₐ and R_{b}, for each occurrence, are independently -H, (C₁-C₃)alkyl, or a charged substituent or an ionizable group Q;
   m is an integer from 1 to 6; L_{C} is represented by 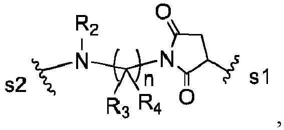 s1 is the site covalently linked to CBA, and s2 is the site covalently linked to the -C(=O)- group on Cy^{C1};
      R₂ is -H or a (C₁-C₃)alkyl
      R₃ and R₄, for each occurrence, are independently -H or a (C₁-C₃)alkyl; and n is an integer between 1 and 10; and L_{C}' is represented by

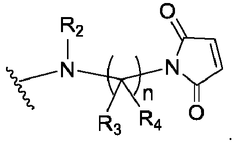

In certain embodiment, the present invention provides method of preparing an immunoconjugate represented by the following formula: 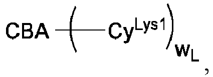 wherein:
CBA is an antibody or antigen-binding fragment thereof;
W_{L} is an integer from 1 to 20; and
Cy^{Lys1} is represented by the following formula: 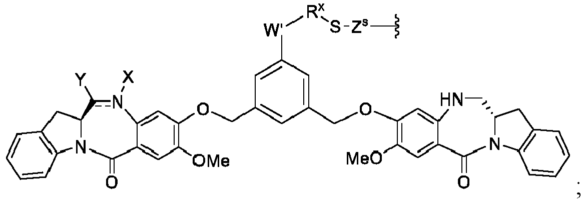
or a pharmaceutically acceptable salt thereof, wherein:
   the double line -̅ -̅ between N and C represents a single bond or a double bond, provided that when it is a double bond, X is absent and Y is -H or a (C₁-C₄)alkyl; and when it is a single bond, X is -H or an amine protecting moiety, and Y is -OH or -SO₃H;
   R^{x} is independently a (C₁-C₆)alkyl;
   W' is -NR^{e},
   R^{e} is -(CH₂-CH₂-O)ₙ₁-R^{k};
   n1 is an integer from 2 to 6;
   R^{k} is -H or -Me;
   Z^{s} is selected from any one of the following formulas: 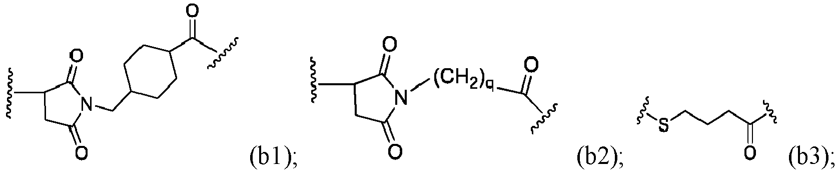 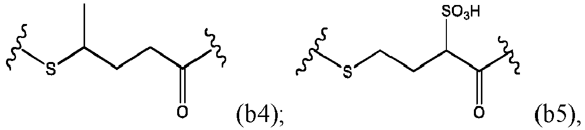 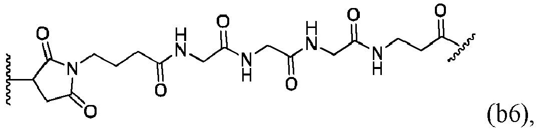 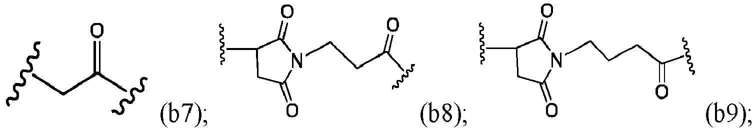 and 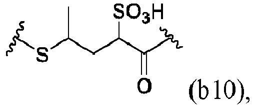
   or a pharmaceutically acceptable salt thereof, wherein q is an integer from 1 to 5, comprising the step of:
      (a) reacting a cytotoxic agent represented by the following formula: 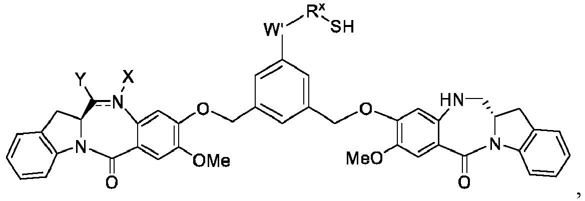
      or a pharmaceutically acceptable salt thereof, with a bifunctional crosslinking agent selected from the following: 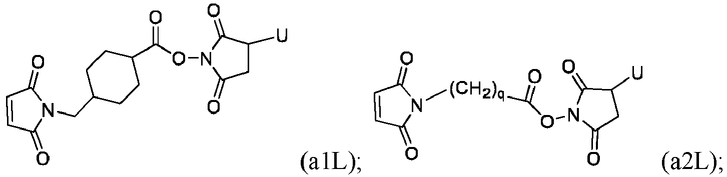 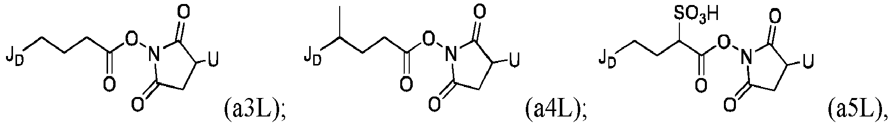 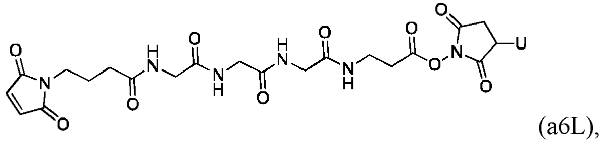 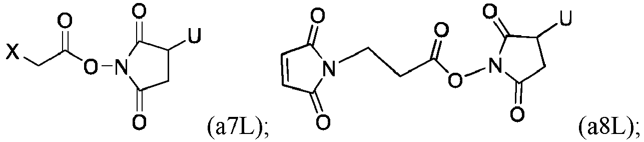 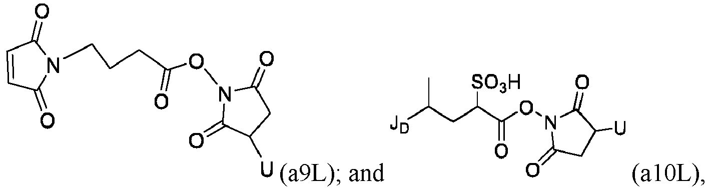 to form a cytotoxic agent-linker compound, wherein X is halogen; J_{D} -SH, -SSR^{d}, or -SC(=O)R^{g}; R^{d} is phenyl, nitrophenyl, dinitrophenyl, carboxynitrophenyl, pyridyl or nitropyridyl; R^{g} is an alkyl; q is an integer from 1 to 5; and U is -H or SO₃H; and
      (b) reacting the CBA with the cytotoxic agent-linker compound in the presence of an antioxidant to form the immunoconjugate.

In certain embodiments, the present invention provides a method of preparing an immunoconjugate represented by the following formula: 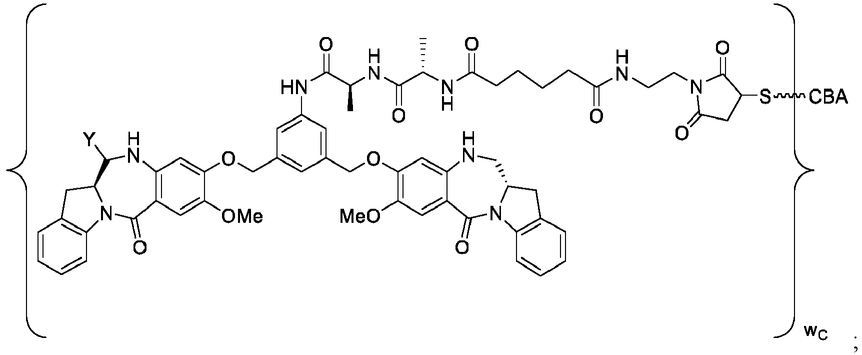 or a pharmaceutically acceptable salt thereof, comprising reacting the CBA with a cytotoxic agent represented by the following formula: 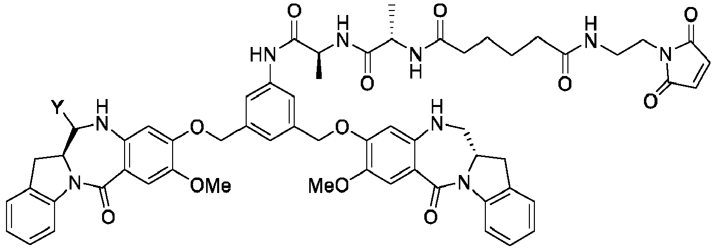 or a pharmaceutically acceptable salt thereof, in the presence of an antioxidant, wherein:
Y is -SO₃H or sodium salt thereof;
Wc is 2; and
CBA is an anti-CD123 antibody comprising: a) an immunoglobulin heavy chain having the amino acid sequence set forth in SEQ ID NO:8; and b) an immunoglobulin light chain having the amino acid sequence set forth in SEQ ID NO: 10.

In certain embodiments, the present invention provides a method of preparing an immunoconjugate represented by the following formula: 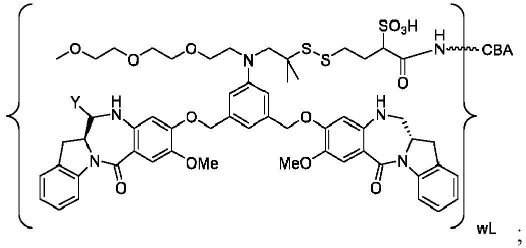 or a pharmaceutically acceptable salt thereof, comprising the steps of:
(a) reacting a cytotoxic agent represented by the following formula: 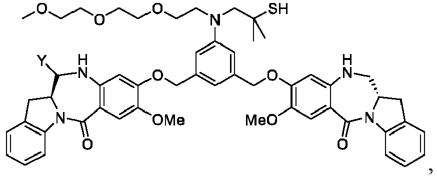 or a pharmaceutically acceptable salt thereof, with the bifunctional crosslinking agent *N-*succinimidyl-4-(2-pyridyldithio)-2-sulfobutanoate (sulfo-SPDB) represented by the following formula: 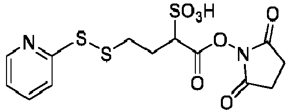 to form a cytotoxic agent-linker compound represented by the following formula: 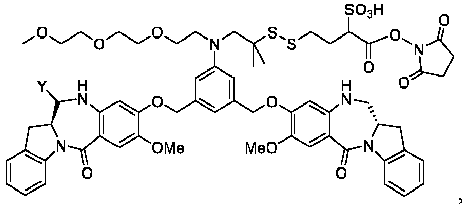 or a pharmaceutically acceptable salt thereof; and
(b) reacting the CBA with the cytotoxic agent-linker compound in the presence of an antioxidant to form the immunoconjugate, wherein:
   Y is -SO₃H;
   W_{L} is an integer from 1 to 10; and
   CBA is an anti-CD33 antibody comprising an immunoglobulin heavy chain having the amino acid sequence set forth in SEQ ID NO: 18 and an immunoglobulin light chain having the amino acid sequence set forth in SEQ ID NO:20.

It is contemplated that any one embodiment described herein, including those described only in one aspect of the invention (but not in others or not repeated in others), and those described only in the Examples, can be combined with any one or more other embodiments of the invention, unless explicitly disclaimed or inapplicable.

### BRIEF DESCRIPTION OF THE DRAWINGS

FIG. 1 shows the percentage of methionine oxidation in IMGN632 samples with and without methionine over the course of 72 hours.
FIG. 2 shows the percentage of monomer in IMGN632 samples with and without methionine over the course of 72 hours.
FIG. 3 shows the percentage of high molecular weight species in IMGN632 samples with and without methionine over the course of 72 hours.
FIG. 4 shows the amount of free drug in IMGN632 samples with and without methionine over the course of 72 hours.
FIGs. 5A and 5B show chromatograms of oxidized and native tryptic peptides detected with UPLC mass spectrometry (A) and UV detection (B). The %Met oxidation (as measured at position 252 EU numbering) is calculated as [100 x oxidized area/(oxidized area + native area)].
FIG. 6 shows the percentage of methionine oxidation in IMGN632 and IMGN779 samples with and without methione over the course of 7 days.

### DETAILED DESCRIPTION OF THE INVENTION

### 1. Definitions

To facilitate an understanding of the present invention, a number of terms and phrases are defined below.

The terms "(human) IL-3Rα," "Interleukine-3 Receptor alpha," or "CD123," as used interchangeably herein, refers to any native (human) IL-3Rα or CD123, unless otherwise indicated. The CD123 protein is an interleukin 3-specific subunit of a heterodimeric cytokine receptor (IL-3 Receptor, or IL-3R). The IL-3R is comprised of a ligand specific alpha subunit, and a signal transducing common beta subunit (also known as CD131) shared by the receptors for interleukin 3 (IL3), colony stimulating factor 2 (CSF2 / GM-CSF), and interleukin 5 (IL5). The binding of CD123/IL-3Rα to IL3 depends on the beta subunit. The beta subunit is activated by the ligand binding, and is required for the biological activities of IL3.

All of these above terms for CD 123 can refer to either a protein or nucleic acid sequence as indicated herein. The term "CD123/IL-3Rα" encompasses "full-length," unprocessed CD123/IL-3Rα, as well as any form of CD123/IL-3Rα that results from processing within the cell. The term also encompasses naturally occurring variants of CD123/IL-3Rα protein or nucleic acid, *e.g.,* splice variants, allelic variants and isoforms. The CD123/IL-3Rα polypeptides and polynucleotides described herein can be isolated from a variety of sources, such as from human tissue types or from another source, or prepared by recombinant or synthetic methods. Examples of CD123/IL-3Rα sequences include, but are not limited to NCBI reference numbers NP_002174 & NM_002183 (protein and nucleic acid sequences for human CD123 variant 1), and NP_001254642 & NM_001267713 (protein and nucleic acid sequences for human CD123 variant 2).

The term "antibody" means an immunoglobulin molecule that recognizes and specifically binds to a target, such as a protein, polypeptide, peptide, carbohydrate, polynucleotide, lipid, or combinations of the foregoing through at least one antigen recognition site within the variable region of the immunoglobulin molecule. As used herein, the term "antibody" encompasses intact polyclonal antibodies, intact monoclonal antibodies, antibody fragments (such as Fab, Fab', F(ab')₂, and Fv fragments), single chain Fv (scFv) mutants, multispecific antibodies such as bispecific antibodies, chimeric antibodies, humanized antibodies, human antibodies, fusion proteins comprising an antigen determination portion of an antibody, and any other modified immunoglobulin molecule comprising an antigen recognition site so long as the antibodies exhibit the desired biological activity. An antibody can be of any of the five major classes of immunoglobulins: IgA, IgD, IgE, IgG, and IgM, or subclasses (isotypes) thereof (*e.g.,* IgG1, IgG2, IgG3, IgG4, IgAl and IgA2), based on the identity of their heavy-chain constant domains referred to as alpha, delta, epsilon, gamma, and mu, respectively. The different classes of immunoglobulins have different and well known subunit structures and three-dimensional configurations. Antibodies can be naked or conjugated to other molecules such as toxins, radioisotopes, *etc.*

In some embodiments, an antibody is a non-naturally occurring antibody. In some embodiments, an antibody is purified from natural components. In some embodiments, an antibody is recombinantly produced. In some embodiments, an antibody is produced by a hybridoma.

A "blocking" antibody or an "antagonist" antibody is one which inhibits or reduces biological activity of the antigen it binds, such as CD123/IL-3Rα. In a certain embodiment, blocking antibodies or antagonist antibodies substantially or completely inhibit the biological activity of the antigen. Desirably, the biological activity is reduced by 10%, 20%, 30%, 50%, 70%, 80%, 90%, 95%, or even 100%.

The term "anti-CD123 antibody," "anti-IL-3Rα antibody" or "an antibody that (specifically) binds to CD123/IL-3Rα" refers to an antibody that is capable of binding CD123 / IL-3Rα with sufficient affinity such that the antibody is useful as a diagnostic and/or therapeutic agent in targeting CD123/IL-3Rα. Unless otherwise specified, the extent of binding of an anti-CD123/IL-3Rα antibody to an unrelated, non-CD123/IL-3Rα protein is less than about 10% of the binding of the antibody to CD123/IL-3Rα as measured, e.g., by a radioimmunoassay (RIA). In certain embodiments, an antibody that binds to CD123/IL-3Rα has a dissociation constant (K_{d}) of ≤0.5 nM, ≤0.3 nM, ≤0.1 nM, ≤0.05 nM, or ≤0.01 nM. In one embodiment, the anti-CD123/IL-3Rα antibody does not bind the common beta chain CD131. In one embodiment, the anti-CD123/IL-3Rα antibody does not bind to the same epitope of CD123 that is bound by the known and commercially available CD123 antibodies such as 7G3 (mouse IgG₂ₐ), 6H6 (mouse IgG₁), and 9F5 (mouse IgG₁) (Sun et al., Blood 87(1): 83-92, 1996).

The sequences of anti-CD123/IL-3Rα antibodies and antigen-binding fragments thereof of the invention are provided herein. The nomenclature for the various antibodies and immuno-conjugates of the invention are provided separately below.

The term "antibody fragment" refers to a portion of an intact antibody and refers to the antigenic determining variable regions of an intact antibody. Examples of antibody fragments include, but are not limited to, Fab, Fab', F(ab')₂, and Fᵥ fragments, linear antibodies, single chain antibodies, and multispecific antibodies formed from antibody fragments. The term "antigen-binding fragment" of an antibody includes one or more fragments of an antibody that retain the ability to specifically bind to an antigen. It has been shown that the antigen-binding function of an antibody can be performed by certain fragments of a full-length antibody. Examples of binding fragments encompassed within the term "antigen-binding fragment" of an antibody include (without limitation): (i) an Fab fragment, a monovalent fragment consisting of the V_{L}, V_{H}, C_{L}, and C_{H1} domains (*e.g.,* an antibody digested by papain yields three fragments: two antigen-binding Fab fragments, and one Fc fragment that does not bind antigen); (ii) a F(ab')₂ fragment, a bivalent fragment comprising two Fab fragments linked by a disulfide bridge at the hinge region (*e.g.,* an antibody digested by pepsin yields two fragments: a bivalent antigen-binding F(ab')₂ fragment, and a pFc' fragment that does not bind antigen) and its related F(ab') monovalent unit; (iii) a F_{d} fragment consisting of the V_{H} and C_{H1} domains (*i.e.,* that portion of the heavy chain which is included in the Fab); (iv) a Fᵥ fragment consisting of the V_{L} and V_{H} domains of a single arm of an antibody, and the related disulfide linked Fᵥ; (v) a dAb (domain antibody) or sdAb (single domain antibody) fragment (Ward et al., Nature 341:544-546, 1989), which consists of a V_{H} domain; and (vi) an isolated complementarity determining region (CDR).

A "monoclonal antibody" refers to a homogeneous antibody population involved in the highly specific recognition and binding of a single antigenic determinant, or epitope. This is in contrast to polyclonal antibodies that typically include different antibodies directed against different antigenic determinants. The term "monoclonal antibody" encompasses both intact and full-length monoclonal antibodies as well as antibody fragments (such as Fab, Fab', F(ab')₂, Fᵥ), single chain (scFv) mutants, fusion proteins comprising an antibody portion, and any other modified immunoglobulin molecule comprising an antigen recognition site. Furthermore, "monoclonal antibody" refers to such antibodies made in any number of manners including but not limited to by hybridoma, phage selection, recombinant expression, and transgenic animals.

The term "humanized antibody" refers to forms of non-human (*e.g.,* murine) antibodies that are specific immunoglobulin chains, chimeric immunoglobulins, or fragments thereof that contain minimal non-human (*e.g.,* murine) sequences. Typically, humanized antibodies are human immunoglobulins in which residues from the complementary determining region (CDR) are replaced by residues from the CDR of a non-human species (e.g., mouse, rat, rabbit, hamster) that have the desired specificity, affinity, and capability (Jones et al., Nature 321:522-525, 1986; Riechmann et al., Nature 332:323-327, 1988; Verhoeyen et al., Science 239:1534-1536, 1988).

In some instances, the Fᵥ framework region (FR) residues of a human immunoglobulin are replaced with the corresponding residues in an antibody from a non-human species that has the desired specificity, affinity, and capability. The humanized antibody can be further modified by the substitution of additional residues either in the Fᵥ framework region and/or within the replaced non-human residues to refine and optimize antibody specificity, affinity, and/or capability. In general, the humanized antibody will comprise substantially all of at least one, and typically two or three, variable domains containing all or substantially all of the CDR regions that correspond to the non-human immunoglobulin whereas all or substantially all of the FR regions are those of a human immunoglobulin consensus sequence. The humanized antibody can also comprise at least a portion of an immunoglobulin constant region or domain (F_{c}), typically that of a human immunoglobulin. Examples of methods used to generate humanized antibodies are described in U.S. Pats. 5,225,539 and 5,639,641, Roguska et al., Proc. Natl. Acad. Sci. USA 91(3):969-973, 1994; and Roguska et al., Protein Eng. 9(10):895-904, 1996 (all incorporated herein by reference). In some embodiments, a "humanized antibody" is a resurfaced antibody. In some embodiments, a "humanized antibody" is a CDR-grafted antibody.

A "variable region" of an antibody refers to the variable region of the antibody light chain or the variable region of the antibody heavy chain, either alone or in combination. The variable regions of the heavy and light chain each consist of four framework regions (FR) connected by three complementarity determining regions (CDRs) also known as hypervariable regions. The CDRs in each chain are held together in close proximity by the FRs and, with the CDRs from the other chain, contribute to the formation of the antigen-binding site of antibodies. There are at least two techniques for determining CDRs: (1) an approach based on cross-species sequence variability (*i.e.,* Kabat et al. Sequences of Proteins of Immunological Interest, 5th ed., 1991, National Institutes of Health, Bethesda Md.); and (2) an approach based on crystallographic studies of antigen-antibody complexes (Al-lazikani et al., J. Molec. Biol. 273:927-948, 1997). In addition, combinations of these two approaches are sometimes used in the art to determine CDRs.

The Kabat numbering system is generally used when referring to a residue in the variable domain (approximately residues 1-107 of the light chain and residues 1-113 of the heavy chain) *(e.g.,* Kabat et al., Sequences of Immunological Interest, 5th Ed., Public Health Service, National Institutes of Health, Bethesda, Md. (1991)).

The amino acid position numbering as in Kabat, refers to the numbering system used for heavy chain variable domains or light chain variable domains of the compilation of antibodies in Kabat et al., Sequences of Proteins of Immunological Interest, 5th Ed., Public Health Service, National Institutes of Health, Bethesda, Md. (1991) (incorporated herein by reference). Using this numbering system, the actual linear amino acid sequence can contain fewer or additional amino acids corresponding to a shortening of, or insertion into, a FR or CDR of the variable domain. For example, a heavy chain variable domain can include a single amino acid insert (residue 52a according to Kabat) after residue 52 of H2 and inserted residues *(e.g.,* residues 82a, 82b, and 82c, *etc.* according to Kabat) after heavy chain FR residue 82. The Kabat numbering of residues can be determined for a given antibody by alignment at regions of homology of the sequence of the antibody with a "standard" Kabat numbered sequence. Chothia refers instead to the location of the structural loops (Chothia and Lesk, J. Mol. Biol. 196:901-917,1987). The end of the Chothia CDR-H1 loop when numbered using the Kabat numbering convention varies between H32 and H34 depending on the length of the loop. This is because the Kabat numbering scheme places the insertions at H35A and H35B - if neither 35A nor 35B is present, the loop ends at 32; if only 35A is present, the loop ends at 33; if both 35A and 35B are present, the loop ends at 34. The AbM hypervariable regions represent a compromise between the Kabat CDRs and Chothia structural loops, and are used by Oxford Molecular's AbM antibody modeling software.

| **Loop** | **Kabat** | **AbM** | **Chiothia** |
|---|---|---|---|
| L1 | L24-L34 | L24-L34 | L24-L34 |
| L2 | L50-L56 | L50-L56 | L50-L56 |
| L3 | L89-L97 | L89-L97 | L89-L97 |
| H1 | H31-H35B | H26-H35B | H26-H32..34 |
| | | **(Kabat Numbering)** | |
| H1 | H31-H35 | H26-H35 | H26-H32 |
| | | **(Chothia Numbering)** | |
| H2 | H50-H65 | H50-H58 | H52-H56 |
| H3 | H9S-H102 | H95-H102 | H95-H102 |

The term "human antibody" means an antibody produced by a human or an antibody having an amino acid sequence corresponding to an antibody produced by a human made using any technique known in the art. In certain embodiments, the human antibody does not have non-human sequence. This definition of a human antibody includes intact or full-length antibodies, or antigen-binding fragments thereof.

The term "chimeric antibodies" refers to antibodies wherein the amino acid sequence of the immunoglobulin molecule is derived from two or more species. Typically, the variable region of both light and heavy chains corresponds to the variable region of antibodies derived from one species of mammals (*e.g.,* mouse, rat, rabbit, *etc.)* with the desired specificity, affinity, and capability while the constant regions are homologous to the sequences in antibodies derived from another (usually human) to avoid or reduce the chance of eliciting an immune response in that species (*e.g.,* human). In certain embodiments, chimeric antibody may include an antibody or antigen-binding fragment thereof comprising at least one human heavy and/or light chain polypeptide, such as, for example, an antibody comprising murine light chain and human heavy chain polypeptides.

The terms "epitope" or "antigenic determinant" are used interchangeably herein and refer to that portion of an antigen capable of being recognized and specifically bound by a particular antibody. When the antigen is a polypeptide, epitopes can be formed both from contiguous amino acids and noncontiguous amino acids juxtaposed by tertiary folding of a protein. Epitopes formed from contiguous amino acids are typically retained upon protein denaturing, whereas epitopes formed by tertiary folding are typically lost upon protein denaturing. An epitope typically includes at least 3, and more usually, at least 5 or 8-10 amino acids in a unique spatial conformation.

"Binding affinity" generally refers to the strength of the sum total of noncovalent interactions between a single binding site of a molecule *(e.g.,* an antibody) and its binding partner *(e.g.,* an antigen). Unless indicated otherwise, as used herein, "binding affinity" refers to intrinsic binding affinity which reflects a 1:1 interaction between members of a binding pair (*e.g.,* antibody and antigen). The affinity of a molecule X for its partner Y can generally be represented by the dissociation constant (K_{d}) or the half-maximal effective concentration (EC₅₀). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present invention. Specific illustrative embodiments are described herein.

"Or better" when used herein to refer to binding affinity refers to a stronger binding between a molecule and its binding partner. "Or better" when used herein refers to a stronger binding, represented by a smaller numerical K_{d} value. For example, an antibody which has an affinity for an antigen of "0.3 nM or better," the antibody's affinity for the antigen is ≤0.3 nM, *e.g.,* 0.29 nM, 0.28 nM, 0.27 nM *etc.,* or any value equal to or less than 0.3 nM. In one embodiment, the antibody's affinity as determined by a K_{d} will be between about 10⁻³ to about 10⁻¹² M, between about 10⁻⁶ to about 10⁻¹¹ M, between about 10⁻⁶ to about 10⁻¹⁰ M, between about 10⁻⁶ to about 10⁻⁹ M, between about 10⁻⁶ to about 10⁻⁸ M, or between about 10⁻⁶ to about 10⁻⁷ M.

By "specifically binds," it is generally meant that an antibody binds to an epitope via its antigen-binding domain, and that the binding entails some complementarity between the antigen-binding domain and the epitope. According to this definition, an antibody is said to "specifically bind" to an epitope when it binds to that epitope, via its antigen-binding domain more readily than it would bind to a random, unrelated epitope. The term "specificity" is used herein to qualify the relative affinity by which a certain antibody binds to a certain epitope. For example, antibody "A" may be deemed to have a higher specificity for a given epitope than antibody "B," or antibody "A" may be said to bind to epitope "C" with a higher specificity than it has for related epitope "D."

In certain embodiments, an antibody or antigen-binding fragment of the invention "specifically binds" to a CD 123 antigen, in that it has a higher binding specificity to the CD 123 antigen (from any species) than that to a non-CD 123 antigen. In certain embodiments, an antibody or antigen-binding fragment of the invention "specifically binds" to a human CD123 antigen, in that it has a higher binding specificity to the human CD 123 antigen than that to a non-human CD123 antigen (*e.g.,* a mouse or a rat CD123).

By "preferentially binds," it is meant that the antibody specifically binds to an epitope more readily than it would bind to a related, similar, homologous, or analogous epitope. Thus, an antibody which "preferentially binds" to a given epitope would more likely bind to that epitope than to a related epitope, even though such an antibody may cross-react with the related epitope. For example, in certain embodiments, an antibody or antigen-binding fragment of the invention "preferentially binds" to a human CD 123 antigen over a mouse CD123.

An antibody is said to "competitively inhibit" binding of a reference antibody to a given epitope if it preferentially binds to that epitope to the extent that it blocks, to some degree, binding of the reference antibody to the epitope. Competitive inhibition may be determined by any method known in the art, for example, competition ELISA assays. An antibody may be said to competitively inhibit binding of the reference antibody to a given epitope by at least 90%, at least 80%, at least 70%, at least 60%, or at least 50%.

The phrase "substantially similar," or "substantially the same," as used herein, denotes a sufficiently high degree of similarity between two numeric values (generally one associated with an antibody of the invention and the other associated with a reference/comparator antibody) such that one of skill in the art would consider the difference between the two values to be of little or no biological and/or statistical significance within the context of the biological characteristics measured by said values (*e.g.,* K_{d} values). The difference between said two values is less than about 50%, less than about 40%, less than about 30%, less than about 20%, or less than about 10% as a function of the value for the reference/comparator antibody.

The term "immunoconjugate," "conjugate," or "ADC" as used herein refers to a compound or a derivative thereof that is linked to a cell binding agent (*i*.*e*., an anti-CD123/IL-3Rα antibody or fragment thereof) and is defined by a generic formula: A-L-C, wherein C = cytotoxin, L = linker, and A = cell binding agent (CBA), such as anti-CD123/IL-3Rα antibody or antibody fragment. Immunoconjugates can also be defined by the generic formula in reverse order: C-L-A.

A "linker" is any chemical moiety that is capable of linking a compound, usually a drug, such as a cytotoxic agent described herein (*e.g.,* IGN (indolinobenzodiazepine) compounds), to a cell-binding agent such as an antibody or a fragment thereof in a stable, covalent manner. Linkers can be susceptible to or be substantially resistant to acid-induced cleavage, light-induced cleavage, peptidase-induced cleavage, esterase-induced cleavage, and disulfide bond cleavage, at conditions under which the compound or the antibody remains active. Suitable linkers are well known in the art and include, for example, disulfide groups, thioether groups, acid labile groups, photolabile groups, peptidase labile groups and esterase labile groups. Linkers also include charged linkers, and hydrophilic forms thereof as described herein and know in the art.

The terms "cancer" and "cancerous" refer to or describe the physiological condition in mammals in which a population of cells are characterized by unregulated cell growth. "Tumor" and "neoplasm" refer to one or more cells that result from excessive cell growth or proliferation, either benign (noncancerous) or malignant (cancerous) including pre-cancerous lesions.

Examples of cancer include lymphoma and leukemia. Examples of cancer or tumorigenic diseases which can be treated and/or prevented by the methods and reagents (*e.g.,* anti-CD123 antibody, antigen-binding fragment thereof, or immuno-conjugate thereof) of the invention include AML, CML, ALL (*e.g.,* B-ALL), CLL, myelodysplastic syndrome, basic plasmacytoid DC neoplasm (BPDCN) leukemia, B-cell lymphomas including non-Hodgkin lymphomas (NHL), precursor B-cell lymphoblastic leukemia / lymphoma and mature B-cell neoplasms, such as B-cell chronic lymphocytic leukemia (B-CLL) / small lymphocytic lymphoma (SLL), B-cell prolymphocytic leukemia, lymphoplasmacytic lymphoma, mantle cell lymphoma (MCL), follicular lymphoma (FL), including low-grade, intermediate-grade and high-grade FL, cutaneous follicle center lymphoma, marginal zone B-cell lymphoma (MALT type, nodal and splenic type), hairy cell leukemia (HCL), diffuse large B-cell lymphoma (DLBCL), Burkitt's lymphoma, plasmacytoma, plasma cell myeloma, post-transplant lymphoproliferative disorder, Waldenstrom's macroglobulinemia, anaplastic large-cell lymphoma (ALCL), and Hodgkin's leukemia (HL).

The term "subject" refers to any animal (*e.g.,* a mammal), including, but not limited to humans, non-human primates, rodents, and the like, which is to be the recipient of a particular treatment. Typically, the terms "subject" and "patient" are used interchangeably herein in reference to a human subject.

The term "pharmaceutical formulation" refers to a preparation which is in such form as to permit the biological activity of the active ingredient to be effective, and which contains no additional components which are unacceptably toxic to a subject to which the formulation would be administered. Such formulation can be sterile.

An "effective amount" of an antibody or immunoconjugate as disclosed herein is an amount sufficient to carry out a specifically stated purpose. An "effective amount" can be determined empirically and in a routine manner, in relation to the stated purpose.

"Alkyl" as used herein refers to a saturated linear or branched-chain monovalent hydrocarbon radical of one to twenty carbon atoms. Examples of alkyl include, but are not limited to, methyl, ethyl, 1-propyl, 2-propyl, 1-butyl, 2-methyl-1-propyl, -CH₂CH(CH₃)₂), 2-butyl, 2-methyl-2-propyl, 1-pentyl, 2-pentyl 3-pentyl, 2-methyl-2-butyl, 3-methyl-2-butyl, 3-methyl-1-butyl, 2-methyl-1-butyl, 1-hexyl), 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl, 1-heptyl, 1-octyl, and the like. Preferably, the alkyl has one to ten carbon atoms. More preferably, the alkyl has one to four carbon atoms.

The number of carbon atoms in a group can be specified herein by the prefix "Cₓ₋ₓₓ", wherein x and xx are integers. For example, "C₁₋₄alkyl" is an alkyl group having from 1 to 4 carbon atoms.

The term "compound" or "cytotoxic compound," are used interchangeably. They are intended to include compounds for which a structure or formula or any derivative thereof has been disclosed in the present invention or a structure or formula or any derivative thereof that has been incorporated by reference. The term also includes, stereoisomers, geometric isomers, tautomers, solvates, metabolites, and salts (*e.g.,* pharmaceutically acceptable salts) of a compound of all the formulae disclosed in the present invention. The term also includes any solvates, hydrates, and polymorphs of any of the foregoing. The specific recitation of "stereoisomers," "geometric isomers," "tautomers," "solvates," "metabolites," "salt", "conjugates," "conjugates salt," "solvate," "hydrate," or "polymorph" in certain aspects of the invention described in this application shall not be interpreted as an intended omission of these forms in other aspects of the invention where the term "compound" is used without recitation of these other forms.

The term "chiral" refers to molecules that have the property of non-superimposability of the mirror image partner, while the term "achiral" refers to molecules that are superimposable on their mirror image partner.

The term "stereoisomer" refers to compounds that have identical chemical constitution and connectivity, but different orientations of their atoms in space that cannot be interconverted by rotation about single bonds.

"Diastereomer" refers to a stereoisomer with two or more centers of chirality and whose molecules are not mirror images of one another. Diastereomers have different physical properties, *e.g.* melting points, boiling points, spectral properties, and reactivities. Mixtures of diastereomers can separate under high resolution analytical procedures such as crystallization, electrophoresis and chromatography.

"Enantiomers" refer to two stereoisomers of a compound that are non-superimposable mirror images of one another.

Stereochemical definitions and conventions used herein generally follow S. P. Parker, Ed., McGraw-Hill, Dictionary of Chemical Terms (1984) McGraw-Hill Book Company, New York; and Eliel, E. and Wilen, S., Stereochemistry of Organic Compounds, John Wiley & Sons, Inc., New York, 1994. The compounds of the invention can contain asymmetric or chiral centers, and therefore exist in different stereoisomeric forms. It is intended that all stereoisomeric forms of the compounds of the invention, including but not limited to, diastereomers, enantiomers and atropisomers, as well as mixtures thereof such as racemic mixtures, form part of the present invention. Many organic compounds exist in optically active forms, *i.e.,* they have the ability to rotate the plane of plane-polarized light. In describing an optically active compound, the prefixes D and L, or R and S, are used to denote the absolute configuration of the molecule about its chiral center(s). The prefixes d and I or (+) and (-) are employed to designate the sign of rotation of plane-polarized light by the compound, with (-) or 1 meaning that the compound is levorotatory. A compound prefixed with (+) or d is dextrorotatory. For a given chemical structure, these stereoisomers are identical except that they are mirror images of one another. A specific stereoisomer can also be referred to as an enantiomer, and a mixture of such isomers is often called an enantiomeric mixture. A 50:50 mixture of enantiomers is referred to as a racemic mixture or a racemate, which can occur where there has been no stereoselection or stereospecificity in a chemical reaction or process. The terms "racemic mixture" and "racemate" refer to an equimolar mixture of two enantiomeric species, devoid of optical activity.

The term "tautomer" or "tautomeric form" refers to structural isomers of different energies that are interconvertible via a low energy barrier. For example, proton tautomers (also known as prototropic tautomers) include interconversions via migration of a proton, such as keto-enol and imine-enamine isomerizations. Valence tautomers include interconversions by reorganization of some of the bonding electrons.

The phrase "pharmaceutically acceptable salt" as used herein, refers to pharmaceutically acceptable organic or inorganic salts of a compound of the invention. Exemplary salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate "mesylate," ethanesulfonate, benzenesulfonate, p-toluenesulfonate, pamoate (*i.e.,* 1,1'-methylene-bis-(2-hydroxy-3-naphthoate)) salts, alkali metal (*e.g.,* sodium and potassium) salts, alkaline earth metal (*e.g.,* magnesium) salts, and ammonium salts. A pharmaceutically acceptable salt can involve the inclusion of another molecule such as an acetate ion, a succinate ion or other counter ion. The counter ion can be any organic or inorganic moiety that stabilizes the charge on the parent compound. Furthermore, a pharmaceutically acceptable salt can have more than one charged atom in its structure. Instances where multiple charged atoms are part of the pharmaceutically acceptable salt can have multiple counter ions. Hence, a pharmaceutically acceptable salt can have one or more charged atoms and/or one or more counter ion.

If the compound of the invention is a base, the desired pharmaceutically acceptable salt can be prepared by any suitable method available in the art, for example, treatment of the free base with an inorganic acid, such as hydrochloric acid, hydrobromic acid, sulfuric acid, nitric acid, methanesulfonic acid, phosphoric acid and the like, or with an organic acid, such as acetic acid, maleic acid, succinic acid, mandelic acid, fumaric acid, malonic acid, pyruvic acid, oxalic acid, glycolic acid, salicylic acid, a pyranosidyl acid, such as glucuronic acid or galacturonic acid, an alpha hydroxy acid, such as citric acid or tartaric acid, an amino acid, such as aspartic acid or glutamic acid, an aromatic acid, such as benzoic acid or cinnamic acid, a sulfonic acid, such as p-toluenesulfonic acid or ethanesulfonic acid, or the like.

If the compound of the invention is an acid, the desired pharmaceutically acceptable salt can be prepared by any suitable method, for example, treatment of the free acid with an inorganic or organic base, such as an amine (primary, secondary or tertiary), an alkali metal hydroxide or alkaline earth metal hydroxide, or the like. Illustrative examples of suitable salts include, but are not limited to, organic salts derived from amino acids, such as glycine and arginine, ammonia, primary, secondary, and tertiary amines, and cyclic amines, such as piperidine, morpholine and piperazine, and inorganic salts derived from sodium, calcium, potassium, magnesium, manganese, iron, copper, zinc, aluminum and lithium.

As used herein, the term "solvate" means a compound that further includes a stoichiometric or non-stoichiometric amount of solvent such as water, isopropanol, acetone, ethanol, methanol, DMSO, ethyl acetate, acetic acid, and ethanolamine dichloromethane, 2-propanol, or the like, bound by non-covalent intermolecular forces. Solvates or hydrates of the compounds are readily prepared by addition of at least one molar equivalent of a hydroxylic solvent such as methanol, ethanol, 1-propanol, 2-propanol or water to the compound to result in solvation or hydration of the imine moiety.

The phrase "pharmaceutically acceptable" indicates that the substance or composition must be compatible chemically and/or toxicologically, with the other ingredients comprising a formulation, and/or the mammal being treated therewith.

The term "amino acid" refers to naturally occurring amino acids or non-naturally occurring amino acid. In one embodiment, the amino acid is represented by NH₂-C(R^{aa'}R^{aa})-C(=O)OH, wherein R^{aa} and R^{aa'} are each independently H, an optionally substituted linear, branched or cyclic alkyl, alkenyl or alkynyl having 1 to 10 carbon atoms, aryl, heteroaryl or heterocyclyl or R^{aa} and the N-terminal nitrogen atom can together form a heteroycyclic ring *(e.g.,* as in proline). The term "amino acid residue" refers to the corresponding residue when one hydrogen atom is removed from the amine and/or carboxy end of the amino acid, such as -NH-C(R^{aa'}R^{aa})-C(=O)O-.

The term "cation" refers to an ion with positive charge. The cation can be monovalent (*e.g.,* Na⁺, K⁺, NH₄⁺ *etc.),* bi-valent (*e.g.,* Ca²⁺, Mg²⁺, *etc.)* or multi-valent (*e.g.,* Al³⁺ *etc.).* Preferably, the cation is monovalent.

The term "methionine oxidation" refers to the oxidation of one or more methionine residues located on the cell-binding agent *(e.g.,* an antibody or antigen-binding fragment thereof). In certain embodiments, oxidation occurs at one or more methionine residues located at the Fc region of an antibody (e.g., at Met252, Met358, Met428 by EU numbering). In certain embodiment, methionine oxidation, particularly methionine oxidation at the Fc region of an antibody, may reduce antibody binding to neonatal Fc receptor (FcRn), which in turn, may affect the circulation half-life of the antibody.

It is understood that wherever embodiments are described herein with the language "comprising," otherwise analogous embodiments described in terms of "consisting of" and/or "consisting essentially of" are also provided.

The term "and/or" as used in a phrase such as "A and/or B" herein is intended to include both "A and B," "A or B," "A," and "B." Likewise, the term "and/or" as used in a phrase such as "A, B, and/or C" is intended to encompass each of the following embodiments: A, B, and C; A, B, or C; A or C; A or B; B or C; A and C; A and B; B and C; A (alone); B (alone); and C (alone).

### Antibodies, Compounds, and Immunoconjugates Nomenclature

As used herein, the nomenclature used for the antibodies, cytotoxic compounds, and their immunoconjugates generally adopt the following meanings.

Exemplary antibodies or antigen-binding fragment thereof of the present invention are shown in table below. For example, G4723A antibody is a humanized anti-CD123 antibody with a cysteine at EU numbering position 442, having a heavy chain full length sequence of SEQ ID NO:8; and a light chain full length sequence of SEQ ID NO: 10.

| **Name** | **Sequence** |
|---|---|
| huCD123-6 LC-CDR1 | RASQDINSYLS (SEQ ID NO: 1) |
| huCD123-6 LC-CDR2 | RVNRLVD (SEQ ID NO:2) |
| huCD123-6 LC-CDR3 | LQYDAFPYT (SEQ ID NO: 3) |
| huCD123-6 HC-CDR1 | SSIMH (SEQ ID NO:4) |
| huCD123-6 HC-CDR2 | YIKPYNDGTKYNEKFKG (SEQ ID NO:5) |
| huCD123-6 HC-CDR3 | EGGNDYYDTMDY (SEQ ID NO:6) |
| huCD123-6Gv7 Heavy Chain Variable Region | QVQLVQSGAEVKKPGASVKVSCKASGYIFTSSIMH WVRQAPGQGLEWIGYIKPYNDGTKYNEKFKGRA TLTSDRSTSTAYMELSSLRSEDTAVYYCAREGGND |
| | YYDTMDYWGQGTLVTVSS (SEQ ID NO:7) |
| huCD123-6Gv7-C442 Heavy Chain Full Length | QVQLVQSGAEVKKPGASVKVSCKASGYIFTSSIMH WVRQAPGQGLEWIGYIKPYNDGTKYNEKFKGRA TLTSDRSTSTAYMELSSLRSEDTAVYYCAREGGND YYDTMDYWGQGTLVTVSSASTKGPSVFPLAPSSKS TSGGTAALGCLVKDYFPEPVTVSWNSGALTSGVHT FPAVLQSSGLYSLSSVVTVPSSSLGTQTYICNVNHKP SNTKVDKKVEPKSCDKTHTCPPCPAPELLGGPSVFL FPPKPKDTLMISRTPEVTCVVVDVSHEDPEVKFNW YVDGVEVHNAKTKPREEQYNSTYRVVSVLTVLHQ DWLNGKEYKCKVSNKALPAPIEKTISKAKGQPREP QVYTLPPSRDELTKNQVSLTCLVKGFYPSDIAVEWE SNGQPENNYKTTPPVLDSDGSFFLYSKLTVDKSRW QQGNVFSCSVMHEALHNHYTQKSLCLSPG (SEQ ID NO:8) |
| huCD123-6Gv4 Light Chain Variable Region | DIQMTQSPSSLSASVGDRVTITCRASQDINSYLSWF QQKPGKAPKTLIYRVNRLVDGVPSRFSGSGSGNDY TLTISSLQPEDFATYYCLQYDAFPYTFGQGTKVEIK R (SEQ ID NO:9) |
| huCD123-6Gv4 Light Chain Full Length | DIQMTQSPSSLSASVGDRVTITCRASQDINSYLSWF QQKPGKAPKTLIYRVNRLVDGVPSRFSGSGSGNDY TLTISSLQPEDFATYYCLQYDAFPYTFGQGTKVEIK RTVAAPSVFIFPPSDEQLKSGTASVVCLLNNFYPRE AKVQWKVDNALQSGNSQESVTEQDSKDSTYSLSST LTLSKADYEKHKVYACEVTHQGLSSPVTKSFNRGE C (SEQ ID NO:10) |

| **Name** | **Sequence** |
|---|---|
| huMy9-6 LC-CDR1 | KSSQSVFFSSSQKNYLA (SEQ ID NO:11) |
| huMy9-6 LC-CDR2 | WASTRES (SEQ ID NO:12) |
| huMy9-6 LC-CDR3 | HQYLSSRT (SEQ ID NO:13) |
| huMy9-6 HC-CDR1 | SYYIH (SEQ ID NO:14) |
| huMy9-6 HC-CDR2 | VIYPGNDDISYNQKFQG (SEQ ID NO:15) |
| huMy9-6 HC-CDR3 | EVRLRYFDV (SEQ ID NO:16) |
| huMy9-6 Heavy Chain Variable Region | QVQLQQPGAEVVKPGASVKMSCKASGYTFTSYYIHWIKQTPGQGLE WVGVIYPGNDDISYNQKFQGKATLTADKSSTTAYMQLSSLTSEDSA VYYCAREVRLRYFDVWGQGTTVTVSS (SEQ ID NO:17) |
| huMy9-6 Heavy Chain Full Length | QVQLQQPGAEVVKPGASVKMSCKASGYTFTSYYIHWIKQTPGQGLE WVGVIYPGNDDISYNQKFQGKATLTADKSSTTAYMQLSSLTSEDSA VYYCAREVRLRYFDVWGQGTTVTVSSASTKGPSVFPLAPSSKSTSG GTAALGCLVKDYFPEPVTVSWNSGALTSGVHTFPAVLQSSGLYSLSS VVTVPSSSLGTQTYICNVNHKPSNTKVDKKVEPKSCDKTHTCPPCPA PELLGGPSVFLFPPKPKDTLMISRTPEVTCVVVDVSHEDPEVKFNWY VDGVEVHNAKTKPREEQYNSTYRVVSVLTVLHQDWLNGKEYKCKV SNKALPAPIEKTISKAKGQPREPQVYTLPPSRDELTKNQVSLTCLVKG FYPSDIAVEWESNGQPENNYKTTPPVLDSDGSFFLYSKLTVDKSRWQ QGNVFSCSVMHEALHNHYTQKSLSLSPG (SEQ ID NO:18) |
| huMy9-6 Light Chain Variable Region | EIVLTQSPGSLAVSPGERVTMSCKSSQSVFFSSSQKNYLAWYQQIPG QSPRLLIYWASTRESGVPDRFTGSGSGTDFTLTISSVQPEDLAIYYCH QYLSSRTFGQGTKLEIKR (SEQ ID NO:19) |
| huMy9-6 Light Chain Full Length | EIVLTQSPGSLAVSPGERVTMSCKSSQSVFFSSSQKNYLAWYQQIPG QSPRLLIYWASTRESGVPDRFTGSGSGTDFTLTISSVQPEDLAIYYCH QYLSSRTFGQGTKLEIKRTVAAPSVFIFPPSDEQLKSGTASVVCLLNN FYPREAKVQWKVDNALQSGNSQESVTEQDSKDSTYSLSSTLTLSKA DYEKHKVYACEVTHQGLSSPVTKSFNRGEC (SEQ ID NO:20) |

The antibodies or antigen-binding fragments thereof of the invention may be conjugated to certain cytotoxic agents, either through linkage with the Lys side chain amino group, or the Cys side chain thiol group. Certain representative (non-limiting) cytotoxic agents and immunoconjugates described in the specification (including the examples) are listed below for illustration purpose. Note that most compounds such as DGN462, DGN549-C, and DGN549-L may be sulfonated (shown as sDGN462 and sDGN549-C respectively).

| Compound Name | Structure |
|---|---|
| DGN462 | 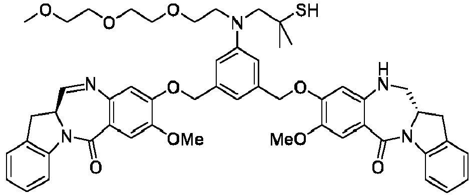 |
| sDGN462 | 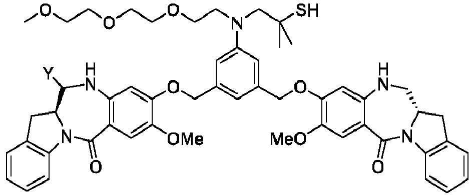 |
| IMGN779 | 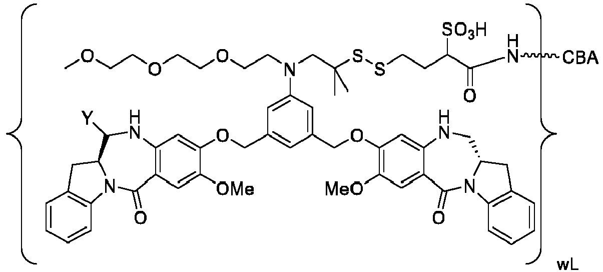 |
| | (CBA = an anti-CD33 antibody comprising an immunoglobulin heavy chain having the amino acid sequence set forth in SEQ ID NO: 18 and an immunoglobulin light chain having the amino acid sequence set forth in SEQ ID NO:20) |
| DGN549-C | 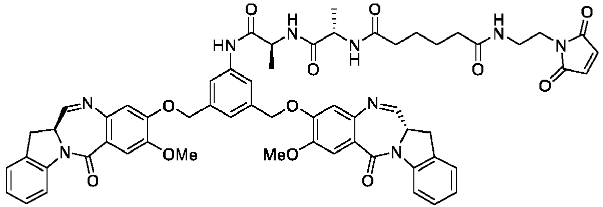 |
| sDGN549-C | 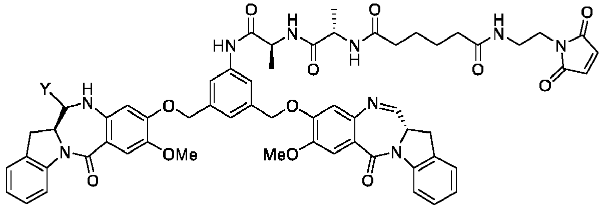 |
| IMGN632 | 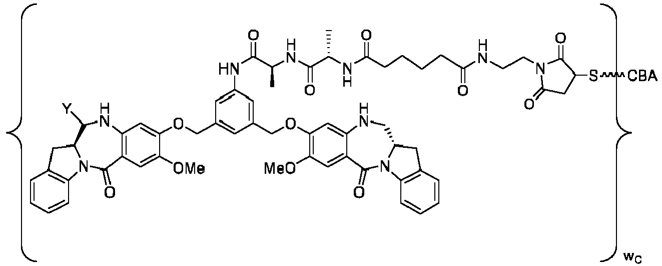 |
| | (CBA= an anti-CD123 antibody having a heavy chain full length sequence of SEQ ID NO: 8; and a light chain full length sequence of SEQ ID NO:10, also referred to as G4723A antibody) |
| DGN549-L | 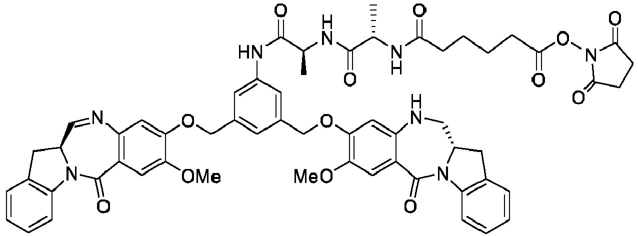 |
| sDGN549-L | 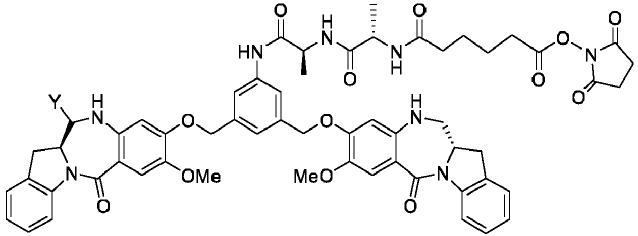 |
| Ab-sDGN549-L | 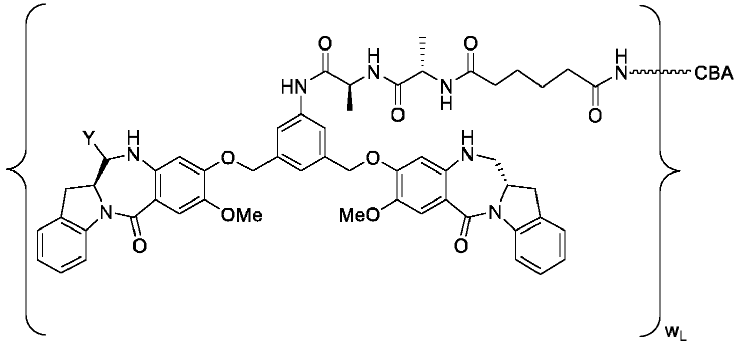 (CBA = an antibody) |

Y is -SO₃H or sodium salt thereof; Wc is 2; and W_{L} is an integer from 1 to 10.

### 2. Pharmaceutical Compositions

In a first aspect, the present invention provides a pharmaceutical composition comprising an immunoconjugate described herein (e.g., immunoconjugate of the 1^{st} to 26^{th} specific embodiment described below) and 0.1 mM to 20 mM methionine. It is surprisingly found that significant amount of methionine oxidation occurs during the preparation and/or storage of the immunoconjugates of the present invention. In particular, light exposure over an extended period, such as over 1, 2, 3, 4, 5, 6, 7, 8, 9, 10, 15, 24, 48, 72 hours or longer, results in large amount of methionine oxidation in the immunoconjugates. Under similar conditions, no significant amount of methionine oxidation is observed for the corresponding naked antibodies upon light exposure and/or storage. The presence of methionine in the pharmaceutical composition of the present invention comprising the immunoconjugates described herein reduces the amount of methionine oxidation in the immunoconjugates as compared to pharmaceutical compositions without methionine, in particular upon light exposure.

In a first embodiment, the pharmaceutical composition of the present invention comprises an immunoconjugate described herein (*e.g.,* immunoconjugates of formula (IA), (IB) or (IC), or immunoconjugate of the 1^{st} to 26^{th} specific embodiment described below) and 0.1 mM to 10 mM methionine.

In certain embodiments, the pharmaceutical composition of the present invention comprises an immunoconjugate described herein (*e.g.,* immunoconjugates of formula (IA), (IB) or (IC), or immunoconjugate of the 1^{st} to 26^{th} specific embodiment described below) and 0.5 mM to 5 mM methionine.

In certain embodiments, the pharmaceutical composition of the present invention comprises an immunoconjugate described herein (*e.g.,* immunoconjugates of formula (IA), (IB) or (IC), or immunoconjugate of the 1^{st} to 26^{th} specific embodiment described below) and 1.0 mM to 4.0 mM methionine. In certain embodiments, the methionine concentration in the pharmaceutical composition is 3 mM.

In certain embodiments, when the pharmaceutical composition of the present invention described above is exposed to light at room temperature for 6 hours or more, the immunoconjugate has less than 50%, less than 40%, less than 35%, 30%, less than 25%, less than 20% or less than 15%, less than 10%, less than 5%, less than 4%, less than 3%, less than 2% or less than 1% of methionine oxidation.

In a second embodiment, the pharmaceutical composition of the present invention comprises 1 mM to 4 mM of methionine and an immunoconjugate represented by the following formula: 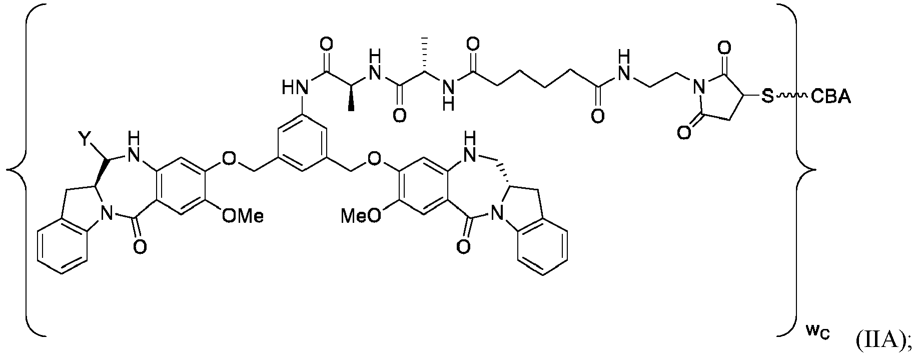 or a pharmaceutically acceptable salt thereof, wherein:
Y is -SO₃H or sodium salt thereof;
Wc is 2; and
CBA is an anti-CD123 antibody comprising: a) an immunoglobulin heavy chain having the amino acid sequence set forth in SEQ ID NO:8; and b) an immunoglobulin light chain having the amino acid sequence set forth in SEQ ID NO: 10.

In a third embodiment, the pharmaceutical composition of the present invention comprises 1 mM to 4 mM of methionine and an immunoconjugate represented by the following formula: 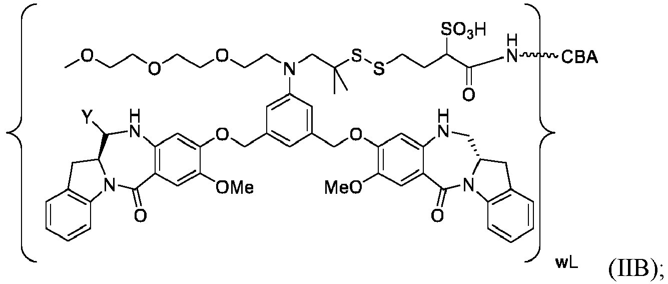 or a pharmaceutically acceptable salt thereof, wherein:
Y is -SO₃H or sodium salt thereof;
W_{L} is an integer from 1 to 10; and
CBA is an anti-CD33 antibody comprising an immunoglobulin heavy chain having the amino acid sequence set forth in SEQ ID NO: 18 and an immunoglobulin light chain having the amino acid sequence set forth in SEQ ID NO:20.

In a fourth embodiment, the pharmaceutical composition of the first, second or third embodiment comprises 3 mM methionine.

In a fifth embodiment, when the pharmaceutical composition of the first, second, third, or fourth embodiment is exposed to light at room temperature for 6 hours, the immunoconjugate has less than 15%, less than 10%, less than 5%, less than 4%, less than 3%, less than 2%, or less than 1% of methionine oxidation.

In a sixth embodiment, the pharmaceutical composition of the first, second, third, fourth or fifth embodiment is in a container that protects the pharmaceutical composition from light exposure. Any suitable container (e.g., vial or syringe) can be used. For example, light-resistant (e.g., amber, yellow-green or blue) container, such as colored-glass or colored plastic container (e.g., vial or syringe), can be used to minimize light exposure. Alternatively, a colorless or translucent container can be used if it is protected by a light-resistant opaque covering, such as a paper carton, plastic box, or aluminum foil. In addition, any container, filter, or vial that blocks light below 400-435 nm to prevent lights of similar wavelength to the absorption spectrum of the cytotoxic payload (e.g., DGN462, DGN549) from entering the pharmaceutical composition are useful in the invention. In addition, light absorbing additives can be added to the formulation to protect the pharmaceutical composition if exposed to light.

In a seventh embodiment, the pharmaceutical composition of the first, second, third, fourth, fifth or sixth embodiment comprises 1 mg/mL to 10 mg/mL, 1 mg/mL to 5 mg/mL, 1 mg/mL to 3 mg/mL, or 1.5 mg/mL to 2.5 mg/mL of the immunoconjugate. In certain embodiments, the pharmaceutical composition of the second, third, fourth, fifth or sixth embodiment comprises 2 mg/mL of the immunoconjugate.

In an eighth embodiment, the pharmaceutical composition of the first, second, third, fourth, fifth, sixth or seventh embodiment further comprises sodium bisulfite. In certain embodiments, the concentration for the sodium bisulfite in the pharmaceutical compositions is 10 µM to 100 µM, 20 µM to 90 µM, 30 µM to 80 µM, 30 µM to 70 µM, 40 µM to 60 µM, or 45 µM to 55 µM of sodium bisulfite. In certain embodiments, the pharmaceutical composition of the second, third, fourth, fifth, sixth or seventh embodiment further comprises 50 µM of sodium bisulfite.

In a ninth embodiment, the pharmaceutical composition of the first, second, third, fourth, fifth, sixth, seventh, or eighth embodiment further comprises one or more pharmaceutically acceptable vehicle (e.g. carrier, excipient) (Remington, The Science and Practice of Pharmacy 20th Edition Mack Publishing, 2000). Suitable pharmaceutically acceptable vehicles include, but are not limited to, nontoxic buffers such as phosphate, citrate, succinate, histidine and other organic acids; salts such as sodium chloride; preservatives (e.g., octadecyldimethylbenzyl ammonium chloride; hexamethonium chloride; benzalkonium chloride; benzethonium chloride; phenol, butyl or benzyl alcohol; alkyl parabens, such as methyl or propyl paraben; catechol; resorcinol; cyclohexanol; 3-pentanol; and m-cresol); low molecular weight polypeptides (e.g., less than about 10 amino acid residues); proteins such as serum albumin, gelatin, or immunoglobulins; hydrophilic polymers such as polyvinylpyrrolidone; amino acids such as glycine, glutamine, asparagine, histidine, arginine, or lysine; carbohydrates such as monosaccharides, disaccharides, glucose, mannose, or dextrins; chelating agents such as EDTA; sugars such as sucrose, mannitol, trehalose or sorbitol; salt-forming counter-ions such as sodium; metal complexes (e.g., Zn-protein complexes); and non-ionic surfactants such as TWEEN or polyethylene glycol (PEG).

In certain embodiments, the pharmaceutical composition of the first, second, third, fourth, fifth, sixth, seventh or eighth embodiment further comprises sodium bisulfite, buffer, sugar and non-ionic surfactant. In certain embodiments, the pharmaceutically composition of the present invention further comprises sodium bisulfite, succinate or histidine buffer, trehalose and polysorbate 20.

In certain embodiments, the buffer (e.g., succinate or histidine) concentration in the pharmaceutical composition is in the range of 5 mM to 50 mM, 5 mM to 25 mM, 5 mM to 15 mM, 10 mM to 25 mM, or 15 mM to 25 mM. In certain embodiments, the buffer concentration is 10 mM or 20 mM.

In certain embodiments, the sugar (e.g., trehalose) concentration in the pharmaceutical composition is in the range of 5-10%, 6-8%, 6.5-7.5%, 7.0-7.4%, 7.1-7.3% (w/v). In certain embodiments, the sugar (e.g., trehalose) concentration in the pharmaceutical composition is 7.2%, (w/v).

In certain embodiments, the non-ionic surfactant (e.g., polysorbate 20) concentration in the pharmaceutical composition is in the range of 0.01-0.1 %, 0.01-0.05 %, or 0.01-0.03%

(w/v). In certain embodiments, the non-ionic surfactant (e.g., polysorbate 20) concentration in the pharmaceutical composition is 0.02% (w/v)

In a tenth embodiment, the pharmaceutical composition of the first, second, fourth, fifth, sixth, seventh, eighth or ninth embodiment has a pH of 4 to 5, 4 to 4.5, 4 to 4.4, or 4.1 to 4.3. In certain embodiments, the pH is 4.2.

In an eleventh embodiment, the pharmaceutical composition of the first, third, fourth, fifth, sixth, seventh, eighth or ninth embodiment has a pH of 5.5 to 6.5, 5.9 to 6.3 or 6.0 to 6.2. In certain embodiments, the pH is 6.1.

In a twelfth embodiment, the pharmaceutical composition of the present invention comprises 2 mg/mL of the immunoconjugate of formula (IIA), 3 mM methionine, 10 mM succinate, 50 µM sodium bisulfite, 7.2% (w/v) trehalose (or 8.0% (w/v) trehalose dihydrate), and 0.01% (w/v) polysorbate 20 and the pH of the pharmaceutical composition is 4.2.

In a thirteenth embodiment, the pharmaceutical composition of the present invention comprises 2 mg/mL of the immunoconjugate of formula (IIB), 3 mM methionine, 20 mM histidine, 50 µM sodium bisulfite, 7.2% (w/v) trehalose (or 8.0% (w/v) trehalose dihydrate), 0.02% (w/v) polysorbate 20 and the pH of the pharmaceutical composition is 6.1.

### 3. Methods of Reducing Methionine Oxidation

In a second aspect, the present invention provides a method of reducing the amount of methionine oxidation in an immunoconjuate described herein (e.g., immunoconjugates of formula (IA), (IB) or (IC), or immunoconjugate of the 1^{st} to 26^{th} specific embodiment described below) comprising mixing the immunoconjugate with 0.1 mM to 20 mM methionine to give a pharmaceutical composition comprising the immunoconjugate and methionine.

In a first embodiment, the method of the second aspect comprises mixing the immunoconjuate described herein (*e.g.,* immunoconjugates of formula (IA), (IB) or (IC), or immunoconjugate of the 1^{st} to 26^{th} specific embodiment described below) with 0.5 mM to 5 mM methionine.

In certain embodiments, the method of the second aspect comprises mixing the immunoconjugate described herein (*e.g.,* immunoconjugates of formula (IA), (IB) or (IC), or immunoconjugate of the 1^{st} to 26^{th} specific embodiment described below) with 1.0 mM to 4.0 mM methionine.

In certain embodiments, when the pharmaceutical composition prepared by the method of the second aspect of the present invention described above is exposed to light at room temperature for 6 hours or more, the immunoconjugate has less than 50%, less than 40%, less than 35%, 30%, less than 25%, less than 20% or less than 15%, less than 10%, less than 5%, less than 4%, less than 3%, less than 2% or less than 1% of methionine oxidation.

In a second embodiment, the method of the second aspect comprises mixing 1 mM to 4 mM of methionine and an immunoconjugate represented by the following formula: 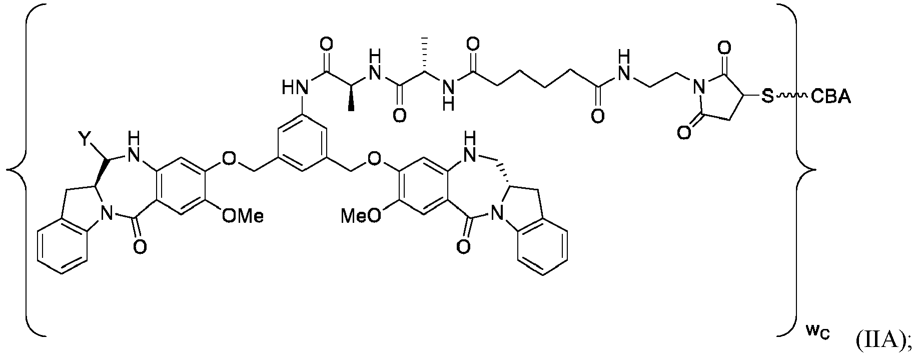 or a pharmaceutically acceptable salt thereof, give a pharmaceutical composition comprising the immunoconjugate and methionine, wherein:
Y is -SO₃H or sodium salt thereof;
Wc is 2; and
CBA is an anti-CD123 antibody comprising: a) an immunoglobulin heavy chain having the amino acid sequence set forth in SEQ ID NO:8; and b) an immunoglobulin light chain having the amino acid sequence set forth in SEQ ID NO: 10.

In a third embodiment, the method of the second aspect comprises mixing 1 mM to 4 mM of methionine and an immunoconjugate represented by the following formula: 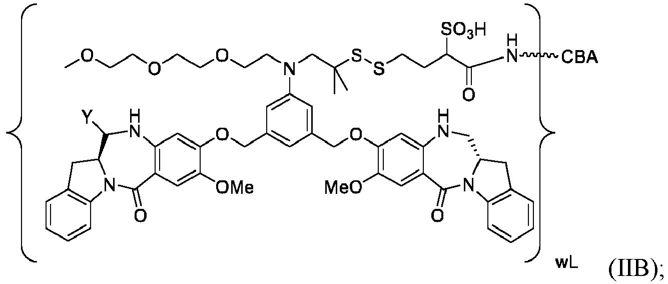 or a pharmaceutically acceptable salt thereof, wherein:
Y is -SO₃H or sodium salt thereof;
W_{L} is an integer from 1 to 10; and
CBA is an anti-CD33 antibody comprising an immunoglobulin heavy chain having the amino acid sequence set forth in SEQ ID NO: 18 and an immunoglobulin light chain having the amino acid sequence set forth in SEQ ID NO:20.

In a fourth embodiment, the method of the first, second or third embodiment of the second aspect comprises mixing 3 mM methionine with the immunoconjugate.

In a fifth embodiment, when the pharmaceutical composition prepared by the method of the first, second, third, or fourth embodiment of the second aspect is exposed to light at room temperature for 6 hours, the immunoconjugate has less than 15%, less than 10%, less than 5%, less than 4%, less than 3%, less than 2%, or less than 1% of methionine oxidation.

In a sixth embodiment, the pharmaceutical composition prepared by the method of the first, second, third, fourth or fifth embodiment of the second aspect is in a container that protects the pharmaceutical composition from light exposure. Any suitable container (e.g., vial or syringe) can be used. For example, light-resistant (e.g., amber, yellow-green or blue) container, such as colored-glass or colored plastic container (e.g., vial or syringe), can be used to minimize light exposure. Alternatively, colorless or translucent container can be used if it is protected by a light-resistant opaque covering, such as a paper carton or plastic box.

In a seventh embodiment, the pharmaceutical composition prepared by the first, second, third, fourth, fifth or sixth embodiment of the second aspect comprises 1 mg/mL to 10 mg/mL, 1 mg/mL to 5 mg/mL, 1 mg/mL to 3 mg/mL, or 1.5 mg/mL to 2.5 mg/mL of the immunoconjugate. In certain embodiments, the pharmaceutical composition prepared by the method of the second, third, fourth, fifth or sixth embodiment of the second aspect comprises 2 mg/mL of the immunoconjugate.

In an eighth embodiment, the pharmaceutical composition prepared by the method of the first, second, third, fourth, fifth, sixth or seventh embodiment of the second aspect further comprises sodium bisulfite. In certain embodiments, the concentration for the sodium bisulfite in the pharmaceutical composition is 10 µM to 100 µM, 20 µM to 90 µM, 30 µM to 80 µM, 30 µM to 70 µM, 40 µM to 60 µM, or 45 µM to 55 µM of sodium bisulfite. In certain embodiments, the pharmaceutical composition prepared by the method of the second, third, fourth, fifth, sixth or seventh embodiment of the second aspect further comprises 50 µM of sodium bisulfite.

In a ninth embodiment, the pharmaceutical composition prepared by the method of the first, second, third, fourth, fifth, sixth, seventh or eighth embodiment of the second aspect further comprises one or more pharmaceutically acceptable vehicle (e.g. carrier, excipient) (Remington, The Science and Practice of Pharmacy 20th Edition Mack Publishing, 2000). Suitable pharmaceutically acceptable vehicles include, but are not limited to, nontoxic buffers such as phosphate, citrate, succinate, histidine and other organic acids; salts such as sodium chloride; preservatives (e.g., octadecyldimethylbenzyl ammonium chloride; hexamethonium chloride; benzalkonium chloride; benzethonium chloride; phenol, butyl or benzyl alcohol; alkyl parabens, such as methyl or propyl paraben; catechol; resorcinol; cyclohexanol; 3-pentanol; and m-cresol); low molecular weight polypeptides (e.g., less than about 10 amino acid residues); proteins such as serum albumin, gelatin, or immunoglobulins; hydrophilic polymers such as polyvinylpyrrolidone; amino acids such as glycine, glutamine, asparagine, histidine, arginine, or lysine; carbohydrates such as monosaccharides, disaccharides, glucose, mannose, or dextrins; chelating agents such as EDTA; sugars such as sucrose, mannitol, trehalose or sorbitol; salt-forming counter-ions such as sodium; metal complexes (e.g., Zn-protein complexes); and non-ionic surfactants such as TWEEN or polyethylene glycol (PEG).

In certain embodiments, the pharmaceutical composition prepared by the method of the first, second, third, fourth, fifth, sixth, seventh, or eighth embodiment of the second aspect further comprises sodium bisulfite, a buffer, sugar and non-ionic surfactant. In certain embodiments, the pharmaceutically composition by the method of the first, second, third, fourth, fifth, sixth, seventh or eighth embodiment of the second aspect further comprises sodium bisulfite, succinate or histidine buffer, trehalose and polysorbate 20.

In certain embodiments, the buffer (e.g., succinate or histidine) concentration in the pharmaceutical composition is in the range of 5 mM to 50 mM, 5 mM to 25 mM, 5 mM to 15 mM, 10 mM to 25 mM, or 15 mM to 25 mM. In certain embodiments, the buffer concentration is 10 mM or 20 mM.

In certain embodiments, the sugar (e.g., trehalose) concentration in the pharmaceutical composition is in the range of 5-10%, 6-8%, 6.5-7.5%, 7.0-7.4%, 7.1-7.3% (w/v). In certain embodiments, the sugar (e.g., trehalose) concentration in the pharmaceutical composition is 7.2%, (w/v).

In certain embodiments, the non-ionic surfactant (e.g., polysorbate 20) concentration in the pharmaceutical composition is in the range of 0.01-0.1 %, 0.01-0.05 %, or 0.01-0.03% (w/v). In certain embodiments, the the non-ionic surfactant (e.g., polysorbate 20) concentration in the pharmaceutical composition is 0.02% (w/v)

In a tenth embodiment, the pharmaceutical composition by the method of the first, second, third, fourth, fifth, sixth, seventh, eighth or ninth embodiment of the second aspect has a pH of 4 to 5, 4 to 4.5, 4 to 4.4, or 4.1 to 4.3. In certain embodiments, the pH is 4.2.

In an eleventh embodiment, the pharmaceutical composition by the method of the first, second, third, fourth, fifth, sixth, seventh or eighth or ninth embodiment of the second aspect has a pH of 5.5 to 6.5, 5.9 to 6.3 or 6.0 to 6.2. In certain embodiments, the pH is 6.1.

In a twelfth embodiment, the pharmaceutical composition prepared by the method of the second, fourth, fifth, sixth, seventh or eighth or ninth embodiment of the second aspect comprises 2 mg/mL of the immunoconjugate of formula (IIA), 3 mM methionine, 10 mM succinate, 50 µM sodium bisulfite, 7.2% (w/v) trehalose (or 8.0% (w/v) trehalose dihydrate), and 0.01% (w/v) polysorbate 20 and the pH of the pharmaceutical composition is 4.2.

In a thirteenth embodiment, the pharmaceutical composition prepared by the method of the third, fourth, fifth, sixth, seventh, eighth, or ninth embodiment of the second aspect comprises 2 mg/mL of the immunoconjugate of formula (IIB), 3 mM methionine, 20 mM histidine, 50 µM sodium bisulfite, 7.2% (w/v) trehalose (or 8.0% (w/v) trehalose dihydrate), 0.02% (w/v) polysorbate 20 and the pH of the pharmaceutical composition is 6.1.

### 4. Methods of Preparing The Immunoconjugates

In a third aspect, the present invention provides a method of preparing an immunoconjugate of the present invention comprising reacting a CBA with a cytotoxic agent or a cytotoxic agent-linker compound in the presence of an antioxidant.

In a first embodiment of the third aspect, the present invention provides a method of preparing an immunoconjugate represented by the following formula: 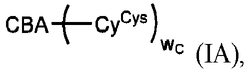 comprising reacting a CBA with a cytotoxic agent represented by the following formula: 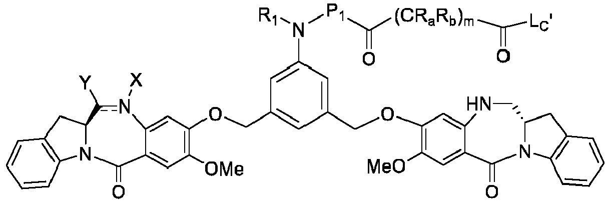 or a pharmaceutically acceptable salt thereof, in the presence of an antioxidant, wherein L_{C}' is represented by 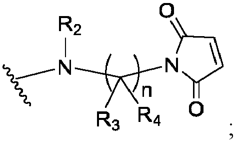 and the remaining variables are as described above for formula (IA), or the 1^{st} - 13^{th} specific embodiment described above.

In certain embodiments, the antioxidant reduces the amount of methionine oxidation in the immunoconjugate.

In certain embodiments, the antioxidant is methionine derivatives with amine and/or carboxyl protecting groups (e.g., N-acetyl, fluorenylmethyloxycarbonyl (Fmoc), tert-butyloxycarbonyl (Boc), and carboxybenzyl (Cbz)), water-soluble, non-nucleophilic thioethers, or dimethyl sulfide.

In certain embodiments, the antioxidant is N-acetylmethionine.

In a second embodiment of the third aspect, the present invention provides a method of preparing an immunoconjugate represented by the following formula: 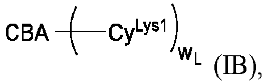 comprising the step of:
(a) reacting a cytotoxic agent represented by the following formula: 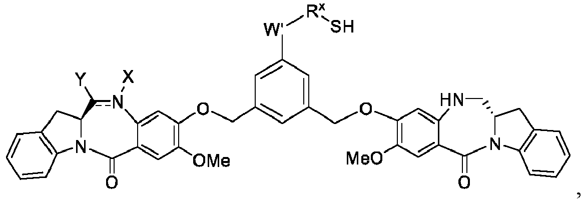 or a pharmaceutically acceptable salt thereof, with a bifunctional crosslinking agent selected from the following: 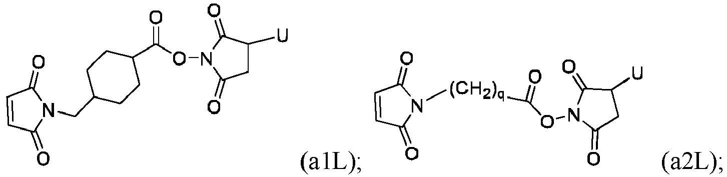 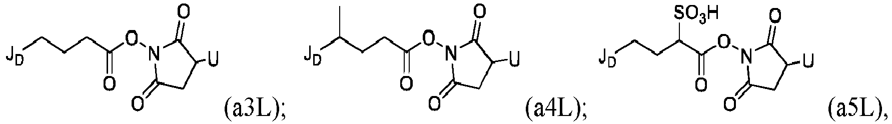 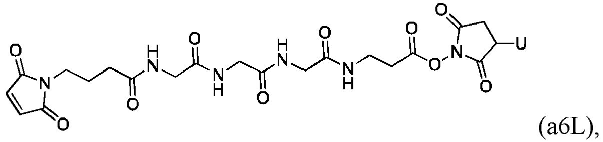 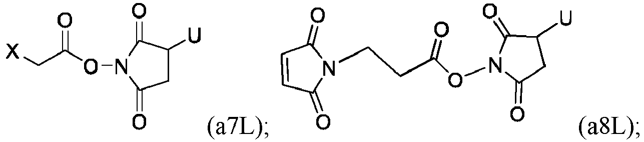 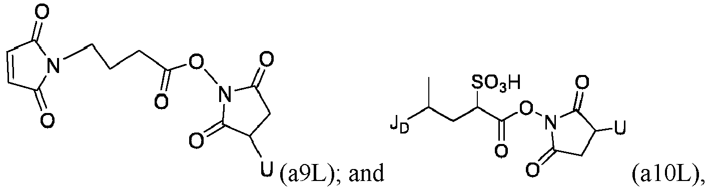 to form a cytotoxic agent-linker compound, wherein X is halogen; J_{D} -SH, -SSR^{d}, or -SC(=O)R^{g}; R^{d} is phenyl, nitrophenyl, dinitrophenyl, carboxynitrophenyl, pyridyl or nitropyridyl; R^{g} is an alkyl; q is an integer from 1 to 5; and U is -H or SO₃H; and
(b) reacting the CBA with the cytotoxic agent-linker compound in the presence of an antioxidant to form the immunoconjugate, wherein the remaining variables are as described above for formula (IB) or the 14^{th}-19^{th} specific embodiments.

In certain embodiments, the cytotoxic agent-linker compound is not purified before reacting with the CBA in step (b).

In certain embodiments, the antioxidant reduces the amount of methionine oxidation in the immunoconjugate.

In certain embodiments, the antioxidant is methionine derivatives with amine and/or carboxyl protecting groups (e.g., N-acetyl, fluorenylmethyloxycarbonyl (Fmoc), tert-butyloxycarbonyl (Boc), and carboxybenzyl (Cbz)), water-soluble, non-nucleophilic thioethers, or dimethyl sulfide

In certain embodiment, the antioxidant is N-acetylmethionine methyl ester.

In a third embodiment of the third aspect, the present invention provides a method of preparing an immunoconjugate represented by the following formula: 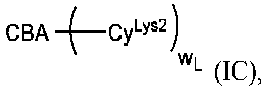 comprising reacting the CBA with a cytotoxic agent represented by the following formula: 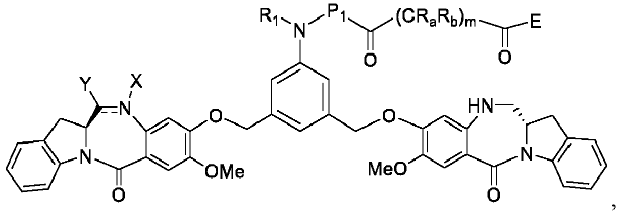 or a pharmaceutically acceptable salt thereof, wherein C(=O)E is a reactive ester group, such as N-hydroxysuccinimde ester, N-hydroxy sulfosuccinimide ester, nitrophenyl (e.g., 2 or 4-nitrophenyl) ester, dinitrophenyl (e.g., 2,4-dinitrophenyl) ester, sulfo-tetraflurophenyl (*e.g.*, 4-sulfo-2,3,5,6-tetrafluorophenyl) ester, or pentafluorophenyl ester, preferably N-hydroxysuccinimide ester; and the remaining variables are as described above in the 20^{th} to 26^{th} specific embodiments.

In certain embodiments, the antioxidant reduces the amount of methionine oxidation in the immunoconjugate.

In certain embodiments, the antioxidant is methionine derivatives with amine and/or carboxyl protecting groups (e.g., N-acetyl, fluorenylmethyloxycarbonyl (Fmoc), tert-butyloxycarbonyl (Boc), and carboxybenzyl (Cbz)), water-soluble, non-nucleophilic thioethers, or dimethyl sulfide

In certain embodiments, the antioxidant is N-acetylmethionine methyl ester.

In a fourth embodiment of the third aspect, the present invention provides a method of preparing an immunoconjugate represented by the following formula: 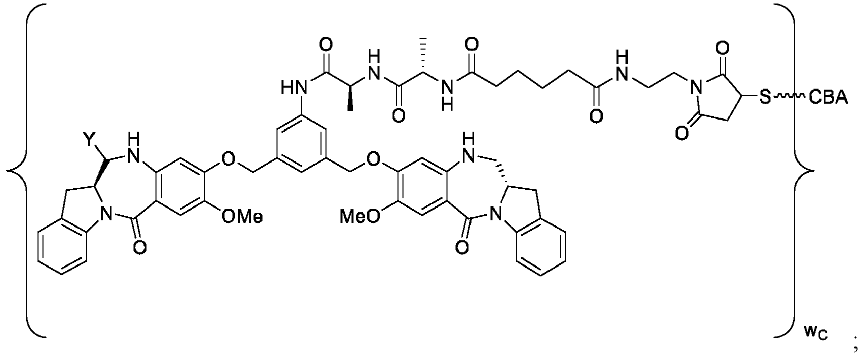 or a pharmaceutically acceptable salt thereof, comprising reacting a CBA with a cytotoxic agent represented by the following formula: 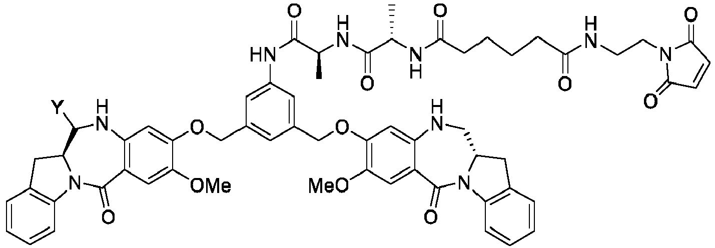 or a pharmaceutically acceptable salt thereof, in the presence of an antioxidant, wherein:
Y is -SO₃H or sodium salt thereof;
W_{C} is 2; and
CBA is an anti-CD123 antibody comprising: a) an immunoglobulin heavy chain full sequence of SEQ ID NO:8; and b) an immunoglobulin light chain full sequence SEQ ID NO:10.

In certain embodiments, the antioxidant reduces the amount of methionine oxidation in the immunoconjugate.

In certain embodiments, the antioxidant is methionine derivatives with amine and/or carboxyl protecting groups (e.g., N-acetyl, fluorenylmethyloxycarbonyl (Fmoc), tert-butyloxycarbonyl (Boc), and carboxybenzyl (Cbz)), water-soluble, non-nucleophilic thioethers, or dimethyl sulfide.

In certain embodiment, the antioxidant is N-acetylmethionine.

In a fifth embodiment of the third aspect, the present invention provides a method of preparing an immunoconjugate represented by the following formula: 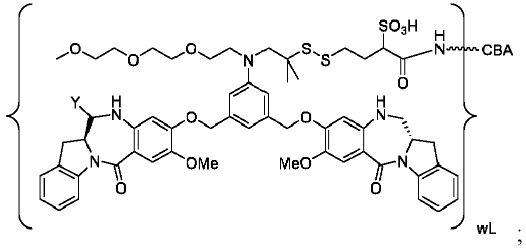 or a pharmaceutically acceptable salt thereof, comprising the steps of:
(a) reacting a cytotoxic agent represented by the following formula: 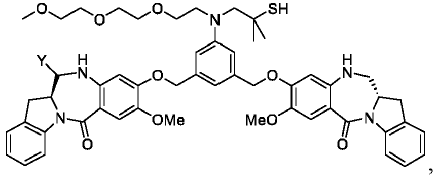 or a pharmaceutically acceptable salt thereof, with a bifunctional crosslinking agent sulfo-SPDB presented by the following formula: 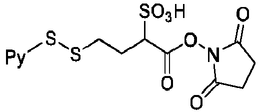 to form a cytotoxic agent-linker compound represented by the following formula: 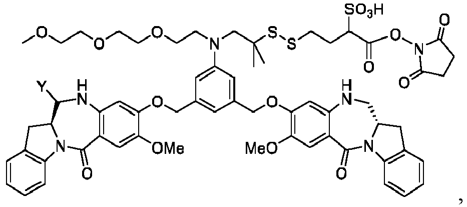 or a pharmaceutically acceptable salt thereof; and
(b) reacting the CBA with the cytotoxic agent-linker compound in the presence of an antioxidant to form the immunoconjugate, wherein:
   Y is -SO₃H;
   W_{L} is an integer from 1 to 10; and
   CBA is an anti-CD33 antibody comprising an immunoglobulin heavy chain having the amino acid sequence set forth in SEQ ID NO: 18 and an immunoglobulin light chain having the amino acid sequence set forth in SEQ ID NO:20.

In certain embodiments, the cytotoxic agent-linker compound is not purified before reacting with the CBA in step (b).

In certain embodiments, the antioxidant reduces the amount of methionine oxidation in the immunoconjugate.

In certain embodiments, the antioxidant is methionine derivatives with amine and/or carboxyl protecting groups (e.g., N-acetyl, fluorenylmethyloxycarbonyl (Fmoc), tert-butyloxycarbonyl (Boc), and carboxybenzyl (Cbz)), water-soluble, non-nucleophilic thioethers, or dimethyl sulfide.

In certain embodiment, the antioxidant is N-acetylmethionine methyl ester.

In a sixth embodiment, the present invention provides a method of preparing an immunoconjugate represented by the following formula: 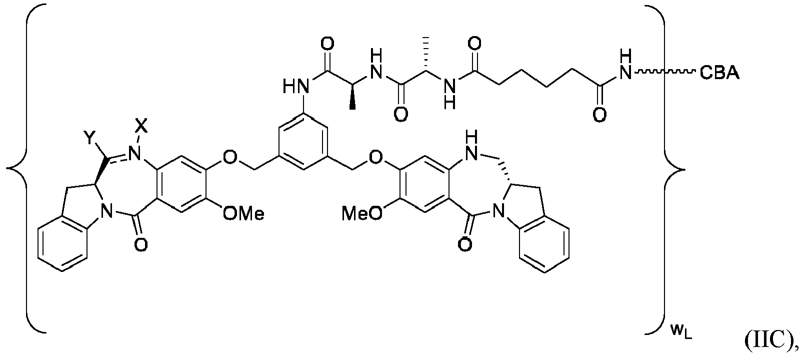 or a pharmaceutically acceptable salt thereof, comprising reacting the CBA with a cytotoxic agent represented by the following formula: 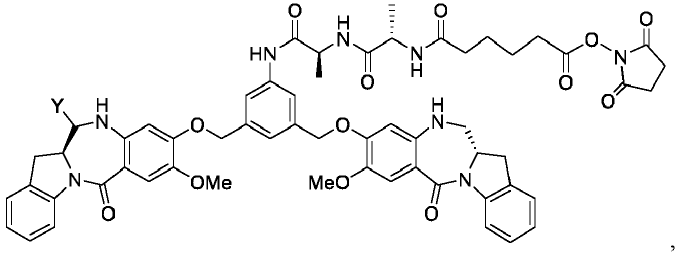 or a pharmaceutically acceptable salt thereof, wherein:
Y is -SO₃H;
W_{L} is an integer from 1 to 10; and
CBA is an antibody or antigen-binding fragment thereof.

In certain embodiments, the antioxidant reduces the amount of methionine oxidation in the immunoconjugate.

In certain embodiments, the antioxidant is methionine derivatives with amine and/or carboxyl protecting groups (e.g., N-acetyl, fluorenylmethyloxycarbonyl (Fmoc), tert-butyloxycarbonyl (Boc), and carboxybenzyl (Cbz)), water-soluble, non-nucleophilic thioethers, or dimethyl sulfide.

In certain embodiments, the antioxidant is N-acetylmethionine methyl ester.

In a seventh embodiment, the method of the first, second, third, fourth, fifth, or sixth embodiment of the third aspect further comprises purifying the immunoconjugate into a formulation buffer to give a pharmaceutical composition comprising the immunoconjugate and 0.1 mM to 20 mM, 0.1 mM to 10 mM, 0.5 mM to 5 mM, or 1 mM to 4 mM methionine. In certain embodiments, the formulation buffer comprises 3 mM methionine.

In a eighth embodiment, the pharmaceutical composition of the seventh embodiment of the third aspect further comprises sodium bisulfite. In certain embodiments, the concentration for the sodium bisulfite in the pharmaceutical composition is 10 µM to 100 µM, 20 µM to 90 µM, 30 µM to 80 µM, 30 µM to 70 µM, 40 µM to 60 µM, or 45 µM to 55 µM of sodium bisulfite. In certain embodiments, the concentration for the sodium bisulfite in the pharmaceutical composition is 50 µM.

In a ninth embodiment, the pharmaceutical composition of the seventh or eighth embodiment of the third aspect further comprises one or more pharmaceutically acceptable vehicle (e.g. carrier, excipient) (Remington, The Science and Practice of Pharmacy 20th Edition Mack Publishing, 2000). Suitable pharmaceutically acceptable vehicles include, but are not limited to, nontoxic buffers such as phosphate, citrate, succinate, histidine and other organic acids; salts such as sodium chloride; preservatives (e.g., octadecyldimethylbenzyl ammonium chloride; hexamethonium chloride; benzalkonium chloride; benzethonium chloride; phenol, butyl or benzyl alcohol; alkyl parabens, such as methyl or propyl paraben; catechol; resorcinol; cyclohexanol; 3-pentanol; and m-cresol); low molecular weight polypeptides (e.g., less than about 10 amino acid residues); proteins such as serum albumin, gelatin, or immunoglobulins; hydrophilic polymers such as polyvinylpyrrolidone; amino acids such as glycine, glutamine, asparagine, histidine, arginine, or lysine; carbohydrates such as monosaccharides, disaccharides, glucose, mannose, or dextrins; chelating agents such as EDTA; sugars such as sucrose, mannitol, trehalose or sorbitol; salt-forming counter-ions such as sodium; metal complexes (e.g., Zn-protein complexes); and non-ionic surfactants such as TWEEN or polyethylene glycol (PEG).

In certain embodiments, the pharmaceutical composition of the ninth embodiment of the third aspect further comprises sodium bisulfite, buffer, sugar and non-ionic surfactant. In certain embodiments, the pharmaceutical composition of the ninth embodiment of the third aspect further comprises sodium bisulfite, succinate or histidine buffer, trehalose and polysorbate 20.

In certain embodiments, the buffer (e.g., succinate or histidine) concentration in the pharmaceutical composition is in the range of 5 mM to 50 mM, 5 mM to 25 mM, 5 mM to 15 mM, 10 mM to 25 mM, or 15 mM to 25 mM. In certain embodiments, the buffer concentration is 10 mM or 20 mM.

In certain embodiments, the sugar (e.g., trehalose) concentration in the pharmaceutical composition is in the range of 5-10%, 6-8%, 6.5-7.5%, 7.0-7.4%, 7.1-7.3% (w/v). In certain embodiments, the sugar (e.g., trehalose) concentration in the pharmaceutical composition is 7.2%, (w/v).

In certain embodiments, the non-ionic surfactant (e.g., polysorbate 20) concentration in the pharmaceutical composition is in the range of 0.01-0.1 %, 0.01-0.05 %, or 0.01-0.03%

(w/v). In certain embodiments, the the non-ionic surfactant (e.g., polysorbate 20) concentration in the pharmaceutical composition is 0.02% (w/v).

In a tenth embodiment, for the method of the seventh, eighth or ninth embodiment of the third aspect, the concentration of the immunoconjugate in the pharmaceutical composition is in the range of 1 mg/mL to 10 mg/mL, 1 mg/mL to 5 mg/mL, 1 mg/mL to 3 mg/mL, or 1.5 mg/mL to 2.5 mg/mL. In certain embodiments, the concentration of the immunoconjugate is 2 mg/mL.

In an eleventh embodiment, for the method of the seventh, eighth, ninth or tenth embodiment of the third aspect, the pharmaceutical composition has a pH of 4 to 5, 4 to 4.5, 4 to 4.4, or 4.1 to 4.3. In certain embodiments, the pH is 4.2.

In a twelfth embodiment, for the method of the seventh, eighth, ninth or tenth embodiment of the third aspect, the pharmaceutical composition has a pH of 5.5 to 6.5, 5.9 to 6.3 or 6.0 to 6.2. In certain embodiments, the pH is 6.1.

In a thirteenth embodiment, for the method of the seventh, eighth or ninth embodiment of the third aspect, the pharmaceutical composition comprises 2 mg/mL of the immunoconjugate of formula (IIA), 3 mM methionine, 10 mM succinate, 50 µM sodium bisulfite, 7.2% (w/v) trehalose (or 8.0% (w/v) trehalose dihydrate), and 0.01% (w/v) polysorbate 20 and the pH of the pharmaceutical composition is 4.2.

In a fourteenth embodiment, the pharmaceutical composition prepared by the method of the seventh, eighth or ninth embodiment of the third aspect comprises 2 mg/mL of the immunoconjugate of formula (IIB), 3 mM methionine, 20 mM histidine, 50 µM sodium bisulfite, 7.2% (w/v) trehalose (or 8.0% (w/v) trehalose dihydrate), 0.02% (w/v) polysorbate 20 and the pH of the pharmaceutical composition is 6.1.

In certain embodiments, for methods described above, any suitable amount of the antioxidant can be used in the reaction of the CBA and the cytotoxic agent or the cytotoxic agent-linker compound. In certain embodiments, excess amount of the antioxidant relative to the CBA can be used. Exemplary molar ratio of the antioxidant relative to the CBA is in the range of 200:1 to 1.5:1, 150:1 to 1.5:1, 100:1 to 1.5:1, 50:1 to 1.5:1, 20:1 to 2:1, 15:1 to 2:1, 10:1 to 2:1, or 10:1 to 5:1. In certain embodiment, the ratio for the antioxidant to the CBA is 10:1.

In certain embodiments, the reaction of the CBA and the cytotoxic agent or the cytotoxic agent-linker compound is carried out in a suitable solvent or solvents. In certain embodiments, the solvent(s) comprise N,N-dimethylacetamide (DMA) and/or propylene glycol. In certain embodiments, the reaction of the CBA and the cytotoxic agent or the cytotoxic agent-linker compound is carried out in DMA, propylene glycol and an aqueous buffer solution. Any suitable aqueous buffer can be used. Exemplary buffers include, but are not limited to, phosphate, citrate, succinate, and histidine.

In certain embodiments, the reaction of the CBA and the cytotoxic agent or the cytotoxic agent-linker compound is carried out at a suitable temperature. In certain embodiments, the reaction is carried out at a temperature between 15 °C to 25 °C, between 5 °C to 15 °C, between 20 °C to 25 °C. In certain embodiments, the reaction is carried out at room temperature.

### 5. Cell-Binding Agents

Cell-binding agents in the immunoconjugates of the present invention can be of any kind presently known, or that become known, including peptides and non-peptides. Generally, these can be antibodies (such as polyclonal antibodies and monoclonal antibodies, especially monoclonal antibodies), lymphokines, hormones, growth factors, vitamins (such as folate etc., which can bind to a cell surface receptor thereof, e.g., a folate receptor), nutrient-transport molecules (such as transferrin), or any other cell-binding molecule or substance.

In certain embodiments, the cell-binding agent is an antibody, a single chain antibody, an antibody fragment that specifically binds to the target cell, a monoclonal antibody, a single chain monoclonal antibody, a monoclonal antibody fragment (or "antigen-binding portion") that specifically binds to a target cell, a chimeric antibody, a chimeric antibody fragment (or "antigen-binding portion") that specifically binds to the target cell, a domain antibody (e.g., sdAb), or a domain antibody fragment that specifically binds to the target cell.

In certain embodiments, the cell-binding agent is a humanized antibody, a humanized single chain antibody, or a humanized antibody fragment (or "antigen-binding portion").

In certain embodiments, the cell-binding agent is a resurfaced antibody, a resurfaced single chain antibody, or a resurfaced antibody fragment (or "antigen-binding portion").

In certain embodiments, wherein the cell-binding agent is an antibody or an antigen-binding portion thereof (including antibody derivatives), the CBA may bind to a ligand on the target cell, such as a cell-surface ligand, including cell-surface receptors.

In certain embodiments, the cell-binding agent is an antibody or antigen-binding fragment thereof that: (a) binds an epitope within amino acids 101 to 346 of human CD123 / IL3-Rα antigen, and (b) inhibits IL3-dependent proliferation in antigen-positive TF-1 cells (see WO2017/004026, incorporated herein by reference in their entirety).

In certain embodiments, the cell-binding agent is an anti-CD123 antibody or antigen-binding fragment thereof as described in WO2017/004026, which is incorporated herein by reference.

In certain embodiments, the anti-CD123 antibody or antigen-binding fragment thereof may comprise: a) at least one heavy chain variable region or fragment thereof comprising three sequential complementarity-determining regions (CDR) CDR1, CDR2, and CDR3, respectively, wherein, CDR1 has the amino acid sequence of SEQ ID NO:4, CDR2 has the amino acid sequence of SEQ ID NO:5, and, CDR3 has the amino acid sequence of SEQ ID NO:6; and b) at least one light chain variable region or fragment thereof comprising three sequential complementarity-determining regions (CDR) CDR1, CDR2, and CDR3, respectively, wherein CDR1 has the amino acid sequence of SEQ ID NO:1, CDR2 has the amino acid sequence of SEQ ID NO:2, and, CDR3 has the amino acid sequence of SEQ ID NO:3.

In certain embodiments, the anti-CD123 antibody or antigen-binding fragment thereof comprises a heavy chain variable region having the amino acid sequence of SEQ ID NO:7 and a light chain variable region having the amino acid sequence of SEQ ID NO:9.

In certain embodiments, the anti-CD123 antibody has a heavy chain full length sequence of SEQ ID NO:8 and a light chain full length sequence of SEQ ID NO:10.

In certain embodiments, the cell-binding agent is an anti-CD33 antibody or an antigen-binding fragment thereof as described in U.S. Pat. Nos. 7,342,110 and 7,557,189, which are incorporated herein by reference.

In certain embodiments, the anti-CD33 antibody or antigen-binding fragment thereof may comprise: a) at least one heavy chain variable region or fragment thereof comprising three sequential complementarity-determining regions (CDR) CDR1, CDR2, and CDR3, respectively, wherein, CDR1 has the amino acid sequence of SEQ ID NO: 14, CDR2 has the amino acid sequence of SEQ ID NO:15, and, CDR3 has the amino acid sequence of SEQ ID NO:16; and b) at least one light chain variable region or fragment thereof comprising three sequential complementarity-determining regions (CDR) CDR1, CDR2, and CDR3, respectively, wherein CDR1 has the amino acid sequence of SEQ ID NO: 11, CDR2 has the amino acid sequence of SEQ ID NO:12, and, CDR3 has the amino acid sequence of SEQ ID NO:13.

In certain embodiments, the anti-CD33 antibody or antigen-binding fragment thereof comprises a heavy chain variable region having the amino sequence of SEQ ID NO:17 and a light chain variable region having the amino acid sequence of SEQ ID NO:19.

In certain embodiments, the anti-CD33 antibody has a heavy chain full length sequence of SEQ ID NO:18 and a light chain full length sequence of SEQ ID NO:20.

In certain embodiments, the anti-CD33 antibody is huMy9-6 antibody.

In certain embodiments, the antibody described herein is a murine, non-human mammal, chimeric, humanized, or human antibody. For example, the humanized antibody may be a CDR-grafted antibody or resurfaced antibody. In certain embodiments, the antibody is a full-length antibody. In certain embodiments, the antigen-binding fragment thereof is an Fab, Fab', F(ab')₂, F_{d}, single chain Fv or scFv, disulfide linked Fᵥ, V-NAR domain, IgNar, intrabody, IgGΔCH₂, minibody, F(ab')₃, tetrabody, triabody, diabody, single-domain antibody, DVD-Ig, Fcab, mAb₂, (scFv)₂, or scFv-Fc.

### 6. Immunoconjugates

The immunoconjugates of the present invention comprises a cell-binding agent described herein (*e.g.,* an antibody or an antigen-binding fragment thereof) covalently linked to one or more molecules of the cytotoxic agent described herein.

In certain embodiments, the cytotoxic agent is a benzodiazepine compounds, such as a pyrrolobenzodiazepine (PBD) or an indolinobenzodiazepine (IGN) compound.

As used herein, a "benzodiazepine" compound is a compound having a benzodiazepine core structure. The benzodiazepine core can be substituted or unsubstituted, and/or fused with one or more ring structures. It also includes a compound having two benzodiazepine core linked by a linker. The imine functionality (-C=N-) as part of benzodiazepine core can be reduced.

As used herein, a "pyrrolobenzodiazepine" (PBD) compound is a compound having a pyrrolobenzodiazepine core structure. The pyrrolobenzodiazepine can be substituted or unsubstituted. It also includes a compound having two pyrrolobenzodiazepine core linked by a linker. The imine functionality (-C=N-) as part of indolinobenzodiazepine core can be reduced.

In certain embodiments, the pyrrolobenzodiazepine compound comprises a core structure represented by 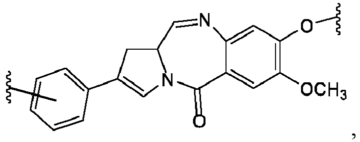 which can be optionally substituted.

In certain embodiments, the pyrrolobenzodiazepine compounds comprises a core structure represented by 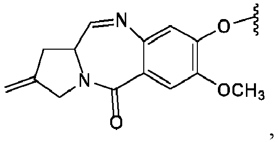 which can be optionally substituted.

As used herein, an "indolinobenzodiazepine" (IGN) compound is a compound having an indolinobenzodiazepine core structure. The indolinobenzodiazepine can be substituted or unsubstituted. It also includes a compound having two indolinobenzodiazepine core linked by a linker. The imine functionality (-C=N-) as part of indolinobenzodiazepine core can be reduced.

In certain embodiments, the indolinobenzodiazepine compound comprises a core structure represented by 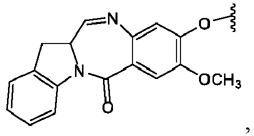 which can be optionally substituted.

In some embodiments, the indolinobenzodiazepine compound comprises a core structure represented by 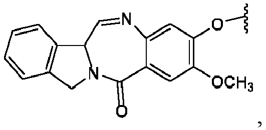 which can be optionally substituted.

In certain embodiments, the immunoconjugates of the present invention comprises a cell-binding agent (including antibody or antigen-binding fragment thereof) described herein covalently linked to a cytotoxic agent described herein through the thiol group (-SH) of one or more cysteine residues located on the cell-binding agent (such as those described in the 1^{st} to 13^{th} specific embodiments below).

In a 1^{st} specific embodiment, the immunoconjugate is represented by the following formula: 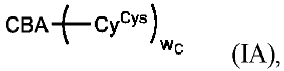 CBA is an antibody or antigen-binding fragment thereof, wherein CBA is linked to Cy^{Cys} through a thiol group of one or more cysteine residues located on the CBA;
Wc is 1 or 2; and
Cy^{Cys} is represented by the following formula: 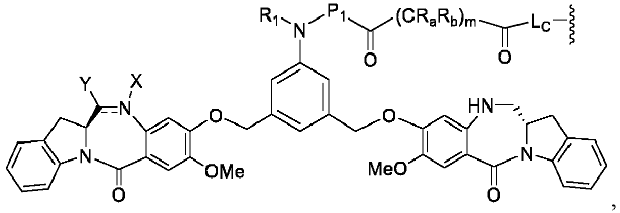
or a pharmaceutically acceptable salt thereof, wherein:
   the double line -̅ -̅ between N and C represents a single bond or a double bond, provided that when it is a double bond, X is absent and Y is -H or a (C₁-C₄)alkyl; and when it is a single bond, X is -H or an amine protecting moiety, Y is -OH or -SO₃H;
   R₁ is -H or a (C₁-C₃)alkyl;
   P₁ is an amino acid residue or a peptide containing 2 to 5 amino acid residues;
   Rₐ and R_{b}, for each occurrence, are independently -H, (C₁-C₃)alkyl, or a charged substituent or an ionizable group Q;
   m is an integer from 1 to 6;
   L_{C} is represented by 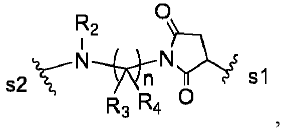
   s1 is the site covalently linked to CBA, and s2 is the site covalently linked to the -C(=O)- group on Cy^{Cys}; wherein:
      R₂ is -H or a (C₁-C₃)alkyl
      R₃ and R₄, for each occurrence, are independently -H or a (C₁-C₃)alkyl; and
      n is an integer between 1 and 10.

In a 2^{nd} specific embodiment, for immunoconjugate of formula (IA), Rₐ and R_{b} are both H; and R₁ is H or Me; and the remaining variables are as described in the 1^{st} specific embodiment.

In a 3^{rd} specific embodiment, for immunoconjugate of formula (IA), P is selected from Gly-Gly-Gly, Ala-Val, Val-Ala, Val-Cit, Val-Lys, Phe-Lys, Lys-Lys, Ala-Lys, Phe-Cit, Leu-Cit, Ile-Cit, Trp, Cit, Phe-Ala, Phe-N⁹-tosyl-Arg, Phe-N⁹-nitro-Arg, Phe-Phe-Lys, D-Phe-Phe-Lys, Gly-Phe-Lys, Leu-Ala-Leu, Ile-Ala-Leu, Val-Ala-Val, Ala-Leu-Ala-Leu (SEQ ID NO:21), β-Ala-Leu-Ala-Leu (SEQ ID NO:22), Gly-Phe-Leu-Gly (SEQ ID NO:23), Val-Arg, Arg-Val, Arg-Arg, Val-D-Cit, Val-D-Lys, Val-D-Arg, D-Val-Cit, D-Val-Lys, D-Val-Arg, D-Val-D-Cit, D-Val-D-Lys, D-Val-D-Arg, D-Arg-D-Arg, Ala-Ala, Ala-D-Ala, D-Ala-Ala, D-Ala-D-Ala, Ala-Met, and Met-Ala; and the remaining variables are as described in the 1^{st} or 2^{nd} specific embodiment.

In a 4^{th} specific embodiment, for immunoconjugate of formula (IA), P is Ala-Ala, Ala-D-Ala, D-Ala-Ala, or D-Ala-D-Ala; and the remaining variables are as described in the 1^{st} or 2^{nd} specific embodiment.

In a 5^{th} specific embodiment, for immunoconjugate of formula (IA), Q is -SO₃H; and the remaining variables are as described in the 1^{st}, 2^{nd}, 3^{rd}, or 4^{th} specific embodiment.

In a 6^{th} specific embodiment, for immunoconjugate of formula (IA), Q is H; and the remaining variables are as described in the 1^{st}, 2^{nd}, 3^{rd}, or 4^{th} specific embodiment.

In a 7^{th} specific embodiment, for immunoconjugate of formula (IA), R₃ and R₄ are both H; n is an integer from 1 to 6; and the remaining variables are as described in the 1^{st}, 2^{nd}, 3^{rd}, 4^{th}, 5^{th} or 6^{th} specific embodiment.

In an 8^{th} specific embodiment, for immunoconjugate of formula (IA), -Lc- is represented by the following formula: 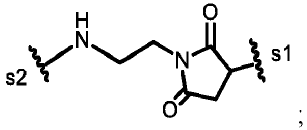 and the remaining variables are as described in the 1^{st}, 2^{nd}, 3^{rd}, 4^{th}, 5^{th}, 6^{th} or 7^{th} specific embodiment.

In a 9^{th} specific embodiment, for immunoconjugate of formula (IA), the immunoconjugate is represented by the following formula: 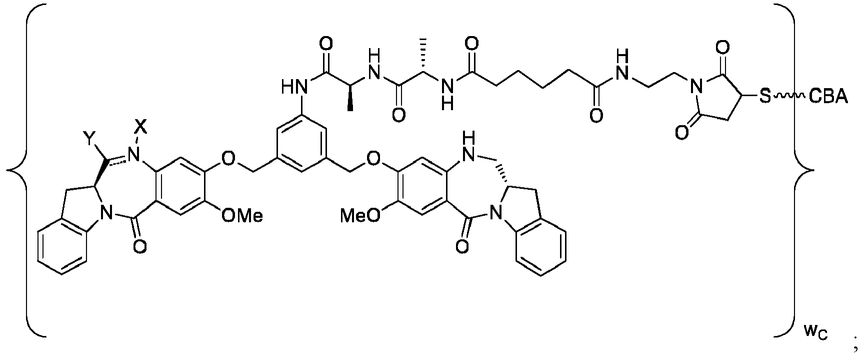 or a pharmaceutically acceptable salt thereof, wherein the double line -̅ -̅ between N and C represents a single bond or a double bond, provided that when it is a double bond, X is absent and Y is -H, and when it is a single bond, X is -H, and Y is -OH or -SO₃H; and the remaining variables are as described in the 1^{st} specific embodiment.

In certain embodiments, the double line -̅ -̅ between N and C represents a single bond, X is -H, and Y is -SO₃H.

In a 10^{th} specific embodiment, for the immunoconjugates of the 1^{st} to 9^{th} specific embodiments, the cell-binding agent (CBA) is an antibody or an antigen-binding fragment thereof that (a) binds an epitope within amino acids 101 to 346 of human CD123 / IL3-Rα antigen, and (b) inhibits IL3-dependent proliferation in antigen-positive TF-1 cells.

In an 11^{th} specific embodiment, for the immunoconjugates of the 1^{st} to 10^{th} specific embodiments, the CBA is an anti-CD123 antibody or antigen-binding fragment thereof comprising:
a) an immunoglobulin heavy chain variable region comprising a CDR1 having an amino acid sequence set forth in SEQ ID NO:4, a CDR2 having an amino acid sequence set forth in SEQ ID NO:5, and a CDR3 having an amino acid sequence set forth in SEQ ID NO:6; and
b) an immunoglobulin light chain variable region comprising a CDR1 having an amino acid sequence set forth in SEQ ID NO: 1, a CDR2 having an amino acid sequence set forth in SEQ ID NO:2, and a CDR3 having an amino acid sequence set forth in SEQ ID NO:3.

In a 12^{th} specific embodiment, for the immunoconjugates of the 1^{st} to 11^{th} specific embodiments, the CBA is an anti-CD123 antibody or antigen-binding fragment thereof comprising a V_{H} sequence of SEQ ID NO:7 and a V_{L} sequence of SEQ ID NO:9.

In a 13^{th} specific embodiment, for the immunoconjugates of the 1^{st} to 11^{th} specific embodiments, the CBA is an anti-CD123 antibody comprising: a) an immunoglobulin heavy chain having the amino acid sequence set forth in SEQ ID NO:8; and b) an immunoglobulin light chain having the amino acid sequence set forth in SEQ ID NO: 10.

In certain embodiments, for immunoconjugates described in the 1^{st} to 13^{th} specific embodiments above, wc is 2.

In certain embodiment, for immunoconjugates described herein (e.g., immunoconjugates described in the 1^{th}-13^{th} specific embodiments described above), the average ratio of the number of cytotoxic agent molecules represented Cy^{Cys} per antibody molecule *(i.e.,* the average value of wc, also referred to as "DAR") in a composition comprising the immunoconjugates is in the range of 1.5 to 2.1, 1.6 to 2.1, 1.7 to 2.1, 1.8 to 2.1, 1.5 to 2.0, 1.6 to 2.0, 1.7 to 2.0 or 1.8 to 2.0. In certain embodiments, DAR is 1.5, 1.6, 1.7, 1.8, 1.9,2.0, or 2.1.

In certain embodiments, the immuoconjugate of the present invention comprises a cell-binding agent (including antibody or antigen-binding fragment thereof) described herein covalently linked to a cytotoxic agent described herein through the ε-amino group of one or more lysine residues located on the cell-binding agent (CBA) (such as the immunoconjugates described in the 14^{th}-26^{th} specific embodiments below).

In a 14^{th} specific embodiment, the immunoconjugate is represented by the following formula: 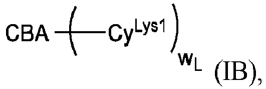 wherein:
CBA is an antibody or antigen-binding fragment thereof;
W_{L} is an integer from 1 to 20; and
Cy^{Lys1} is represented by the following formula: 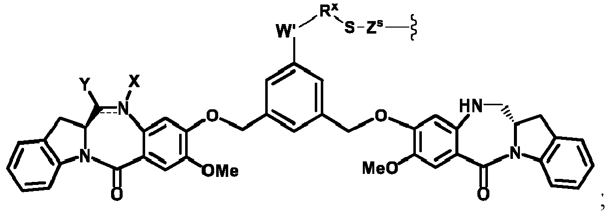
or a pharmaceutically acceptable salt thereof, wherein:
the double line -̅ -̅ between N and C represents a single bond or a double bond, provided that when it is a double bond, X is absent and Y is -H or a (C₁-C₄)alkyl; and when it is a single bond, X is -H or an amine protecting moiety, and Y is -OH or -SO₃H;
R^{x} is independently a (C₁-C₆)alkyl;
W' is -NR^{e},
R^{e} is -(CH₂-CH₂-O)ₙ₁-R^{k};
n1 is an integer from 2 to 6;
R^{k} is -H or -Me;
Z^{s} is selected from any one of the following formulas: 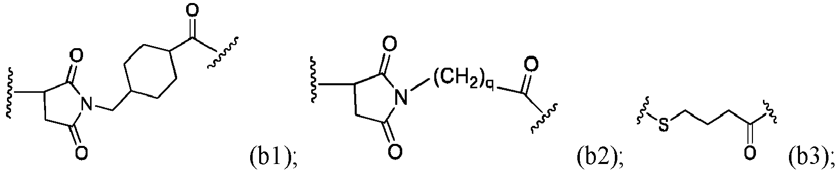 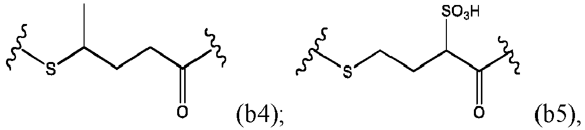 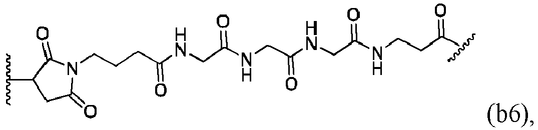 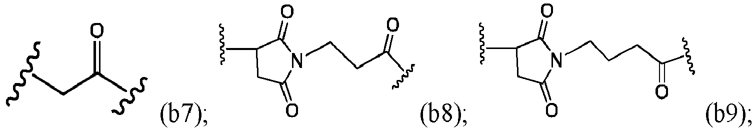 and 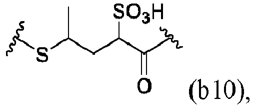
wherein:
   q is an integer from 1 to 5; and
   M⁺ is -H⁺ or a cation.

In a 15^{th} specific embodiment, for immunoconjugate of formula (IB), R^{x} is independently -(CH₂)ₚ-(CR^{f}R^{g})-, wherein R^{f} and R^{g} are each independently -H or a (C₁-C₄)alkyl; and p is 0, 1, 2 or 3; and the remaining variables are described in the 14^{th} specific embodiment.

In a 16^{th} specific embodiment, for immunoconjugate of formula (IB), R^{f} and R^{g} are the same or different, and are selected from -H and -Me; and the remaining variables are as described in the 15^{th} specific embodiment.

In a 17^{th} specific embodiment, for immunoconjugate of formula (IB), the immunoconjugate is represented by: 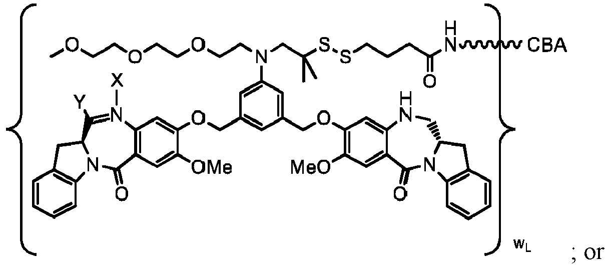 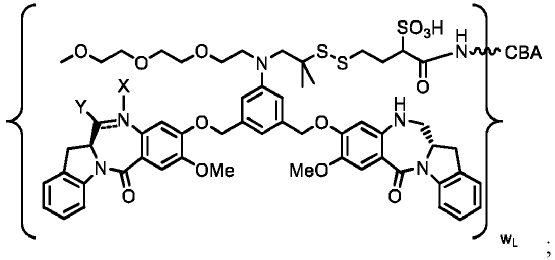 or a pharmaceutically acceptable salt thereof, wherein W_{L} is an integer from 1 to 10; the double line -̅ -̅ between N and C represents a single bond or a double bond, provided that when it is a double bond, X is absent and Y is -H; and when it is a single bond, X is -H and Y is -OH or -SO₃H; and the remaining variables are described in the 14^{th} specific embodiment.

In certain embodiments, the double line -̅ -̅ between N and C represents a single bond, X is -H, and Y is -SO₃H.

In a 18^{th} specific embodiment, for immunoconjugate of formula (IB), the immunoconjugate is represented by: 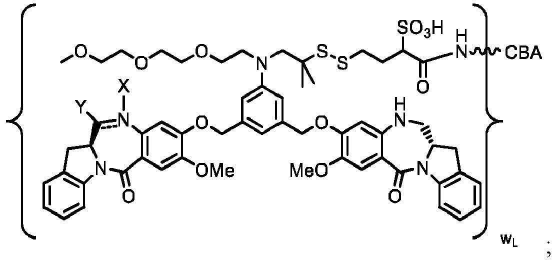 or a pharmaceutically acceptable salt thereof, wherein the variables are described in the 13^{th} specific embodiment.

In a 19^{th} specific embodiment, for the immunoconjugates of the 14^{th}-18^{th} specific embodiments, the CBA is an anti-CD33 antibody comprising an immunoglobulin heavy chain having the amino acid sequence set forth in SEQ ID NO: 18 and an immunoglobulin light chain having the amino acid sequence set forth in SEQ ID NO:20.

In a 20^{th} specific embodiment, the immunoconjugate is represented by the following formula: 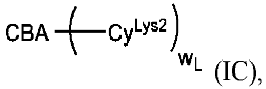 wherein:
CBA is an antibody or antigen-binding fragment thereof, wherein CBA is linked to Cy^{Lys1} through the ε-amino group of one or more lysine residues located on the CBA;
W_{L} is an integer from 1 to 20; and
Cy^{Lys1} is represented by the following formula: 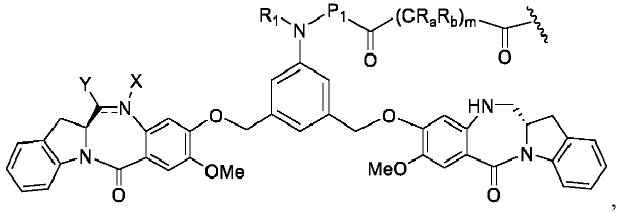
or a pharmaceutically acceptable salt thereof, wherein:
   the double line -̅ -̅ between N and C represents a single bond or a double bond, provided that when it is a double bond, X is absent and Y is -H or a (C₁-C₄)alkyl; and when it is a single bond, X is -H or an amine protecting moiety, Y is -OH or -SO₃H;
   R₁ is -H or a (C₁-C₃)alkyl;
   P₁ is an amino acid residue or a peptide containing 2 to 5 amino acid residues;
   Rₐ and R_{b}, for each occurrence, are independently -H, (C₁-C₃)alkyl, or a charged substituent or an ionizable group Q;
   m is an integer from 1 to 6.

In a 21^{st} specific embodiment, for immunoconjugate of formula (IC), Rₐ and R_{b} are both H; and R₁ is H or Me; and the remaining variables are as described in the 20^{th} specific embodiment.

In a 22^{nd} specific embodiment, for immunoconjugate of formula (IC), P is selected from Gly-Gly-Gly, Ala-Val, Val-Ala, Val-Cit, Val-Lys, Phe-Lys, Lys-Lys, Ala-Lys, Phe-Cit, Leu-Cit, Ile-Cit, Trp, Cit, Phe-Ala, Phe-N⁹-tosyl-Arg, Phe-N⁹-nitro-Arg, Phe-Phe-Lys, D-Phe-Phe-Lys, Gly-Phe-Lys, Leu-Ala-Leu, Ile-Ala-Leu, Val-Ala-Val, Ala-Leu-Ala-Leu (SEQ ID NO:21), β-Ala-Leu-Ala-Leu (SEQ ID NO:22), Gly-Phe-Leu-Gly (SEQ ID NO:23), Val-Arg, Arg-Val, Arg-Arg, Val-D-Cit, Val-D-Lys, Val-D-Arg, D-Val-Cit, D-Val-Lys, D-Val-Arg, D-Val-D-Cit, D-Val-D-Lys, D-Val-D-Arg, D-Arg-D-Arg, Ala-Ala, Ala-D-Ala, D-Ala-Ala, D-Ala-D-Ala, Ala-Met, and Met-Ala; and the remaining variables are as described in the 20^{th} or 21^{st} specific embodiment.

In a 23^{rd} specific embodiment, for immunoconjugate of formula (IC), P is Ala-Ala, Ala-D-Ala, D-Ala-Ala, or D-Ala-D-Ala; and the remaining variables are as described in the 20^{th} or 21^{st} specific embodiment.

In a 24^{th} specific embodiment, for immunoconjugate of formula (IC), Q is -SO₃H; and the remaining variables are as described in the 20^{th}, 21^{st}, 22^{nd} or 23^{rd} specific embodiment.

In a 25^{th} specific embodiment, for immunoconjugate of formula (IA), Q is H; and the remaining variables are as described in the 20^{th}, 21^{st}, 22^{nd} or 23^{rd} specific embodiment.

In a 26^{th} specific embodiment, for immunoconjugate of formula (IC), the immunoconjugate is represented by the following formula: 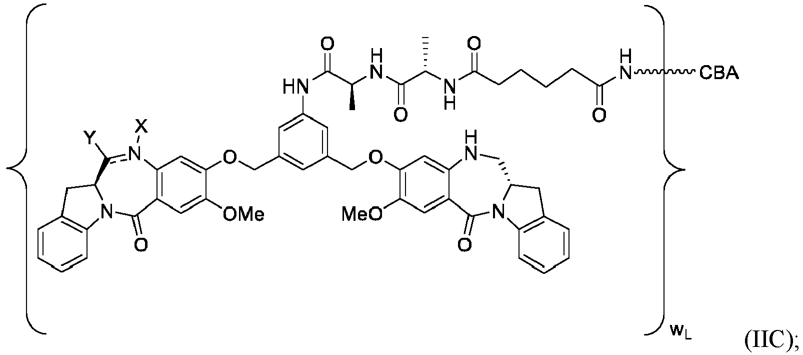 or a pharmaceutically acceptable salt thereof, wherein the double line -̅ -̅ between N and C represents a single bond or a double bond, provided that when it is a double bond, X is absent and Y is -H, and when it is a single bond, X is -H, and Y is -OH or -SO₃H; and the remaining variables are as described in the 20^{th} specific embodiment.

In certain embodiments, the double line -̅ -̅ between N and C represents a single bond, X is -H, and Y is -SO₃H. In certain embodiments, CBA is an antibody or an antigen-binding fragment thereof.

In certain embodiments, for immunoconjugates described herein (*e*.*g*., immunoconjugates described in the 14^{th}-26^{th} specific embodiments described above), the average ratio of the number of cytotoxic agent molecules represented Cy^{Lys1} or Cy^{Lys2} per antibody molecule (*i.e.*, the average value of w_{L}) in a composition (also referred to as "DAR") comprising the immunoconjugates is in the range of 1.0 to 5.0, 1.5 to 4.0, 2.0 to 3.5 or 2.5 to 3.0. In certain embodiments, DAR is 2.0, 2.1, 2.2, 2.3, 2.4, 2.5, 2.6, 2.7, 2.8, 2.9 or 3.0. In certain embodiments, DAR is 2.8 or 2.7.

In one embodiment, the DAR value for the immunoconjugate described in the 19^{th} specific embodiment is in the range of 2.4 to 3.0. In one embodiment, DAR is 2.4, 2.5, 2.6, 2.7, 2.8, 2.9 or 3.0. In another embodiment, the DAR value is 2.8.

### 7. Methods of Use

The pharmaceutical compositions described herein can be administered in any number of ways for either local or systemic treatment. Administration can be topical (such as to mucous membranes including vaginal and rectal delivery) such as transdermal patches, ointments, lotions, creams, gels, drops, suppositories, sprays, liquids and powders; pulmonary (*e.g.,* by inhalation or insufflation of powders or aerosols, including by nebulizer; intratracheal, intranasal, epidermal and transdermal); oral; or parenteral including intravenous, intraarterial, subcutaneous, intraperitoneal or intramuscular injection or infusion; or intracranial (*e.g.,* intrathecal or intraventricular) administration. In some particular embodiments, the administration is intravenous. The pharmaceutical compositions described herein can also be used *in vitro* or in *ex vivo.*

The pharmaceutical composition of the present invention can be used with a second compound, such as one that is known to be effective in treating a disease or disorder of interest, as combination therapy. In some embodiments, the second compound is an anti-cancer agent. In some embodiments, the methods encompass administration of the second compound and the pharmaceutical composition of the invention that results in a better efficacy as compared to administration of the pharmaceutical composition alone. The second compound can be administered via any number of ways, including for example, topical, pulmonary, oral, parenteral, or intracranial administration. In some embodiments, the administration is oral. In some embodiments, the administration is intravenous. In some embodiments, the administration is both oral and intravenous.

The pharmaceutical composition of the present invention can also be combined in a pharmaceutical combination formulation, or dosing regimen as combination therapy, with an analgesic, or other medications.

The pharmaceutical composition of the present invention can be combined in a pharmaceutical combination formulation, or dosing regimen as combination therapy, with a second compound having anti-cancer properties. The second compound of the pharmaceutical combination formulation or dosing regimen can have complementary activities to the ADC of the combination such that they do not adversely affect each other.

The present invention includes a method of inhibiting abnormal cell growth or treating a proliferative disorder in a mammal (*e.g.,* human) comprising administering to said mammal a therapeutically effective amount of the pharmaceutical composition of the present invention, alone or in combination with a second therapeutic agent.

In certain embodiments, the abnormal cell growth or proliferative disorder in a mammal is cancer, including hematologic cancer, leukemia, or lymphoma. In certain embodiments, the proliferative disorder is a cancer of a lymphatic organ, or a hematological malignancy.

For example, the cancer may be selected from the group consisting of: acute myeloid leukemia (AML, including CD33-low AML, P-glycoprotein positive AML, relapsed AML, or refractory AML), chronic myelogenous leukemia (CML), including blastic crisis of CML and Abelson oncogene associated with CML (Bcr-ABL translocation), myelodysplastic syndrome (MDS), acute lymphoblastic leukemia (ALL), including, but not limited to, acute B lymphoblastic leukemia or B-cell acute lymphoblastic leukemia (B-ALL), chronic lymphocytic leukemia (CLL), including Richter's syndrome or Richter's transformation of CLL, hairy cell leukemia (HCL), acute promyelocytic leukemia (APL), B-cell chronic lymphoproliferative disease (B-CLPD), atypical chronic lymphocytic leukemia (preferably with a marked CD11c expression), diffuse large B-cell lymphoma (DLBCL), blastic plasmacytoid dendritic cell neoplasm (BPDCN), non-Hodgkin lymphomas (NHL), including mantel cell leukemia (MCL), and small lymphocytic lymphoma (SLL), Hodgkin's lymphoma, systemic mastocytosis, and Burkitt's lymphoma.

In certain embodiments, the B-ALL is a CD19 positive B-ALL. In certain other embodiments, the B-ALL is a CD 19 negative B-ALL.

In certain embodiments, the cancer has at least one negative prognostic factor, e.g., overexpression of P-glycoprotein, overexpression of EVI1, a p53 alteration, DNMT3A mutation, FLT3 internal tandem duplication.

Cancer therapies and their dosages, routes of administration and recommended usage are known in the art and have been described in such literature as the Physician's Desk Reference (PDR). The PDR discloses dosages of the agents that have been used in treatment of various cancers. The dosing regimen and dosages of these aforementioned chemotherapeutic drugs that are therapeutically effective will depend on the particular cancer being treated, the extent of the disease and other factors familiar to the physician of skill in the art and can be determined by the physician. The contents of the PDR are expressly incorporated herein in its entirety by reference. One of skill in the art can review the PDR, using one or more of the following parameters, to determine dosing regimen and dosages of the chemotherapeutic agents and conjugates that can be used in accordance with the teachings of this invention. These parameters include: Comprehensive index; Manufacturer; Products (by company's or trademarked drug name); Category index; Generic/chemical index (non-trademark common drug names); Color images of medications; Product information, consistent with FDA labeling; Chemical information; Function/action; Indications & Contraindications; Trial research, side effects, warnings.

Examples of *in vitro* uses include treatments of autologous bone marrow prior to their transplant into the same patient in order to kill diseased or malignant cells: treatments of bone marrow prior to their transplantation in order to kill competent T cells and prevent graft-versus-host-disease (GVHD); treatments of cell cultures in order to kill all cells except for desired variants that do not express the target antigen; or to kill variants that express undesired antigen.

The conditions of non-clinical *in vitro* use are readily determined by one of ordinary skill in the art.

Examples of clinical *ex vivo* use are to remove tumor cells or lymphoid cells from bone marrow prior to autologous transplantation in cancer treatment or in treatment of autoimmune disease, or to remove T cells and other lymphoid cells from autologous or allogenic bone marrow or tissue prior to transplant in order to prevent GVHD. Treatment can be carried out as follows. Bone marrow is harvested from the patient or other individual and then incubated in medium containing serum to which is added the pharmaceutical composition of the invention, with concentrations for the immunoconjugates range from about 10 µM to 1 pM, for about 30 minutes to about 48 hours at about 37°C. The exact conditions of concentration and time of incubation, i.e., the dose, are readily determined by one of ordinary skill in the art. After incubation the bone marrow cells are washed with medium containing serum and returned to the patient intravenously according to known methods. In circumstances where the patient receives other treatment such as a course of ablative chemotherapy or total-body irradiation between the time of harvest of the marrow and reinfusion of the treated cells, the treated marrow cells are stored frozen in liquid nitrogen using standard medical equipment.

For clinical *in vivo* use, the cytotoxic compounds or conjugates of the invention will be supplied as a solution or a lyophilized powder that are tested for sterility and for endotoxin levels.

The method of the invention for inducing cell death in selected cell populations, for inhibiting cell growth, and/or for treating cancer, can be practiced *in vitro*, *in vivo*, or ex vivo.

### EXAMPLES

### Example 1. Addition of Methionine-based Antioxidants in IMGN632 Conjugation Reaction

G4723A antibody bearing two engineered cysteine residues (at the C442 position in the heavy chain CH3 region) in the reduced state was prepared by complete reduction and reoxidation of interchain disulfide bonds by known methods. To a solution of this intermediate in 50 mM potassium phosphate, 50 mM sodium chloride pH 6.0 was added 10 molar equivalents of *N*-acetylmethionine, *N*,*N*-dimethylacetamide (DMA), propylene glycol, and 10 molar equivalents of sDGN549-C to give a reaction mixture with a final solvent composition of 2% v/v DMA and 38% v/v propylene glycol in 50 mM potassium phosphate, 50 mM sodium chloride pH 6.0. The reaction was allowed to proceed overnight at 25 °C.

The conjugate was purified into 10 mM succinate, 8% trehalose dihydrate, 1 mM methionine, 0.01% Tween-20, 50 µM sodium bisulfite pH 4.2 formulation buffer using Sephadex G25 desalting columns, concentrated by ultrafiltration through a regenerated cellulose membrane with 10 kDa molecular weight cutoff, and filtered through a 0.22 µm syringe filter. The conjugate was dialyzed against the same formulation buffer using a dialysis cassette with 10 kDa molecular weight cutoff and filtered again through a 0.22 µm syringe filter.

The purified conjugate was found to have an average of 2 mol sDGN549/mol antibody by UV-Vis; 97.7% monomer by SEC; and 0.7% unconjugated DGN549 by tandem SEC-C18 RPLC. As seen in Table 1, methionine additives do not affect conjugation yield or conjugate quality as compared to conjugation reaction without the additives. In addition, the inclusion of methionine additives in reaction and methionine in formulation appear to reduce the percentage oxidation of Met256 (absolution numbering or Met252 in EU numbering).

Lysine-linked conjugation of antibody to cytotoxic agent follows similar reaction conditions except 10 molar equivalents of *N*-acetylmethionine methyl ester and 5 molar equivalents of DGN549-L (pretreated with a 5-fold excess of sodium bisulfite in a 95:5 mixture of DMA and 50 mM succinate pH 5.5 for 4 hours at 25 °C) are used.

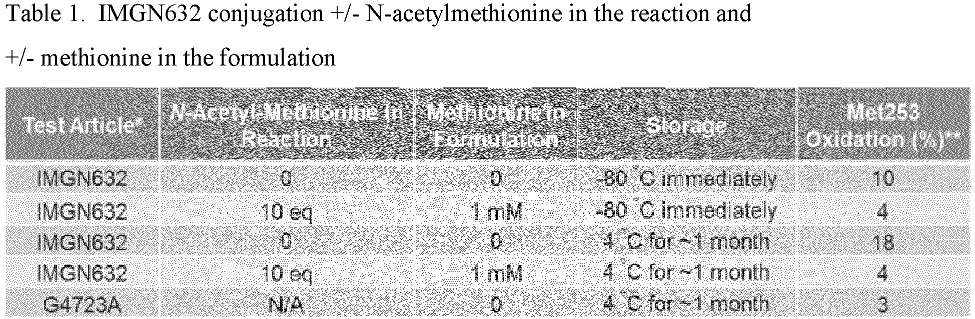

### Example 2. Methionine Oxidation and Photostability Studies of IMGN632

To further investigate the cause of high levels of methionine oxidation observed in IMGN632 samples and to determine methods for reduce methionine oxidation levels, three experiments were conducted: 1) a freeze thaw and stability study, 2) a methionine spiking study, and 3) a white light experiment study.

In study 1, the possible impact of storage vial types on oxidation levels were assessed, the use of nitrogen overlay to reduce oxidation levels were determined, the impact of temperature on oxidation was assessed, exposure to different wavelengths of light were assessed, and the impact of freeze thaw cycles were assessed. As seen in Table 2, initial experimental trends show that there was no clear significant impact of vial types on methionine oxidation levels. Nitrogen overlay may be protective but the results of these experiments were not conclusive. There was also no significant impact of temperature on methionine oxidation. Table 3 provides the results of UV and bench top light exposure on methionine oxidation levels. Exposure to light on the bench top or UV light causes high levels of methionine oxidation after 6 hours. These results suggest that protection from light by wrapping the vials in aluminum foil may be an efficient way to prevent levels of oxidation over time. Table 4 shows that exposure to light during freeze thaw (2 hours per cycle) causes oxidation but freeze thaw itself with light protection has no significant impact on methionine oxidation.

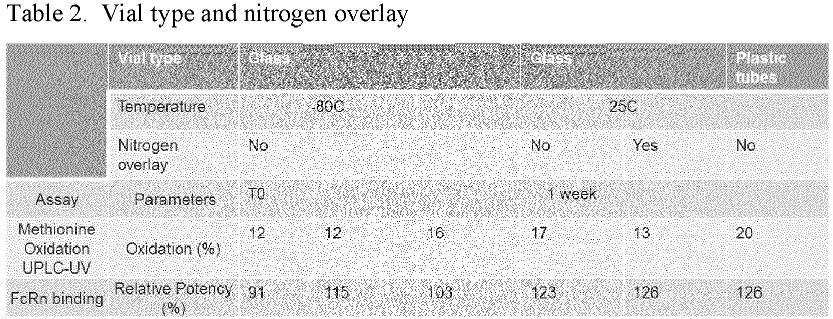

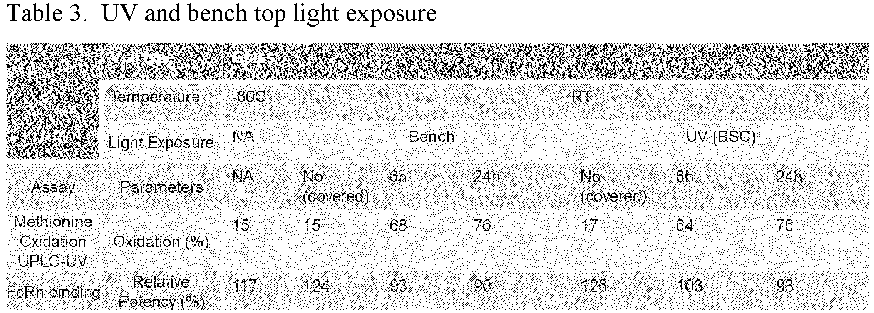

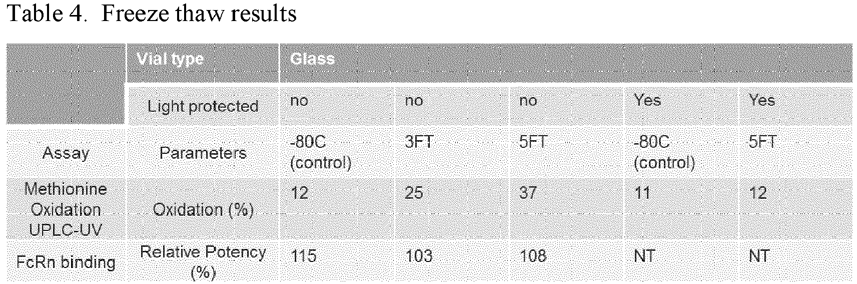

In study 2, spiking of different concentrations of methionine in the formulation of IMGN632 and antibody alone (G4723A) and its effect on oxidation were tested at room temperature with 6 hours of UV exposure. As seen in Table 5, methionine spiking at levels of 1-3mM in the formulation buffer can prevent methionine oxidation in the immunoconjugate formulation. Surprisingly, G4723A antibody alone diluted in immunoconjugate formulation buffer and light exposed did not undergo oxidation suggesting methionine oxidation in the antibody is related to the conjugation process or the presence of the payload when the antibody is present as an immunoconjugate in solution.

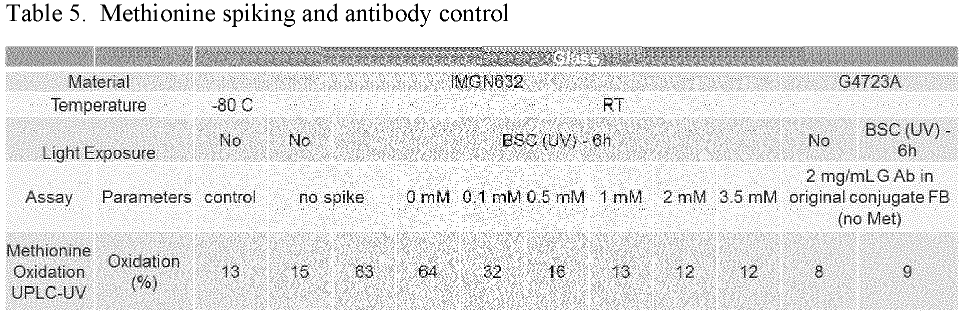

In study 3, immunoconjugate samples were exposed to 4kLux of white light at 25°C for 72 hours with 0mM, 1mM, or 3mM methionine in the formulation. The percent methionine oxidation, percent monomer, percent high molecular weight, free drug concentration, and DAR were measured.

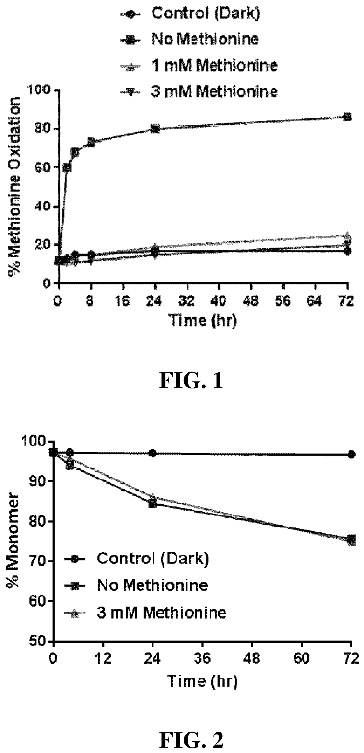

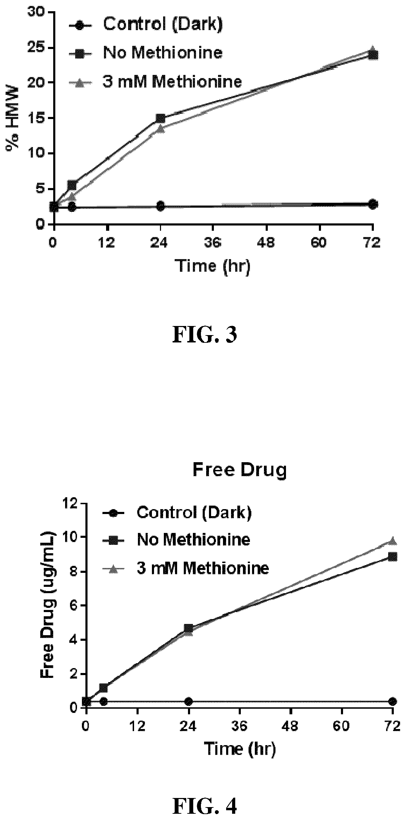

Figure 1 shows that the addition of at least 1mM methionine in formulation can reduce the levels of methionine oxidation over time when the solution is exposed to light. These results further support the addition of methionine (between 1mM to 3mM methionine) in the formulation of immunoconjugates containing DNA alkylating payloads such as DGN549. Figures 2 and 3 show that light exposure over time results in a slight decrease in percent monomer in the presence or absence of methionine in the formulation and an increase in percent of high molecular weight species in the presence or absence of methionine in the formulation. Figure 4 shows that light exposure causes degradation of the payload (DGN549) resulting in increased free drug. However, as seen in Table 6, there is minimal to no impact of light exposure on concentration/DAR.

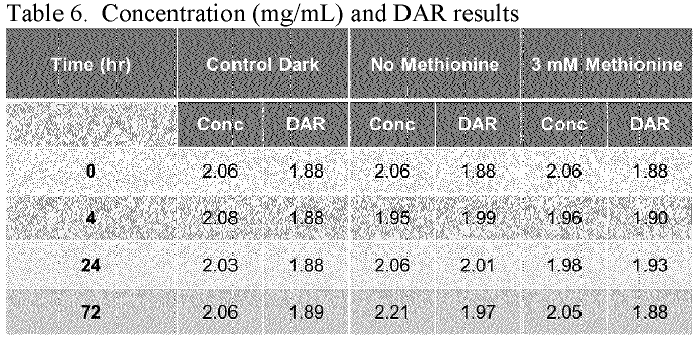

### Example 3. Monitoring of methionine oxidation in immunoconjugates by UV detection

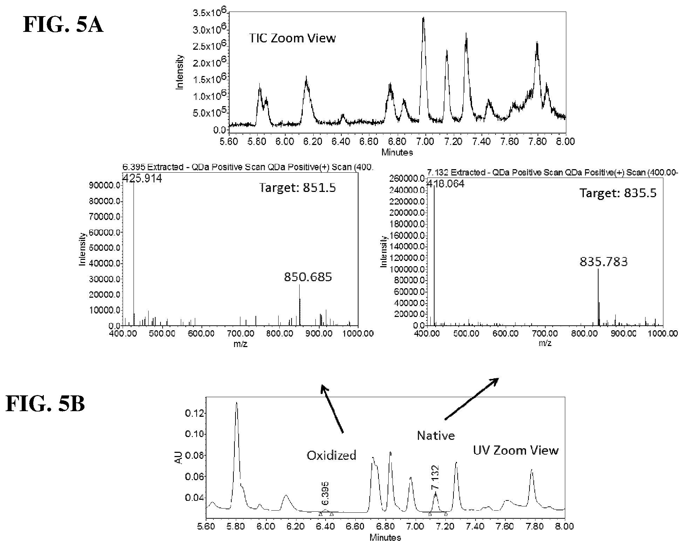

An analytical method for monitoring methionine oxidation of the G4723A antibody bearing two engineered cysteine residues (at position 442 in the heavy chain CH3 region) by UV detection was developed. Conditions for a platform peptide mapping with mass spectrometric detection method were optimized to replace the mass spectrometric detection step with UV detection. As seen in FIGs 5A and 5B, oxidized and native tryptic peptides can, in principle, be identified using mass spectrometry as well as UV detection methods, therefore, optimization of the platform method would allow the interchangeable use of UV detection to monitor methionine oxidation. The optimized conditions for the platform method consisted of the following steps:
- Sample denaturation using 4.5M guanidine HCl, 1.2M Tris, 10mM EDTA, 7mM DTT, pH7.8 for 10-15 minutes at 70°C
- Sample alkylation using 7µL 1M indole-3-acetic acid (IAA) for 45 minutes in the dark at room temperature without DTT quenching
- Buffer exchange with Illustra NAP-5 purification column loading full reaction volume (507 µL), washing with 400 µL, and eluting with 300 µL in 50MM Tris, 10mM calcium chloride, SmM methionine, pH 8.0
- Digestion with trypsin with a trypsin: antibody ratio of 1:33 for 1 hour at 37°C, sample quenching with TFA and transfer to HPLC vials
- Sample analysis on UPLC with UV detection

Using the above steps, methionine oxidation can be successfully quantified by UPLC-UV detection with similar results in the oxidation range of interest to the peptide mapping-mass spectrometric detection method. In particular, the peptide mapping steps were optimized with respect to sample preparation and sample run time (i.e., shorter preparation and run time). Further, the sensitivity of the UV detection steps was improved by optimizing the buffer exchange elution/collection volume (i.e., more concentrated sample collected) and increasing sample injection volume (i.e., between 10-50 µL injection volume).

### Example 4. Photostabilities studies of IMGN632 and IMGN779

Immunoconjugate samples of IMGN 632 and IMGN779 with or without methionine were exposed to ~1000 lux of white light at room temperature over time. Percentage of methione oxidation at Met252 was measured over time. The photostabilities of the following immunoconjugate samples were tested:
(1) IMGN779 without methionine: 2 mg/ml IMGN779, 20 mM histidine, 8.0% (w/v) trehalose dihydrate (also referred to as 7.2% w/v trehalose), 0.02% (w/v) polysorbate 20, and 50 µM sodium bisulfite, pH 6.1
(2) IMGN779 with methionine: 2 mg/ml IMGN779, 20 mM histidine, 8.0% (w/v) trehalose dihydrate (also referred to as 7.2% w/v trehalose), 0.02% (w/v) polysorbate 20, 50 µM sodium bisulfite, and 3 mM methionine, pH 6.1.
(3) IMGN632 without methionine: 2 mg/mL of IMGN632, 10 mM succinate, 50 µM sodium bisulfite, 8.0% (w/v) trehalose dihydrate (also referred to as 7.2% (w/v) trehalose), and 0.01% (w/v) polysorbate 20, pH 4.2.
(4) IMGN632 with methionine:2 mg/mL of IMGN632, 10 mM succinate, 50 µM sodium bisulfite, 8.0% (w/v) trehalose dihydrate (also referred to as 7.2% (w/v) trehalose), 0.01% (w/v) polysorbate 20, and 3 mM methionine, pH 4.2.

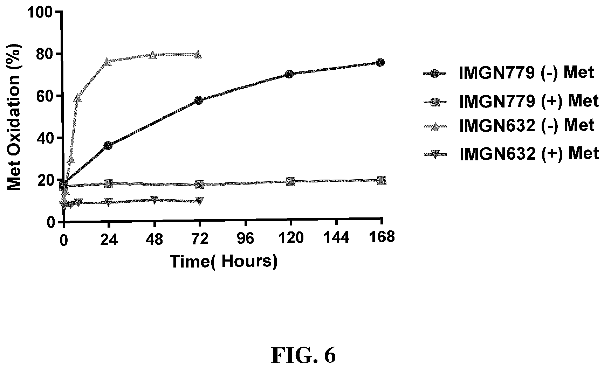

As shown in FIG. 6, when IMGN779 formulation (without 3 mM methionine) is exposed to ~1000 lux of white light at room temperature, Met252 residue oxidation increased from 18 to 74 % over 7 days. Under similar conditions, little or no increase in methionine oxidation was observed for the IMGN779 formulation containing 3 mM methionine. Similar results were observed for IMGN632 immunoconjugates.

The invention may be further understood with regard to the following non-limiting clauses:
1. A pharmaceutical composition comprising an immunoconjugate and 0.1 mM to 20 mM methionine, wherein the immunoconjugate is represented by the following formula: 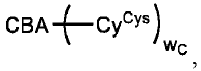
   CBA is an antibody or antigen-binding fragment thereof;
   Wc is 1 or 2; and
   Cy^{Cys} is represented by the following formula: 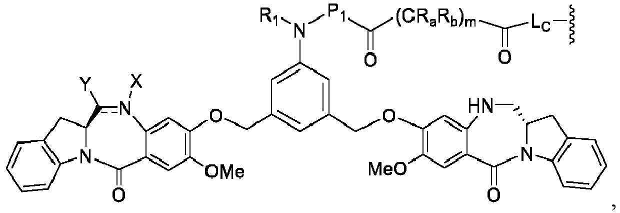
   or a pharmaceutically acceptable salt thereof, wherein:
      the double line -̅ -̅ between N and C represents a single bond or a double bond, provided that when it is a double bond, X is absent and Y is -H or a (C₁-C₄)alkyl; and when it is a single bond, X is -H or an amine protecting moiety, Y is -OH or -SO₃H;
      R₁ is -H or a (C₁-C₃)alkyl;
      P₁ is an amino acid residue or a peptide containing 2 to 5 amino acid residues;
      Rₐ and R_{b}, for each occurrence, are independently -H, (C₁-C₃)alkyl, or a charged substituent or an ionizable group Q;
      m is an integer from 1 to 6; L_{C} is represented by 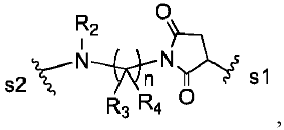 s1 is the site covalently linked to CBA, and s2 is the site covalently linked to the -C(=O)- group on Cy^{Cys}; wherein:
         R₂ is -H or a (C₁-C₃)alkyl
         R₃ and R₄, for each occurrence, are independently -H or a (C₁-C₃)alkyl; and
         n is an integer between 1 and 10.
2. The pharmaceutical composition of clause 1, wherein Rₐ and R_{b} are both H; and R₁ is H or Me.
3. The pharmaceutical composition of clause 1 or 2, wherein P is selected from Gly-Gly-Gly, Ala-Val, Val-Ala, Val-Cit, Val-Lys, Phe-Lys, Lys-Lys, Ala-Lys, Phe-Cit, Leu-Cit, Ile-Cit, Trp, Cit, Phe-Ala, Phe-N⁹-tosyl-Arg, Phe-N⁹-nitro-Arg, Phe-Phe-Lys, D-Phe-Phe-Lys, Gly-Phe-Lys, Leu-Ala-Leu, Ile-Ala-Leu, Val-Ala-Val, Ala-Leu-Ala-Leu (SEQ ID NO:21), β-Ala-Leu-Ala-Leu (SEQ ID NO:22), Gly-Phe-Leu-Gly (SEQ ID NO:23), Val-Arg, Arg-Val, Arg-Arg, Val-D-Cit, Val-D-Lys, Val-D-Arg, D-Val-Cit, D-Val-Lys, D-Val-Arg, D-Val-D-Cit, D-Val-D-Lys, D-Val-D-Arg, D-Arg-D-Arg, Ala-Ala, Ala-D-Ala, D-Ala-Ala, D-Ala-D-Ala, Ala-Met, and Met-Ala.
4. The pharmaceutical composition of clause 3, wherein P is Ala-Ala, Ala-D-Ala, D-Ala-Ala, or D-Ala-D-Ala.
5. The pharmaceutical composition of any one of clauses 1-4, wherein Q is - SO₃H.
6. The pharmaceutical composition of any one of clauses 1-4, wherein Q is H.
7. The pharmaceutical composition of any one of clauses 1-6, wherein R₃ and R₄ are both H; and n is an integer from 1 to 6.
8. The pharmaceutical composition of any one of clauses 1-7, wherein -L_{C}- is represented by the following formula: 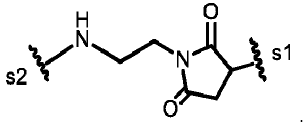
9. The pharmaceutical composition of clause 1, wherein the immunoconjugate is represented by the following formula: 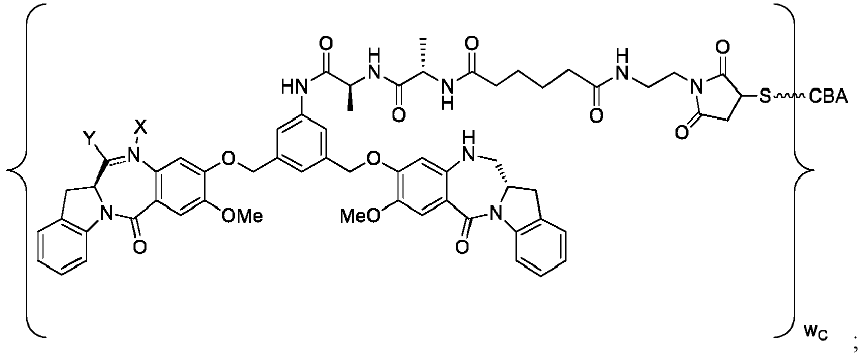 or a pharmaceutically acceptable salt thereof, wherein the double line -̅ -̅ between N and C represents a single bond or a double bond, provided that when it is a double bond, X is absent and Y is -H, and when it is a single bond, X is -H, and Y is -OH or -SO₃H..
10. The pharmaceutical composition of any one of clauses 1-9, wherein CBA is an antibody or an antigen-binding fragment thereof that (a) binds an epitope within amino acids 101 to 346 of human CD123 / IL3-Rα antigen, and (b) inhibits IL3-dependent proliferation in antigen-positive TF-1 cells.
11. The pharmaceutical composition of clause 10, wherein the antibody or antigen-binding fragment thereof comprises:
   a) an immunoglobulin heavy chain variable region comprising a CDR1 having an amino acid sequence set forth in SEQ ID NO:4, a CDR2 having an amino acid sequence set forth in SEQ ID NO:5, and a CDR3 having an amino acid sequence set forth in SEQ ID NO:6; and
   b) an immunoglobulin light chain variable region comprising a CDR1 having an amino acid sequence set forth in SEQ ID NO: 1, a CDR2 having an amino acid sequence set forth in SEQ ID NO:2, and a CDR3 having an amino acid sequence set forth in SEQ ID NO:3.
12. The pharmaceutical composition of clause 10, wherein the antibody or antigen-binding fragment thereof comprises a V_{H} sequence of SEQ ID NO:7 and a V_{L} sequence of SEQ ID NO:9.
13. The pharmaceutical composition of clause 10, wherein the antibody comprises:
   a) an immunoglobulin heavy chain having the amino acid sequence set forth in SEQ ID NO:8; and
   b) an immunoglobulin light chain having the amino acid sequence set forth in SEQ ID NO:10.
14. A pharmaceutical composition comprising an immunoconjugate and 0.1 mM to 20 mM methionine, wherein the immunoconjugate is represented by the following formula: 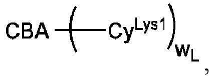 wherein:
   CBA is an antibody or antigen-binding fragment thereof;
   W_{L} is an integer from 1 to 20; and
   Cy^{Lys1} is represented by the following formula: 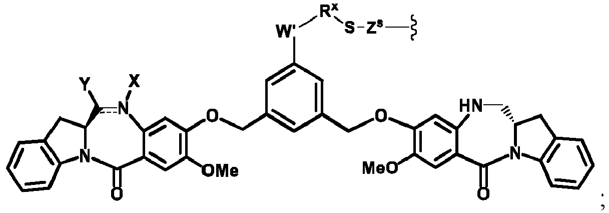
   or a pharmaceutically acceptable salt thereof, wherein:
      the double line -̅ -̅ between N and C represents a single bond or a double bond, provided that when it is a double bond, X is absent and Y is -H or a (C₁-C₄)alkyl; and when it is a single bond, X is -H or an amine protecting moiety, and Y is -OH or -SO₃H;
      R^{x} is independently a (C₁-C₆)alkyl;
      W' is -NR^{e},
      R^{e} is -(CH₂-CH₂-O)ₙ₁-R^{k};
      n1 is an integer from 2 to 6;
      R^{k} is -H or -Me;
      Z^{s} is selected from any one of the following formulas: 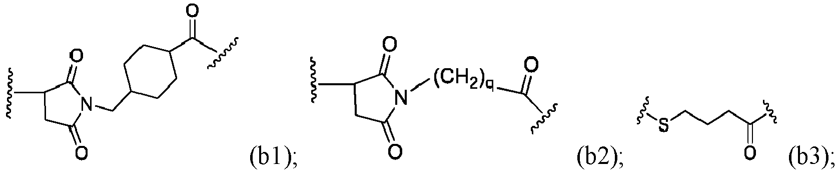 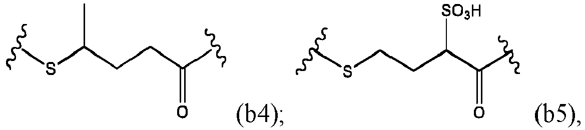 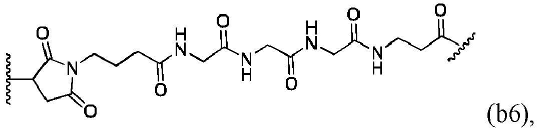 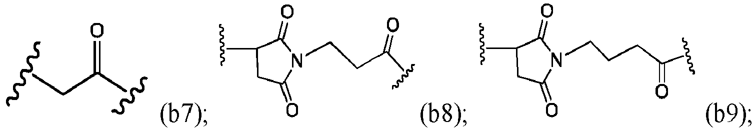 and 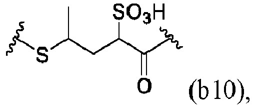
      or a pharmaceutically acceptable salt thereof, wherein q is an integer from 1 to 5.
15. The pharmaceutical composition of clause 14, wherein R^{x} is independently - (CH₂)ₚ-(CR^{f}R^{g})-, wherein R^{f} and R^{g} are each independently -H or a (C₁-C₄)alkyl; and p is 0, 1, 2 or 3.
16. The pharmaceutical composition of clause 15, wherein R^{f} and R^{g} are the same or different, and are selected from -H and -Me.
17. The pharmaceutical composition of clause 14, wherein the immunoconjugate is represented by: 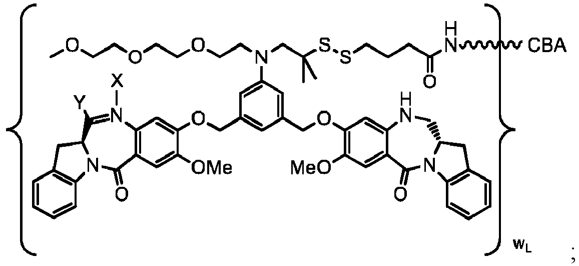 or 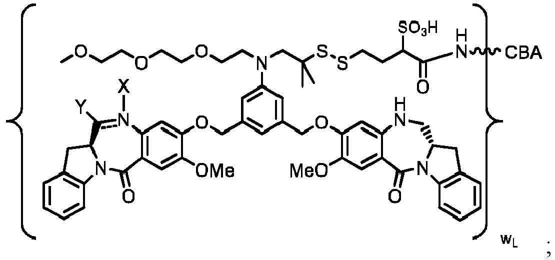 or a pharmaceutically acceptable salt thereof, wherein W_{L} is an integer from 1 to 10; the double line -̅ -̅ between N and C represents a single bond or a double bond, provided that when it is a double bond, X is absent and Y is -H; and when it is a single bond, X is -H and Y is -OH or -SO₃H.
18. The pharmaceutical composition of clause 17, wherein the immunoconjugate is represented by the following formula: 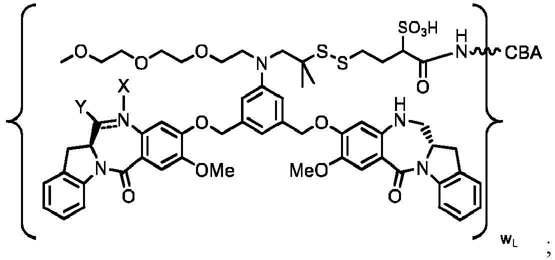 or a pharmaceutically acceptable salt thereof.
19. The pharmaceutical composition of any one of clauses 14-18, wherein the antibody is an anti-CD33 antibody comprising an immunoglobulin heavy chain having the amino acid sequence set forth in SEQ ID NO: 18 and an immunoglobulin light chain having the amino acid sequence set forth in SEQ ID NO:20.
20. The pharmaceutical composition of any one of clauses 1-19, wherein the double line -̅ -̅ between N and C represents a single bond, X is -H and Y is -SO₃H.
21. The pharmaceutical composition of any one of clauses 1-20, wherein Y is - SO₃H or the sodium or potassium salt thereof.
22. The pharmaceutical composition of any one of clauses 1-21, wherein the pharmaceutical composition comprises 0.1 mM to 10 mM methionine.
23. The pharmaceutical composition of clause 22, wherein the pharmaceutical composition comprises 0.5 mM to 5 mM methionine.
24. The pharmaceutical composition of clause 22, wherein the pharmaceutical composition comprises 1.0 mM to 4.0 mM methionine.
25. The pharmaceutical composition of clause 24, wherein the pharmaceutical composition comprises 3.0 mM methionine.
26. The pharmaceutical composition of any one of clauses 1-25, wherein when the pharmaceutical composition is exposed to light at room temperature for 6 hours or more, the immunoconjugate has less than 40%, less than 35%, 30%, less than 25%, less than 20% or less than 15% of methionine oxidation.
27. The pharmaceutical composition of any one of clauses 1-26, wherein the pharmaceutical composition is in a container that protects the pharmaceutical composition from light exposure.
28. The pharmaceutical composition of any one of clauses 1-27, wherein the pharmaceutical composition comprises 1 mg/mL to 10 mg/mL of the immunoconjugate.
29. The pharmaceutical composition of any one of clauses 1-27, wherein the pharmaceutical composition comprises 1 mg/mL to 5 mg/mL of the immunoconjugate.
30. The pharmaceutical composition of any one of clauses 1-29, further comprising sodium bisulfite.
31. The pharmaceutical composition of clause 30, wherein the pharmaceutical composition further comprises 10 µM to 100 µM, 20 µM to 90 µM, or 30 µM to 80 µM of sodium bisulfite.
32. The pharmaceutical composition of clause 30, wherein the pharmaceutical composition further comprises 40 µM to 60 µM of sodium bisulfite.
33. The pharmaceutical composition of clause 30, wherein the pharmaceutical composition further comprises 50 µM of sodium bisulfite.
34. A pharmaceutical composition comprising 1 mM to 4 mM of methionine and an immunoconjugate represented by the following formula: 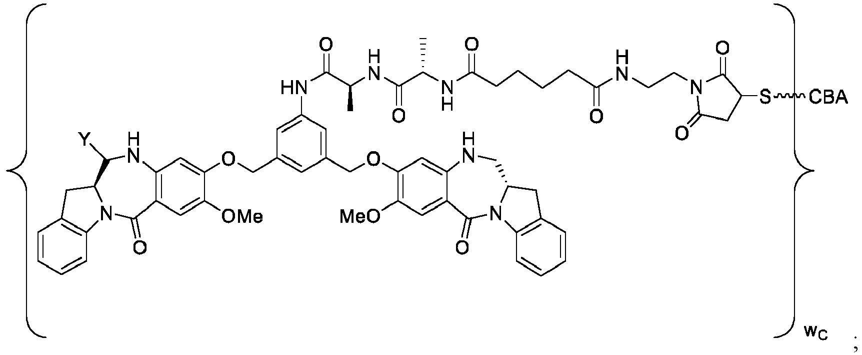 or a pharmaceutically acceptable salt thereof, wherein:
   Y is -SO₃H or sodium salt thereof;
   W_{C} is 2; and
   CBA is an anti-CD123 antibody comprising: a) an immunoglobulin heavy chain having the amino acid sequence set forth in SEQ ID NO:8; and b) an immunoglobulin light chain having the amino acid sequence set forth in SEQ ID NO: 10.
35. The pharmaceutical composition of clause 34, wherein the pharmaceutical composition comprises 3 mM methionine.
36. The pharmaceutical composition of clause 34 or 35, wherein when the pharmaceutical composition is exposed to light at room temperature for 6 hours, the immunoconjugate has less than 15% of methionine oxidation.
37. The pharmaceutical composition of any one of clauses 34-36, wherein the pharmaceutical composition is in a container that protects the pharmaceutical composition from light exposure.
38. The pharmaceutical composition of any one of clauses 34-37, wherein the pharmaceutical composition comprises 1 mg/mL to 5 mg/mL of the immunoconjugate.
39. The pharmaceutical composition of clause 38, wherein the pharmaceutical composition comprises 1 mg/mL to 3 mg/mL of the immunoconjugate.
40. The pharmaceutical composition of clause 38, wherein the pharmaceutical composition comprises 1.5 mg/mL to 2.5 mg/mL of the immunoconjugate.
41. The pharmaceutical composition of clause 38, wherein the pharmaceutical composition comprises 2 mg/mL of the immunoconjugate.
42. The pharmaceutical composition of any one of clauses 34-41, further comprising sodium bisulfite.
43. The pharmaceutical composition of clause 42, wherein the pharmaceutical composition further comprises 10 µM to 100 µM, 20 µM to 90 µM, or 30 µM to 80 µM of sodium bisulfite.
44. The pharmaceutical composition of clause 43, wherein the pharmaceutical composition further comprises 40 µM to 60 µM of sodium bisulfite.
45. The pharmaceutical composition of clause 43, wherein the pharmaceutical composition further comprises 50 µM of sodium bisulfite.
46. The pharmaceutical composition of any one of clauses 34-45, wherein the pharmaceutical composition further comprises trehalose, polysorbate 20, and succinate.
47. The pharmaceutical composition of any one of clauses 34-41, wherein the pharmaceutical composition further comprises 10 mM succinate, 50 µM sodium bisulfite, 7.2% (w/v) trehalose, and 0.01% (w/v) polysorbate 20.
48. The pharmaceutical composition of any one of clauses 34-47, wherein the pharmaceutical composition has a pH of 4 to 4.5.
49. The pharmaceutical composition of clause 48, wherein the pharmaceutical composition has a pH of 4.2.
50. A pharmaceutical composition comprising 1 mM to 4 mM of methionine and an immunoconjugate represented by the following formula: 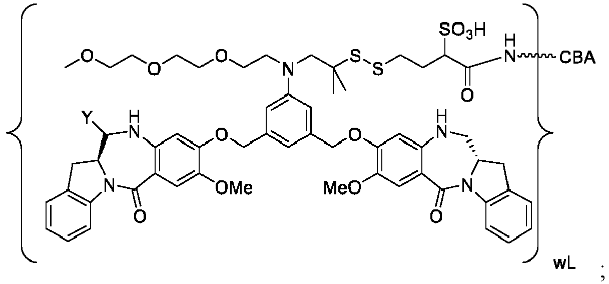 or a pharmaceutically acceptable salt thereof, wherein:
   Y is -SO₃H or sodium salt therof;
   W_{L} is an integer from 1 to 10; and
   CBA is an anti-CD33 antibody comprising an immunoglobulin heavy chain having the amino acid sequence set forth in SEQ ID NO:18 and an immunoglobulin light chain having the amino acid sequence set forth in SEQ ID NO:20.
51. The pharmaceutical composition of clause 50, wherein the pharmaceutical composition comprises 3 mM methionine.
52. The pharmaceutical composition of clause 50 or 51, wherein when the pharmaceutical composition is exposed to light at room temperature for 6 hours, the immunoconjugate has less than 15% of methionine oxidation.
53. The pharmaceutical composition of any one of clauses 50-52, wherein the pharmaceutical composition is in a container that protects the pharmaceutical composition from light exposure.
54. The pharmaceutical composition of any one of clauses 50-53, wherein the pharmaceutical composition comprises 1 mg/mL to 5 mg/mL of the immunoconjugate.
55. The pharmaceutical composition of clause 54, wherein the pharmaceutical composition comprises 1 mg/mL to 3 mg/mL of the immunoconjugate.
56. The pharmaceutical composition of clause 55, wherein the pharmaceutical composition comprises 1.5 mg/mL to 2.5 mg/mL of the immunoconjugate.
57. The pharmaceutical composition of clause 56, wherein the pharmaceutical composition comprises 2 mg/mL of the immunoconjugate.
58. The pharmaceutical composition of any one of clauses 50-57, further comprising sodium bisulfite.
59. The pharmaceutical composition of clause 58, wherein the pharmaceutical composition further comprises 10 µM to 100 µM, 20 µM to 90 µM, or 30 µM to 80 µM of sodium bisulfite.
60. The pharmaceutical composition of clause 59, wherein the pharmaceutical composition further comprises 40 µM to 60 µM of sodium bisulfite.
61. The pharmaceutical composition of clause 59, wherein the pharmaceutical composition further comprises 50 µM of sodium bisulfite.
62. The pharmaceutical composition of any one of clauses 50-61, wherein the pharmaceutical composition further comprises histidine, trehalose, and polysorbate 20.
63. The pharmaceutical composition of any one of clauses 50-57, wherein the pharmaceutical composition further comprises 20 mM histidine, 50 µM sodium bisulfite, 7.2% (w/v) trehalose, 0.02% (w/v) polysorbate 20.
64. The pharmaceutical composition of any one of clauses 50-63, wherein the pharmaceutical composition has a pH of 5.5 to 6.5.
65. The pharmaceutical composition of clause 64, wherein the pharmaceutical composition has a pH of 6.1.
66. A method of reducing the amount of methionine oxidation in an immunoconjugate comprising mixing the immunoconjugate with 0.1 mM to 20 mM methionine to give a pharmaceutical composition comprising the immunoconjugate and methionine, wherein the immunoconjugate is represented by the following formula:

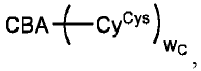

CBA is an antibody or antigen-binding fragment thereof;
Wc is 1 or 2; and
Cy^{Cys} is represented by the following formula: 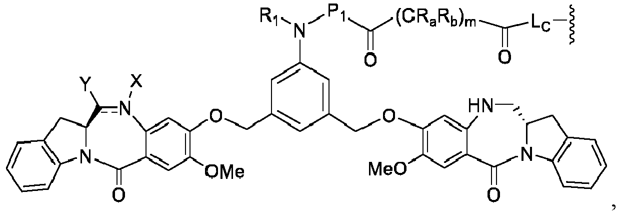 or a pharmaceutically acceptable salt thereof, wherein:
   the double line -̅ -̅ between N and C represents a single bond or a double bond, provided that when it is a double bond, X is absent and Y is -H or a (C₁-C₄)alkyl; and when it is a single bond, X is -H or an amine protecting moiety, Y is -OH or -SO₃H;
   R₁ is -H or a (C₁-C₃)alkyl;
   P₁ is an amino acid residue or a peptide containing 2 to 5 amino acid residues;
   Rₐ and R_{b}, for each occurrence, are independently -H, (C₁-C₃)alkyl, or a charged substituent or an ionizable group Q;
   m is an integer from 1 to 6; L_{C} is represented by 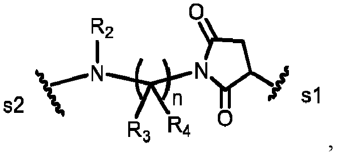 s1 is the site covalently linked to CBA, and s2 is the site covalently linked to the -C(=O)- group on Cy^{C1}; wherein:
   R₂ is -H or a (C₁-C₃)alkyl
   R₃ and R₄, for each occurrence, are independently -H or a (C₁-C₃)alkyl; and
   n is an integer between 1 and 10.
67. The method of clause 66, wherein Rₐ and R_{b} are both H; and R₁ is H or Me.
68. The method of clause 66 or 67, wherein P is selected from Gly-Gly-Gly, Ala-Val, Val-Ala, Val-Cit, Val-Lys, Phe-Lys, Lys-Lys, Ala-Lys, Phe-Cit, Leu-Cit, Ile-Cit, Trp, Cit, Phe-Ala, Phe-N⁹-tosyl-Arg, Phe-N⁹-nitro-Arg, Phe-Phe-Lys, D-Phe-Phe-Lys, Gly-Phe-Lys, Leu-Ala-Leu, Ile-Ala-Leu, Val-Ala-Val, Ala-Leu-Ala-Leu (SEQ ID NO:21), β-Ala-Leu-Ala-Leu (SEQ ID NO:22), Gly-Phe-Leu-Gly (SEQ ID NO:23), Val-Arg, Arg-Val, Arg-Arg, Val-D-Cit, Val-D-Lys, Val-D-Arg, D-Val-Cit, D-Val-Lys, D-Val-Arg, D-Val-D-Cit, D-Val-D-Lys, D-Val-D-Arg, D-Arg-D-Arg, Ala-Ala, Ala-D-Ala, D-Ala-Ala, D-Ala-D-Ala, Ala-Met, and Met-Ala.
69. The method of clause 68, wherein P is Ala-Ala, Ala-D-Ala, D-Ala-Ala, or D-Ala-D-Ala.
70. The method of any one of clauses 66-69, wherein Q is -SO₃H.
71. The method of any one of clauses 66-69, wherein Q is H.
72. The method of any one of clauses 66-71, wherein R₃ and R₄ are both H; and n is an integer from 1 to 6.
73. The method of any one of clauses 66-72, wherein -L_{C}- is represented by the following formula: 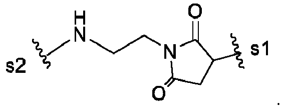
74. The method of clause 66, wherein the immunoconjugate is represented by the following formula: 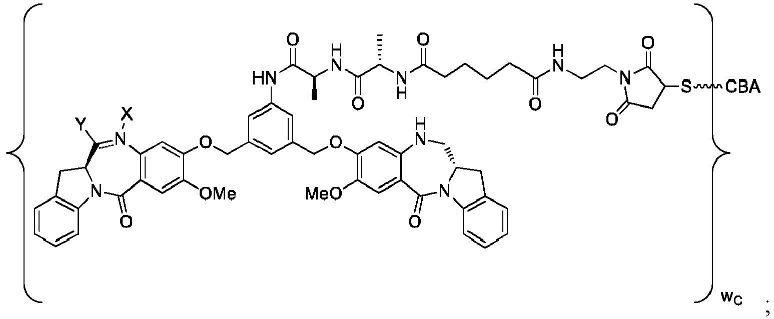 or a pharmaceutically acceptable salt thereof, wherein the double line -̅ -̅ between N and C represents a single bond or a double bond, provided that when it is a double bond, X is absent and Y is -H, and when it is a single bond, X is -H, and Y is -OH or -SO₃H.
75. The method of any one of clauses 66-74, wherein CBA is an antibody or an antigen-binding fragment thereof that (a) binds an epitope within amino acids 101 to 346 of human CD123 / IL3-Rα antigen, and (b) inhibits IL3-dependent proliferation in antigen-positive TF-1 cells.
76. The method of clause 75, wherein the antibody or antigen-binding fragment thereof comprises:
   a) an immunoglobulin heavy chain variable region comprising a CDR1 having an amino acid sequence set forth in SEQ ID NO:4, a CDR2 having an amino acid sequence set forth in SEQ ID NO:5, and a CDR3 having an amino acid sequence set forth in SEQ ID NO:6; and
   b) an immunoglobulin light chain variable region comprising a CDR1 having an amino acid sequence set forth in SEQ ID NO: 1, a CDR2 having an amino acid sequence set forth in SEQ ID NO:2, and a CDR3 having an amino acid sequence set forth in SEQ ID NO:3.
77. The method of clause 75, wherein the antibody or antigen-binding fragment thereof comprises a V_{H} sequence of SEQ ID NO:7 and a V_{L} sequence of SEQ ID NO:9.
78. The method of clause 75, wherein the antibody comprises:
   a) an immunoglobulin heavy chain having the amino acid sequence set forth in SEQ ID NO:8; and
   b) an immunoglobulin light chain having the amino acid sequence set forth in SEQ ID NO: 10.
79. A method of reducing the amount of methionine oxidation in an immunoconjugate comprising mixing the immunoconjugate with 0.1 mM to 20 mM methionine to give a pharmaceutical composition comprising the immunoconjugate and methionine, wherein the immunoconjugate is represented by the following formula: 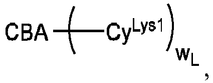 wherein:
   CBA is an antibody or antigen-binding fragment thereof;
   W_{L} is an integer from 1 to 20; and
   Cy^{Lys1} is represented by the following formula: 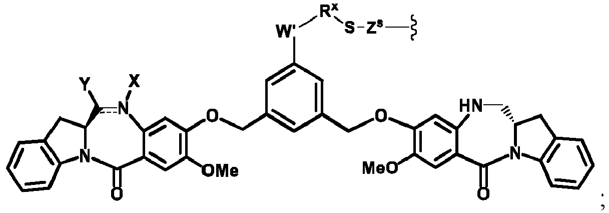
   or a pharmaceutically acceptable salt thereof, wherein:
      the double line -̅ -̅ between N and C represents a single bond or a double bond, provided that when it is a double bond, X is absent and Y is -H or a (C₁-C₄)alkyl; and when it is a single bond, X is -H or an amine protecting moiety, and Y is -OH or -SO₃H;
   R^{x} is independently a (C₁-C₆)alkyl;
   W' is -NR^{e},
   R^{e} is -(CH₂-CH₂-O)ₙ₁-R^{k};
   n1 is an integer from 2 to 6;
   R^{k} is -H or -Me;
   Z^{s} is selected from any one of the following formulas: 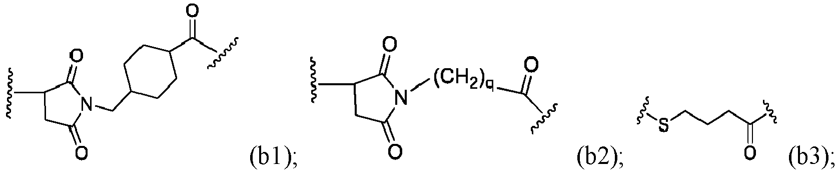 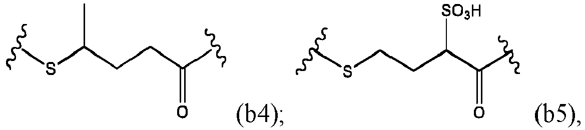 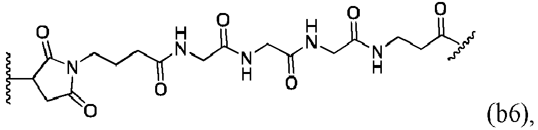 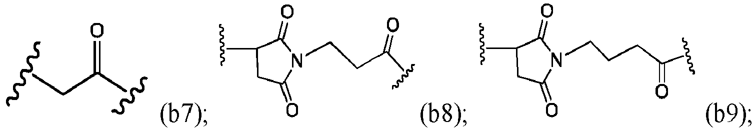 and 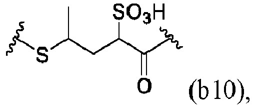
   wherein:
      q is an integer from 1 to 5; and
      M⁺ is -H⁺ or a cation.
80. The method of clause 79, wherein R^{x} is independently -(CH₂)ₚ-(CR^{f}R^{g})-, wherein R^{f} and R^{g} are each independently -H or a (C₁-C₄)alkyl; and p is 0, 1, 2 or 3.
81. The method of clause 80, wherein R^{f} and R^{g} are the same or different, and are selected from -H and -Me.
82. The method of clause 79, wherein the immunoconjugate is represented by: 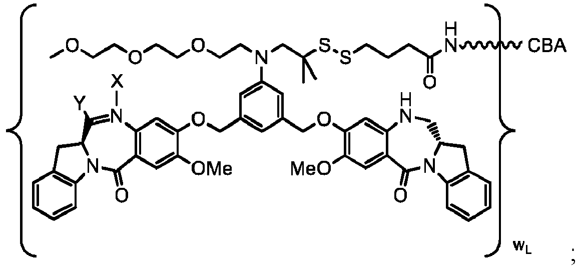 or 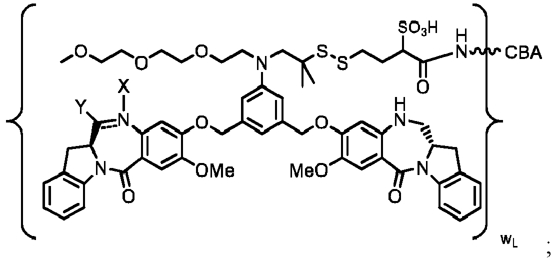 or a pharmaceutically acceptable salt thereof, wherein W_{L} is an integer from 1 to 10; the double line -̅ -̅ between N and C represents a single bond or a double bond, provided that when it is a double bond, X is absent and Y is -H; and when it is a single bond, X is -H and Y is -OH or -SO₃H.
83. The method of clause 82, wherein the immunoconjugate is represented by the following formula: 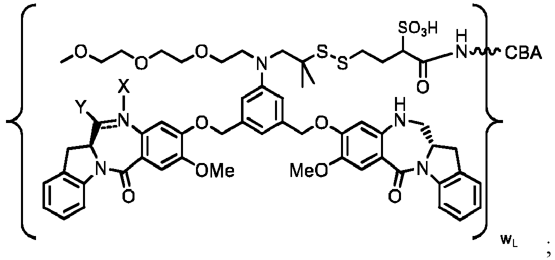 or a pharmaceutically acceptable salt thereof.
84. The method of any one of clauses 79-83, wherein the antibody is an anti-CD33 antibody comprising an immunoglobulin heavy chain having the amino acid sequence set forth in SEQ ID NO: 18 and an immunoglobulin light chain having the amino acid sequence set forth in SEQ ID NO:20.
85. The method of any one of clauses 66-84, wherein the double line -̅ -̅ between N and C represents a single bond, X is -H and Y is -SO₃H.
86. The method of any one of clauses 66-85, wherein Y is -SO₃H or sodium or potassium salt thereof.
87. The method of any one of clauses 66-86, wherein the pharmaceutical composition comprises 0.1 mM to 10 mM methionine.
88. The method of clause 87, wherein the pharmaceutical composition comprises 0.5 to 5 mM methionine.
89. The method of clause 87, wherein the pharmaceutical composition comprises 1.0 to 4.0 mM methionine.
90. The method of clause 89, wherein the pharmaceutical composition comprises 3.0 mM of methionine.
91. The method of any one of clauses 66-90, wherein when the pharmaceutical composition is exposed to light at room temperature for 6 hours or more, the immunoconjugate has less than 40%, less than 35%, less than 30%, less than 25%, less than 20% or less than 15% of methionine oxidation
92. The method of any one of clauses 66-91, wherein the pharmaceutical composition is in a container that protects the pharmaceutical composition from light exposure.
93. The method of any one of clauses 66-92, wherein the pharmaceutical composition comprises 1 mg/mL to 10 mg/mL of the immunoconjugate.
94. The method of any one of clauses 66-92, wherein the pharmaceutical composition comprises 1 mg/mL to 5 mg/mL of the immunoconjugate.
95. The method of any one of clauses 66-94, further comprising sodium bisulfite.
96. The method of clause 95, wherein the pharmaceutical composition further comprises 10 µM to 100 µM, 20 µM to 90 µM, or 30 µM to 80 µM of sodium bisulfite.
97. The method of clause 95, wherein the pharmaceutical composition further comprises 40 µM to 60 µM of sodium bisulfite.
98. The method of clause 95, wherein the pharmaceutical composition further comprises 50 µM of sodium bisulfite.
99. A method of preparing an immunoconjugate represented by the following formula: 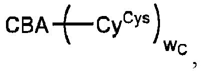 comprising reacting a CBA with a cytotoxic agent represented by the following formula: 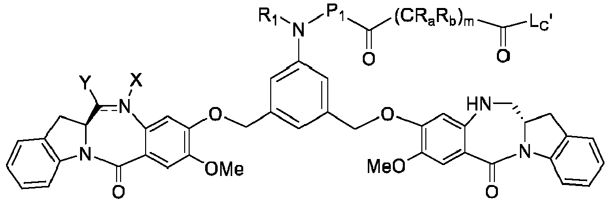 or a pharmaceutically acceptable salt thereof, in the presence of an antioxidant, wherein:
   CBA is an antibody or antigen-binding fragment thereof;
   Wc is 1 or 2; and
   Cy^{Cys} is represented by the following formula: 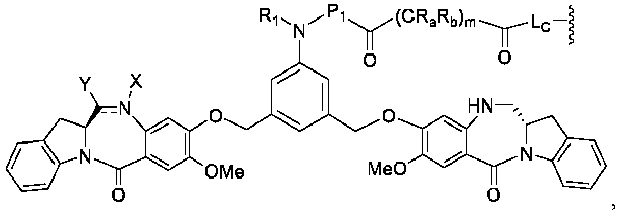
   or a pharmaceutically acceptable salt thereof, wherein:
      the double line -̅ -̅ between N and C represents a single bond or a double bond, provided that when it is a double bond, X is absent and Y is -H or a (C₁-C₄)alkyl; and when it is a single bond, X is -H or an amine protecting moiety, Y is -OH or -SO₃H;
      R₁ is -H or a (C₁-C₃)alkyl;
      P₁ is an amino acid residue or a peptide containing 2 to 5 amino acid residues;
      Rₐ and R_{b}, for each occurrence, are independently -H, (C₁-C₃)alkyl, or a charged substituent or an ionizable group Q;
      m is an integer from 1 to 6;
      L_{C} is represented by 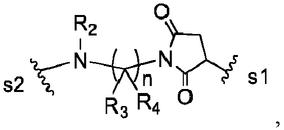 s1 is the site covalently linked to
   CBA, and s2 is the site covalently linked to the -C(=O)- group on Cy^{C1};
      R₂ is -H or a (C₁-C₃)alkyl
      R₃ and R₄, for each occurrence, are independently -H or a (C₁-C₃)alkyl; and n is an integer between 1 and 10; and
      L_{C}' is represented by 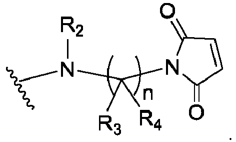
100. The method of clause 99, wherein the antioxidant reduces the amount of methionine oxidation in the immunoconjugate.
101. The method of clause 99, wherein the antioxidant is N-acetylmethionine.
102. The method of any one of clauses 99-101, wherein Rₐ and R_{b} are both H; and R₁ is H or Me.
103. The method of any one of clauses 99-102, wherein P is selected from Gly-Gly-Gly, Ala-Val, Val-Ala, Val-Cit, Val-Lys, Phe-Lys, Lys-Lys, Ala-Lys, Phe-Cit, Leu-Cit, Ile-Cit, Trp, Cit, Phe-Ala, Phe-N⁹-tosyl-Arg, Phe-N⁹-nitro-Arg, Phe-Phe-Lys, D-Phe-Phe-Lys, Gly-Phe-Lys, Leu-Ala-Leu, Ile-Ala-Leu, Val-Ala-Val, Ala-Leu-Ala-Leu (SEQ ID NO:21), β-Ala-Leu-Ala-Leu (SEQ ID NO:22), Gly-Phe-Leu-Gly (SEQ ID NO:23), Val-Arg, Arg-Val, Arg-Arg, Val-D-Cit, Val-D-Lys, Val-D-Arg, D-Val-Cit, D-Val-Lys, D-Val-Arg, D-Val-D-Cit, D-Val-D-Lys, D-Val-D-Arg, D-Arg-D-Arg, Ala-Ala, Ala-D-Ala, D-Ala-Ala, D-Ala-D-Ala, Ala-Met, and Met-Ala.
104. The method of clause 103, wherein P is Ala-Ala, Ala-D-Ala, D-Ala-Ala, or D-Ala-D-Ala.
105. The method of any one of clauses 99-104, wherein Q is -SO₃H.
106. The method of any one of clauses 99-104, wherein Q is H.
107. The method of any one of clauses 99-106, wherein R₃ and R₄ are both H; and n is an integer from 1 to 6.
108. The method of any one of clauses 99-107, wherein -L_{C}- is represented by the following formula: 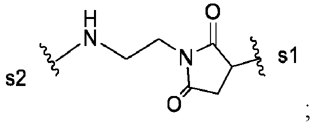 and
   - L_{C}' is represented by the following formula: 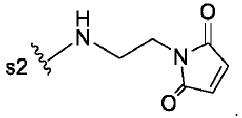
109. The method of any one of clauses 99-101, wherein the immunoconjugate is represented by the following formula: 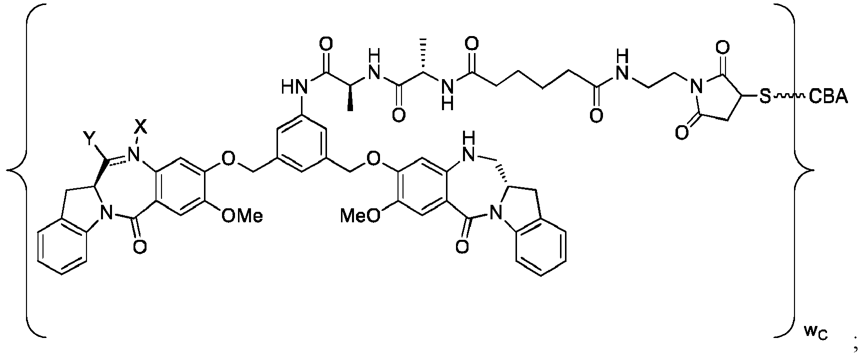 or a pharmaceutically acceptable salt thereof, wherein the double line -̅ -̅ between N and C represents a single bond or a double bond, provided that when it is a double bond, X is absent and Y is -H, and when it is a single bond, X is -H, and Y is -OH or -SO₃H.
110. The method of any one of clauses 99-109, wherein CBA is an antibody or an antigen-binding fragment thereof that (a) binds an epitope within amino acids 101 to 346 of human CD123 / IL3-Rα antigen, and (b) inhibits IL3-dependent proliferation in antigen-positive TF-1 cells.
111. The method of clause 110, wherein the antibody or antigen-binding fragment thereof comprises:
   a) an immunoglobulin heavy chain variable region comprising a CDR1 having an amino acid sequence set forth in SEQ ID NO:4, a CDR2 having an amino acid sequence set forth in SEQ ID NO:5, and a CDR3 having an amino acid sequence set forth in SEQ ID NO:6; and
   b) an immunoglobulin light chain variable region comprising a CDR1 having an amino acid sequence set forth in SEQ ID NO: 1, a CDR2 having an amino acid sequence set forth in SEQ ID NO:2, and a CDR3 having an amino acid sequence set forth in SEQ ID NO:3.
112. The method of clause 110, wherein the antibody or antigen-binding fragment thereof comprises a V_{H} sequence of SEQ ID NO:7 and a V_{L} sequence of SEQ ID NO:9.
113. The method of clause 110, wherein the antibody comprises:
   a) an immunoglobulin heavy chain having the amino acid sequence set forth in SEQ ID NO:8; and
   b) an immunoglobulin light chain having the amino acid sequence set forth in SEQ ID NO:10.
114. A method of preparing an immunoconjugate represented by the following formula: 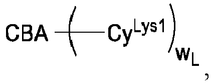 wherein:
   CBA is an antibody or antigen-binding fragment thereof;
   W_{L} is an integer from 1 to 20; and
   Cy^{Lys1} is represented by the following formula: 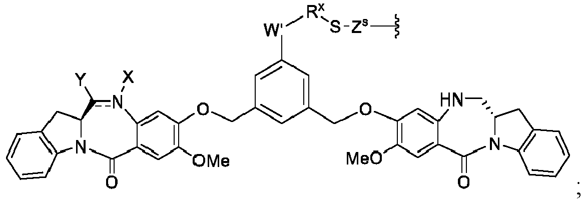
   or a pharmaceutically acceptable salt thereof, wherein:
   the double line -̅ -̅ between N and C represents a single bond or a double bond, provided that when it is a double bond, X is absent and Y is -H or a (C₁-C₄)alkyl; and when it is a single bond, X is -H or an amine protecting moiety, and Y is -OH or -SO₃H;
   R^{x} is independently a (C₁-C₆)alkyl;
   W' is -NR^{e},
   R^{e} is -(CH₂-CH₂-O)ₙ₁-R^{k};
   n1 is an integer from 2 to 6;
   R^{k} is -H or -Me;
   Z^{s} is selected from any one of the following formulas: 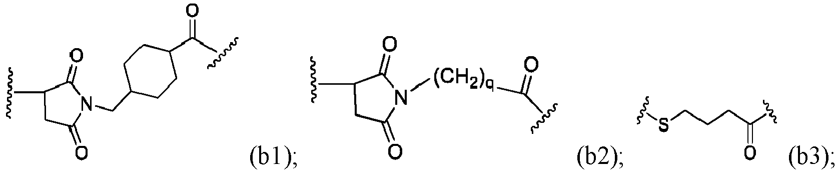 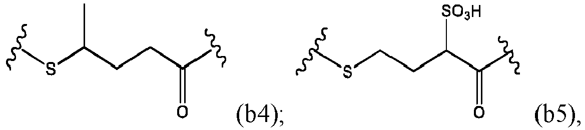 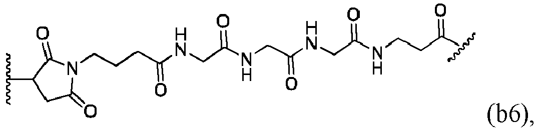 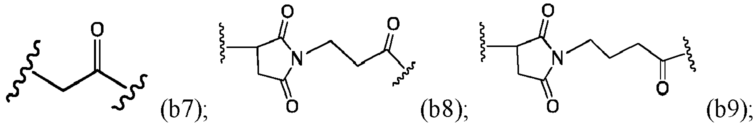 and 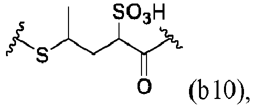
   or a pharmaceutically acceptable salt thereof, wherein q is an integer from 1 to 5, comprising the step of:
   (a) reacting a cytotoxic agent represented by the following formula: 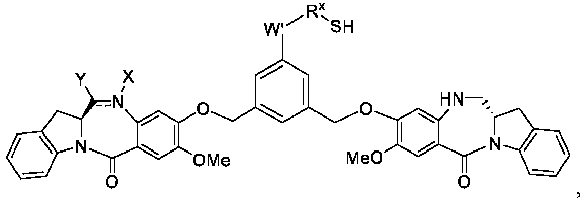 or a pharmaceutically acceptable salt thereof, with a bifunctional crosslinking agent selected from the following: 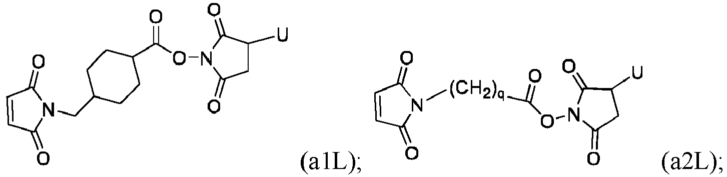 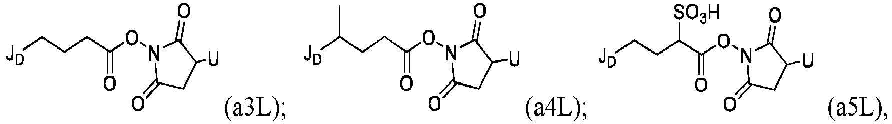 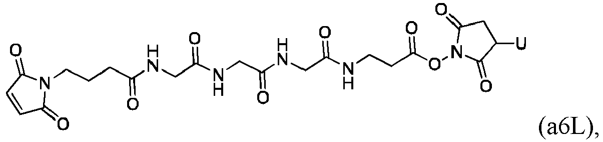 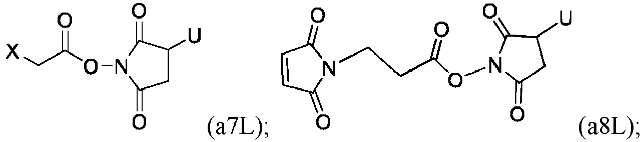 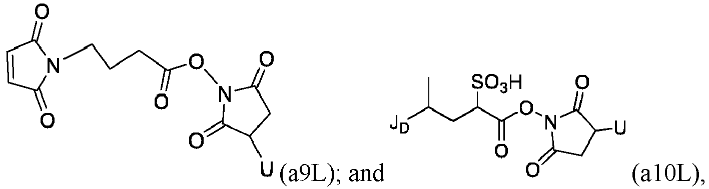 to form a cytotoxic agent-linker compound, wherein X is halogen; J_{D} -SH, -SSR^{d}, or - SC(=O)R^{g}; R^{d} is phenyl, nitrophenyl, dinitrophenyl, carboxynitrophenyl, pyridyl or nitropyridyl; R^{g} is an alkyl; q is an integer from 1 to 5; and U is -H or SO₃H; and
   (b) reacting the CBA with the cytotoxic agent-linker compound in the presence of an antioxidant to form the immunoconjugate.
115. The method of clause 114, wherein the cytotoxic agent-linker compound is not purified before reacting with the CBA in step (b).
116. The method of clause 114 or 115, wherein the antioxidant reduces the amount of methionine oxidation in the immunoconjugate.
117. The method of clause 116, wherein the antioxidant is N-acetylmethionine methyl ester.
118. The method of any one of clauses 114-117, wherein R^{x} is independently - (CH₂)ₚ-(CR^{f}R^{g})-, wherein R^{f} and R^{g} are each independently -H or a (C₁-C₄)alkyl; and p is 0, 1, 2 or 3.
119. The method of clause 118, wherein R^{f} and R^{g} are the same or different, and are selected from -H and -Me.
120. The method of any one of clauses 114-117, wherein the immunoconjugate is represented by: 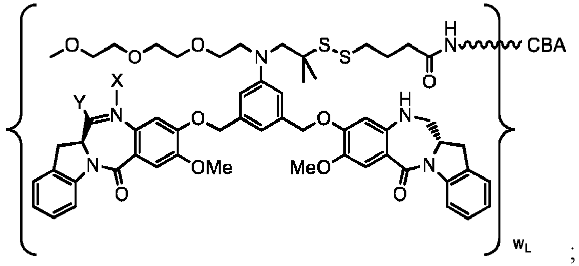 or 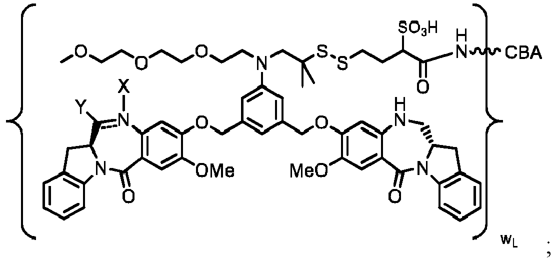 or a pharmaceutically acceptable salt thereof, wherein W_{L} is an integer from 1 to 10; the double line -̅ -̅ between N and C represents a single bond or a double bond, provided that when it is a double bond, X is absent and Y is -H; and when it is a single bond, X is -H and Y is -OH or -SO₃H.
121. The method of any one of clauses 114-120, wherein the antibody is an anti-CD33 antibody comprising an immunoglobulin heavy chain having the amino acid sequence set forth in SEQ ID NO:18 and an immunoglobulin light chain having the amino acid sequence set forth in SEQ ID NO:20.
122. The method of any one of clauses 99-121, wherein the double line -̅ -̅ between N and C represents a single bond, X is -H and Y is -OH or -SO₃H.
123. The method of any one of clauses 99-122, wherein Y is -SO₃H or sodium or potassium salt thereof.
124. The method of any one of clauses 99-123, wherein the method further comprises purifying the immunoconjugate into a formulation buffer to give a pharmaceutical composition comprising the immunoconjugate and 0.1 mM to 10 mM methionine.
125. The method of clause 124, wherein the pharmaceutical composition comprises 0.5 mM to 5 mM methionine.
126. The method of clause 124, wherein the pharmaceutical composition comprises 1 mM to 4 mM methionine.
127. The method of clause 124, wherein the pharmaceutical composition comprises 3 mM methionine.
128. The method of any one of clauses 124-127, wherein the pharmaceutical composition further comprises sodium bisulfite.
129. The method of clause 128, wherein the pharmaceutical composition further comprises 10 µM to 100 µM, 20 µM to 90 µM, or 30 µM to 80 µM of sodium bisulfite.
130. The method of clause 129, wherein the pharmaceutical composition further comprises 40 µM to 60 µM of sodium bisulfite.
131. The method of clause 129, wherein the pharmaceutical composition further comprises 50 µM of sodium bisulfite.
132. A method of preparing an immunoconjugate represented by the following formula: 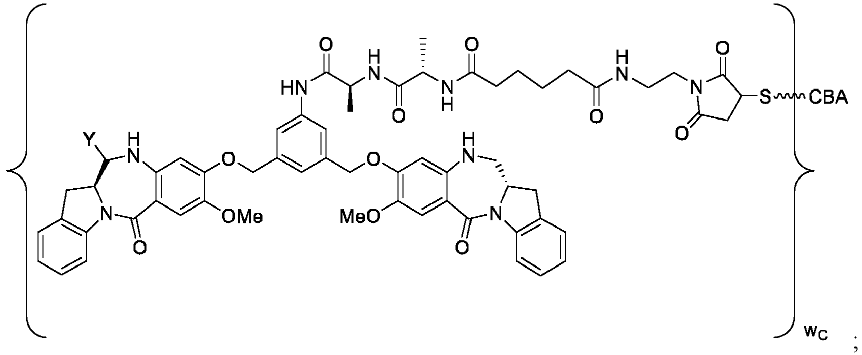 or a pharmaceutically acceptable salt thereof, comprising reacting the CBA with a cytotoxic agent represented by the following formula: 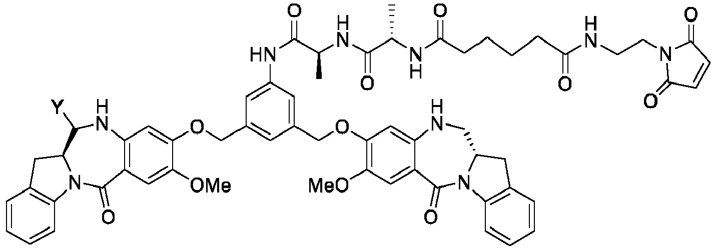 or a pharmaceutically acceptable salt thereof, in the presence of an antioxidant, wherein:
   Y is -SO₃H or sodium salt thereof;
   W_{C} is 2; and
   CBA is an anti-CD123 antibody comprising: a) an immunoglobulin heavy chain having the amino acid sequence set forth in SEQ ID NO:8; and b) an immunoglobulin light chain having the amino acid sequence set forth in SEQ ID NO: 10.
133. The method of clause 131, wherein the antioxidant reduces the amount of methionine oxidation in the immunoconjugate.
134. The method of clause 131, wherein the antioxidant is N-acetylmethionine.
135. The method of any one of clauses 131-134, wherein the method further comprises purifying the immunoconjugate into a formulation buffer to give a pharmaceutical composition comprising the immunoconjugate and 1 mM to 4 mM methionine.
136. The method of clause 135, the pharmaceutical composition comprises 3 mM methionine.
137. The method of clause 135 or 136, wherein the pharmaceutical composition further comprises 10 µM to 100 µM, 20 µM to 90 µM, or 30 µM to 80 µM of sodium bisulfite.
138. The method of clause 137, wherein the pharmaceutical composition further comprises 40 µM to 60 µM of sodium bisulfite.
139. The method of clause 138, wherein the pharmaceutical composition further comprises 50 µM of sodium bisulfite.
140. The method of clause 135, wherein the pharmaceutical composition comprises further comprises 10 mM succinate, 50 µM sodium bisulfite, 7.2% (w/v) trehalose, and 0.01% (w/v) polysorbate 20.
141. A method of preparing an immunoconjugate represented by the following formula: 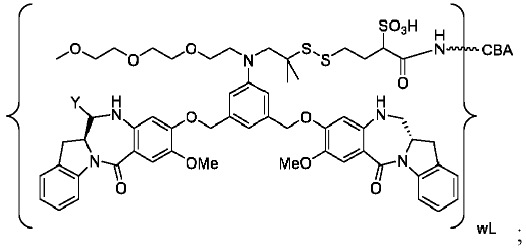 or a pharmaceutically acceptable salt thereof, comprising the steps of:
   (a) reacting a cytotoxic agent represented by the following formula: 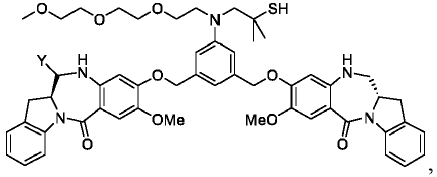 or a pharmaceutically acceptable salt thereof, with a bifunctional crosslinking agent sulfo-SPDB presented by the following formula: 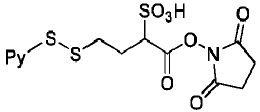 to form a cytotoxic agent-linker compound represented by the following formula: 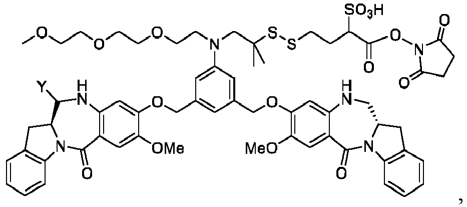 or a pharmaceutically acceptable salt thereof; and
   (b) reacting the CBA with the cytotoxic agent-linker compound in the presence of an antioxidant to form the immunoconjugate, wherein:
      Y is -SO₃H;
      W_{L} is an integer from 1 to 10; and
      CBA is an anti-CD33 antibody comprising an immunoglobulin heavy chain having the amino acid sequence set forth in SEQ ID NO: 18 and an immunoglobulin light chain having the amino acid sequence set forth in SEQ ID NO:20.
142. The method of clause 141, wherein the cytotoxic agent-linker compound is not purified before reacting with the CBA in step (b).
143. The method of clause 141 or 142, wherein the antioxidant reduces the amount of methionine oxidation in the immunoconjugate.
144. The method of clause 141 or 142, wherein the antioxidant is N-acetylmethionine methyl ester.
145. The method of any one of clauses 141-144, wherein the method further comprises purifying the immunoconjugate into a formulation buffer to give a pharmaceutical composition comprising the immunoconjugate and 1 mM to 4 mM methionine.
146. The method of clause 145, the pharmaceutical composition comprises 3 mM methionine.
147. The method of clause 145 or 146, wherein the pharmaceutical composition further comprises 10 µM to 100 µM, 20 µM to 90 µM, or 30 µM to 80 µM of sodium bisulfite.
148. The method of clause 147, wherein the pharmaceutical composition further comprises 40 µM to 60 µM of sodium bisulfite.
149. The method of clause 148, wherein the pharmaceutical composition further comprises 50 µM of sodium bisulfite.
150. The method of clause 145, wherein the pharmaceutical composition comprises further comprises 20 mM histidine, 50 µM sodium bisulfite, 7.2% (w/v) trehalose, 0.02% (w/v) polysorbate 20.

## Claims

1. A method of reducing the amount of methionine oxidation in an immunoconjugate comprising mixing the immunoconjugate with 0.1 mM to 20 mM methionine to give a pharmaceutical composition comprising the immunoconjugate and methionine, wherein the immunoconjugate is represented by the following formula: 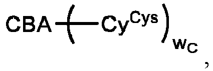
CBA is an antibody or antigen-binding fragment thereof;
Wc is 1 or 2; and
Cy^{Cys} is represented by the following formula: 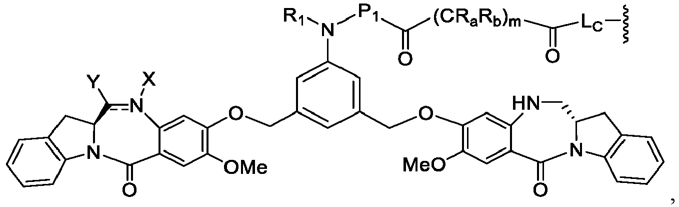
or a pharmaceutically acceptable salt thereof, wherein:
the double line -̅ -̅ between N and C represents a single bond or a double bond, provided that when it is a double bond, X is absent and Y is -H or a (C₁-C₄)alkyl; and when it is a single bond, X is -H or an amine protecting moiety, Y is -OH or -SO₃H;
R₁ is -H or a (C₁-C₃)alkyl;
P₁ is an amino acid residue or a peptide containing 2 to 5 amino acid residues;
Rₐ and R_{b}, for each occurrence, are independently -H, (C₁-C₃)alkyl, or a charged substituent or an ionizable group Q;
m is an integer from 1 to 6; Lc is represented by 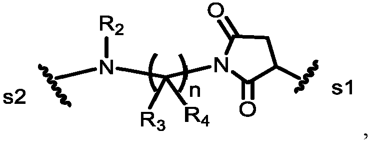 s1 is the site covalently linked to CBA, and s2 is the site covalently linked to the -C(=O)- group on Cy^{C1}; wherein:
R₂ is -H or a (C₁-C₃)alkyl
R₃ and R₄, for each occurrence, are independently -H or a (C₁-C₃)alkyl; and
n is an integer between 1 and 10.

2. The method of claim 1, wherein the pharmaceutical composition comprises:
(i) 0.1 mM to 10 mM methionine;
(ii) 0.5 mM to 5 mM methionine;
(iii) 1.0 mM to 4.0 mM methionine; or
(iv) 3.0 mM methionine.

3. The method of claim 2, wherein the pharmaceutical composition has a pH of 4 to 4.5, or a pH of 4.2.

4. The method of any one of claims 1-3, wherein Rₐ and R_{b} are both H; and R₁ is H or Me.

5. The method of any one of claims 1-4, wherein P is selected from Gly-Gly-Gly, Ala-Val, Val-Ala, Val-Cit, Val-Lys, Phe-Lys, Lys-Lys, Ala-Lys, Phe-Cit, Leu-Cit, Ile-Cit, Trp, Cit, Phe-Ala, Phe-N⁹-tosyl-Arg, Phe-N⁹-nitro-Arg, Phe-Phe-Lys, D-Phe-Phe-Lys, Gly-Phe-Lys, Leu-Ala-Leu, Ile-Ala-Leu, Val-Ala-Val, Ala-Leu-Ala-Leu (SEQ ID NO:21), β-Ala-Leu-Ala-Leu (SEQ ID NO:22), Gly-Phe-Leu-Gly (SEQ ID NO:23), Val-Arg, Arg-Val, Arg-Arg, Val-D-Cit, Val-D-Lys, Val-D-Arg, D-Val-Cit, D-Val-Lys, D-Val-Arg, D-Val-D-Cit, D-Val-D-Lys, D-Val-D-Arg, D-Arg-D-Arg, Ala-Ala, Ala-D-Ala, D-Ala-Ala, D-Ala-D-Ala, Ala-Met, and Met-Ala, optionally, wherein P is Ala-Ala, Ala-D-Ala, D-Ala-Ala, or D-Ala-D-Ala.

6. The method of any one of claims 1-5, wherein Q is -SO₃H.

7. The method of any one of claims 1-6, wherein R₃ and R₄ are both H; and n is an integer from 1 to 6.

8. The method of any one of claims 1-7, wherein -Lc- is represented by the following formula:

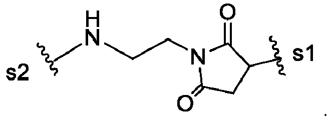

9. The method of any one of claims 1-3, wherein the immunoconjugate is represented by the following formula: 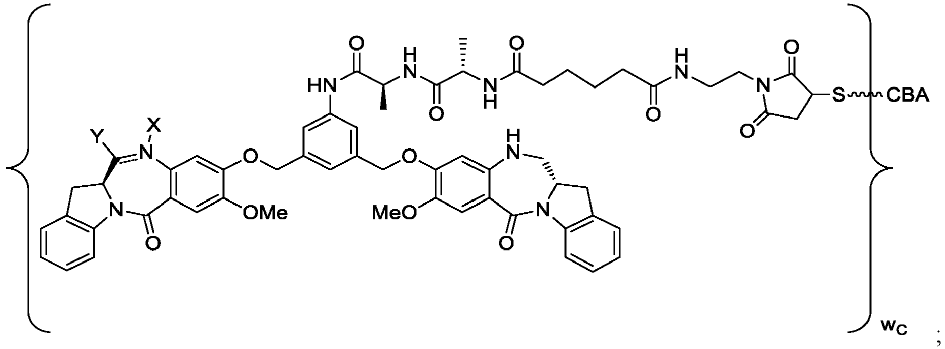 or a pharmaceutically acceptable salt thereof, wherein the double line -̅ -̅ between N and C represents a single bond or a double bond, provided that when it is a double bond, X is absent and Y is -H, and when it is a single bond, X is -H, and Y is -OH or -SO₃H.

10. The method of any one of claims 1-9, wherein the antibody or antigen-binding fragment thereof is an anti-CD 123 antibody or antigen-binding fragment thereof comprising:
a) an immunoglobulin heavy chain variable region comprising a CDR1 having an amino acid sequence set forth in SEQ ID NO:4, a CDR2 having an amino acid sequence set forth in SEQ ID NO:5, and a CDR3 having an amino acid sequence set forth in SEQ ID NO:6; and
b) an immunoglobulin light chain variable region comprising a CDR1 having an amino acid sequence set forth in SEQ ID NO:1, a CDR2 having an amino acid sequence set forth in SEQ ID NO:2, and a CDR3 having an amino acid sequence set forth in SEQ ID NO:3.

11. The method of claim 10, wherein the antibody or antigen-binding fragment thereof comprises a V_{H} sequence of SEQ ID NO:7 and a V_{L} sequence of SEQ ID NO:9.

12. The method of claim 10, wherein the antibody comprises:
a) an immunoglobulin heavy chain having the amino acid sequence set forth in SEQ ID NO:8; and
b) an immunoglobulin light chain having the amino acid sequence set forth in SEQ ID NO: 10.

13. The method of any one of claims 1-12, wherein the pharmaceutical composition comprises 1 mg/mL to 5 mg/mL, 1 mg/mL to 3 mg/mL, or 1.5 mg/mL to 2.5 mg/mL of the immunoconjugate, or the pharmaceutical composition comprises 2 mg/mL of the immunoconjugate.

14. The method of any one of claims 1-13, wherein the pharmaceutical composition further comprises 10 µM to 100 µM, 20 µM to 90 µM, 30 µM to 80 µM, or 40 µM to 60 µM of sodium bisulfite, or the pharmaceutical composition further comprises 50 µM of sodium bisulfite.

15. The method of any one of claims 1-14, wherein the pharmaceutical composition further comprises 5 mM to 50 mM succinate, 5-10% (w/v) trehalose, and 0.01-0.1% (w/v) polysorbate 20.
